# EUROPEAN PATENT SPECIFICATION

(11) **EP 3 154 949 B1**
(45) Date of publication and mention of the grant of the patent: **07.03.2018**
(21) Application number: 15725017.6
(22) Date of filing: 28.05.2015
(51) Int. Cl.: C07D 233/60, C07D 249/08, A01N 43/653, A01N 43/50

(54) **SUBSTITUTED [1,2,4]TRIAZOLE AND IMIDAZOLE COMPOUNDS AS FUNGICIDES**
SUBSTITUIERTE [1,2,4]TRIAZOL- UND IMIDAZOLVERBINDUNGEN ALS FUNGIZIDE
COMPOSÉS [1,2,4]-TRIAZOLES ET IMIDAZOLES SUBSTITUÉS UTILISÉS COMME FONGICIDES

(30) Priority: 10.06.2014 EP 14171796
(43) Date of publication of application: 19.04.2017
(73) Proprietor: BASF SE, 67056 Ludwigshafen am Rhein (DE)
(72) Inventor: GRAMMENOS, Wassilios, 67071 Ludwigshafen (DE); BOUDET, Nadege, 69493 Hirschberg (DE); MUELLER, Bernd, 67227 Frankenthal (DE); QUINTERO PALOMAR, Maria Angelica, 68165 Mannheim (DE); ESCRIBANO CUESTA, Ana, 68161 Mannheim (DE); LAUTERWASSER, Erica May Wilson, 67157 Wachenheim (DE); LOHMANN, Jan Klaas, 67245 Lambsheim (DE); GROTE, Thomas, 67157 Wachenheim (DE); KRETSCHMER, Manuel, 68165 Mannheim (DE); CRAIG, Ian Robert, 67063 Ludwigshafen (DE)
(74) Representative: BASF IP Association
(86) International application number: PCT/EP2015/061772
(87) International publication number: WO 2015/189035

(56) References cited:
- EP-A2- 0 071 568
- WO-A1-2014/082880
- WO-A1-2014/095548

## Description

The present invention relates to substituted [1,2,4]triazole and imidazole compounds and the N-oxides and the salts thereof for combating phytopathogenic fungi, and to the use and methods for combating phytopathogenic fungi and to seeds coated with at least one such compound. The invention also relates to processes for preparing these compounds, intermediates, processes for preparing such intermediates, and to compositions comprising at least one compound I.

DE 4003180 relates to halogenallyl azolyl derivatives and their use as microbiocides. DE 3222191 relates to hydroxy alkynyl azolyl derivatives and their use as fungicides. US 4935436 relates to substituted triazoles and their use as fungicides. DE 3440116 relates to substituted azolylmethyl cyclopropyl carbinols and their use as fungicides. DE 3402166 relates to azolyl aryl alkanol derivatives and their use as fungicides. FR 2469408 relates to 1,2,4-triazoles and their use as fungicides.

EP-A 071568 discloses compounds useful for combating phytopathogenic fungi having the same core structure as the present compounds I. However, the known compounds differ in the definition of the group R¹, which there is an electro with drawing (thio)ester group.

WO 2014/082880 discloses fungicides wherein the definition of the group R¹ overlaps with the present one. However, said known compounds must have an oxygen linker between the biphenyl moiety.

None oft he prior art documents disclose or render obvious the biphenyl triazole and imidazole compounds of the present invention having a specific substitution pattern at the biphenyl unit.

In many cases, in particular at low application rates, the fungicidal activity of the known fungicidal compounds is unsatisfactory. Based on this, it was an object of the present invention to provide compounds having improved activity and/or a broader activity spectrum against phytopathogenic harmful fungi.

Surprisingly, this object is achieved by the use of the inventive substituted [1,2,4]triazole and imidazole compounds of formula I having favorable fungicidal activity against phytopathogenic fungi.

Accordingly, the present invention relates to the compounds of the formula I 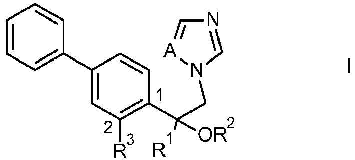 wherein
- A: is CH or N;
- R¹: is C₁-C₆-alkyl, C₂-C₆-alkenyl, C₂-C₆-alkynyl or C₃-C₆-cycloalkyl;
wherein the aliphatic moieties of R¹ are not further substituted or carry one, two, three or up to the maximum possible number of identical or different groups R^{1a} which independently of one another are selected from:
R^{1a} halogen, OH, CN, C₁-C₄-alkoxy, C₃-C₆-cycloalkyl, C₃-C₆-halogencycloalkyl and C₁-C₄-halogenalkoxy;
wherein the cycloalkyl moieties of R¹ are not further substituted or carry one, two, three, four, five or up to the maximum number of identical or different groups R^{1b} which independently of one another are selected from:
R^{1b} halogen, OH, CN, C₁-C₄-alkyl, C₁-C₄-alkoxy, C₁-C₄-halogenalkyl, C₃-C₆-cycloalkyl, C₃-C₆-halogencycloalkyl and C₁-C₄-halogenalkoxy;
R² is hydrogen, C₁-C₄-alkyl, C₂-C₄-alkenyl or C₂-C₄-alkynyl;
wherein the aliphatic moieties of R² are not further substituted or carry one, two, three or up to the maximum possible number of identical or different groups R^{2a} which independently of one another are selected from:
R^{2a} halogen, OH, CN, C₁-C₄-alkoxy, C₃-C₆-cycloalkyl, C₃-C₆-halogencycloalkyl and C₁-C₄-halogenalkoxy;
R³ is selected from halogen, CN, C₁-C₄-alkyl, C₁-C₄-alkoxy, C₂-C₄-alkenyl, C₂-C₄-alkynyl, C₃-C₆-cycloalkyl and S(O)ₚ(C₁-C₄-alkyl), wherein each of R³ is unsubstituted or further substituted by one, two, three or four R^{3a}; wherein
R^{3a} is independently selected from halogen, CN, OH, C₁-C₄-alkyl, C₁-C₄-halogenalkyl, C₃-C₆-cycloalkyl, C₃-C₆-halogencycloalkyl, C₁-C₄-alkoxy and C₁-C₄-halogenalkoxy;
p is 0, 1 or 2;
and the N-oxides and the agriculturally acceptable salts thereof. Compounds I can be accessed from compounds II by using a suitable cross coupling method. A skilled person will recognize that compounds II can be reacted with [Phenyl-M], with M being preferably Mg, Zn or a boronic acid (derivative) in the presence of a catalyst. Preferably Cul, Pd(PPh₃)Cl₂ or Pd(PPh₃)₄ or Pd(dppf)₂Cl₂, or mixtures thereof are used in a solvent (such as THF, DMF, MeCN, 1,4-dioxane, or 1,2-dimethoxyethane), if appropriate at elevated temperatures and in the presence of a base (such as NaOH, NEt₃, Et(iPr₂)N, K₂CO₃, or K₃PO₄).

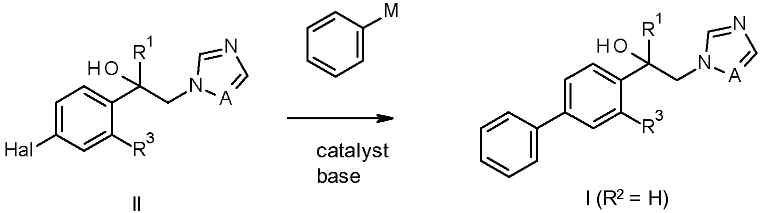

Compounds I, wherein R² is different from hydrogen can be obtained from alcohol compounds I (R²=H) by reacting the alcohol compound with R²-LG, wherein LG represents a nucleophilically replaceable leaving group, such as halogen, alkylsulfonyl, alkylsulfonyloxy and arylsulfonyloxy, preferably chloro, bromo or iodo, particularly preferably bromo, preferably in the presence of a base, such as for example, NaH in a suitable solvent such as THF.

Halo compounds II can be obtained as follows. For example, a phenyliodide of type V bearing additional substituents with Hal preferably being Br can be transformed into an acetophenone of type IVa by several methods known to a skilled person. Preferably, V is transformed into a Grignard reagent by the reaction with a suitable transmetallation agent such as iso-propyl magnesium chloride under inert conditions in an ethereal solvent such as THF and reacted with acetyl chloride. If necessary, an additive such as LaCl₃, CuCl₂, AlCl₃ or mixtures hereof can be added. Acetyl compounds of type IVa can be halogenated using a method known to the skilled person. Preferably, IVa is chlorinated using a chlorination agent such as Cl₂, NCS or SO₂Cl₂ to afford α-chloro ketones of type IVb which subsequently can be treated with an appropriate organometallic species, preferably a Grignard compound, to furnish a tertiary alcohol of type IIIa:

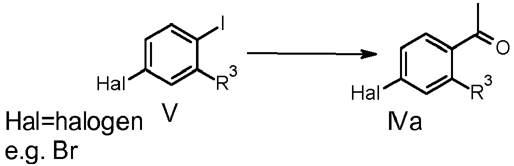

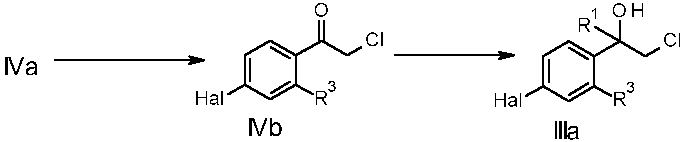

Exposure of IIIa to a suitable azole compound in the presence of base leads to the formation of compounds of type II through nucleophilic replacement of the chloride:

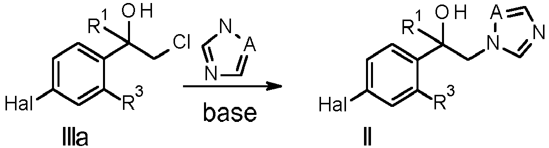

Alternatively, intermediate compounds of type II can be prepared as follows: Compounds V can be transformed into their respective Grignard analoga using the methodology described above before being treated with an appropriate acid chloride to furnish compounds of type VI:

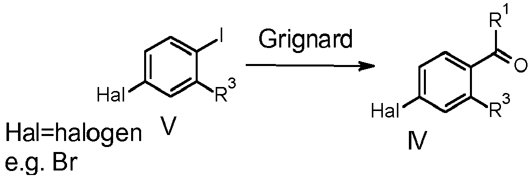

Thereafter, intermediates IV are e.g. reacted with trimethylsulf(ox)onium halides, preferably iodide, preferably in the presence of a base such as sodium hydride, to result in epoxides III.

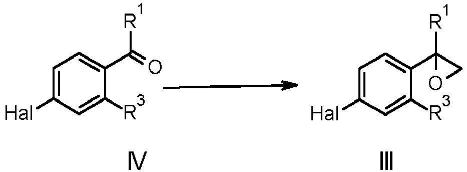

The epoxides III are then reacted with 1H-1,2,4-triazole or imidazole preferably in the presence of a base such as potassium carbonate and preferably in the presence of an organic solvent such as DMF to obtain intermediate II.

The N-oxides may be prepared from the inventive compounds according to conventional oxidation methods, e. g. by treating compounds I with an organic peracid such as metachloroper-benzoic acid (cf. WO 03/64572 or J. Med. Chem. 38(11), 1892-903, 1995); or with inorganic oxidizing agents such as hydrogen peroxide (cf. J. Heterocyc. Chem. 18(7), 1305-8, 1981) or oxone (cf. J. Am. Chem. Soc. 123(25), 5962-5973, 2001). The oxidation may lead to pure mono-N-oxides or to a mixture of different N-oxides, which can be separated by conventional methods such as chromatography.

If the synthesis yields mixtures of isomers, a separation is generally not necessarily required since in some cases the individual isomers can be interconverted during work-up for use or during application (e. g. under the action of light, acids or bases). Such conversions may also take place after use, e. g. in the treatment of plants in the treated plant, or in the harmful fungus to be controlled.

In the following, the intermediate compounds are further described. A skilled person will readily understand that the preferences for the substituents, also in particular the ones given in the tables below for the respective substituents, given herein in connection with compounds I apply for the intermediates accordingly. Thereby, the substituents in each case have independently of each other or more preferably in combination the meanings as defined herein.

Compounds of formula V are at least partially new. Consequently, a further embodiment of the present invention are compounds of formula V (see above), wherein the variables are as defined and preferably defined for formula I herein.

Compounds of formula IV are at least partially new. Consequently, a further embodiment of the present invention are compounds of formula IV (see above), wherein the variables are as defined and preferably defined for formula I herein.

Compounds of formula IVa are at least partially new. Consequently, a further embodiment of the present invention are compounds of formula IVa (see above), wherein the variables are as defined and preferably defined for formula I herein.

Compounds of formula IVb are at least partially new. Consequently, a further embodiment of the present invention are compounds of formula IVb (see above), wherein the variables are as defined and preferably defined for formula I herein.

Compounds of formula III are at least partially new. Consequently, a further embodiment of the present invention are compounds of formula III (see above), wherein the variables are as defined and preferably defined for formula I herein.

Compounds of formula IIIa are at least partially new. Consequently, a further embodiment of the present invention are compounds of formula IIIa (see above), wherein the variables are as defined and preferably defined for formula I herein.

In the definitions of the variables given above, collective terms are used which are generally representative for the substituents in question. The term "Cₙ-Cₘ" indicates the number of carbon atoms possible in each case in the substituent or substituent moiety in question.

The term "halogen" refers to fluorine, chlorine, bromine and iodine.

The term "C₁-C₆-alkyl" refers to a straight-chained or branched saturated hydrocarbon group having 1 to 6 carbon atoms, e.g. methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, 1,1-dimethylpropyl, 1,2-dimethylpropyl, hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl. Likewise, the term "C₂-C₄-alkyl" refers to a straight-chained or branched alkyl group having 2 to 4 carbon atoms, such as ethyl, propyl (n-propyl), 1-methylethyl (iso-propoyl), butyl, 1-methylpropyl (sec.-butyl), 2-methylpropyl (iso-butyl), 1,1-dimethylethyl (tert.-butyl).

The term "C₁-C₆-haloalkyl" refers to an alkyl group having 1 or 6 carbon atoms as defined above, wherein some or all of the hydrogen atoms in these groups may be replaced by halogen atoms as mentioned above. Examples are "C₁-C₂-haloalkyl" groups such as chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl or pentafluoroethyl.

The term "C₁-C₆-hydroxyalkyl" refers to an alkyl group having 1 or 6 carbon atoms as defined above, wherein some or all of the hydrogen atoms in these groups may be replaced by OH groups.

The term "C₂-C₆-alkenyl" refers to a straight-chain or branched unsaturated hydrocarbon radical having 2 to 6 carbon atoms and a double bond in any position. Examples are "C₂-C₄-alkenyl" groups, such as ethenyl, 1-propenyl, 2-propenyl (allyl), 1-methylethenyl, 1-butenyl, 2-butenyl, 3-butenyl, 1-methyl-1-propenyl, 2-methyl-1-propenyl, 1-methyl-2-propenyl, 2-methyl-2-propenyl.

1-alkenyl such as 1-(C₂-C₆)-alkenyl, 1-(C₂-C₄)-alkenyl or 1-C₃-alkenyl means that the alkenyl group is attached to the respective skeleton via a carbon atom of the double bond (e.g. CH=CHCH₃).

The term "C₂-C₆-alkynyl" refers to a straight-chain or branched unsaturated hydrocarbon radical having 2 to 6 carbon atoms and containing at least one triple bond. Examples are "C₂-C₄-alkynyl" groups, such as ethynyl, prop-1-ynyl, prop-2-ynyl (propargyl), but-1-ynyl, but-2-ynyl, but-3-ynyl, 1-methyl-prop-2-ynyl.

1-alkynyl such as 1-(C₂-C₆)-alkynyl, 1-(C₂-C₄)-alkynyl or 1-C₃-alkynyl means that the alkynyl group is attached to the respective skeleton via a carbon atom of the triple bond (e.g. C≡C-CH₃).

The term "C₃-C₆-cycloalkyl" refers to monocyclic saturated hydrocarbon radicals having 3 to 6 carbon ring members, such as cyclopropyl, cyclobutyl, cyclopentyl, cyclohexyl.

The term "C₃-C₈-cycloalkyl-C₁-C₄-alkyl" refers to alkyl having 1 to 4 carbon atoms (as defined above), wherein one hydrogen atom of the alkyl radical is replaced by a cycloalkyl radical having 3 to 8 carbon atoms (as defined above).

The term "C₁-C₆-alkoxy" refers to a straight-chain or branched alkyl group having 1 to 6 carbon atoms which is bonded via an oxygen, at any position in the alkyl group. Examples are "C₁-C₄-alkoxy" groups, such as methoxy, ethoxy, n-propoxy, 1-methylethoxy, butoxy, 1-methyl¬propoxy, 2-methylpropoxy or 1,1-dimethylethoxy.

The term "C₁-C₆-haloalkoxy" refers to a C₁-C₆-alkoxy radical as defined above, wherein some or all of the hydrogen atoms in these groups may be replaced by halogen atoms as mentioned above. Examples are "C₁-C₄-haloalkoxy" groups, such as OCH₂F, OCHF₂, OCF₃, OCH₂Cl, OCHCl₂, OCCl₃, chlorofluoromethoxy, dichlorofluoromethoxy, chlorodifluoromethoxy, 2-fluoroethoxy, 2-chloroethoxy, 2-bromoethoxy, 2-iodoethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-tri-chloro¬ethoxy, OC₂F₅, 2-fluoropropoxy, 3-fluoropropoxy, 2,2-difluoropropoxy, 2,3-difluoro¬propoxy, 2 chloropropoxy, 3-chloropropoxy, 2,3-dichloropropoxy, 2-bromo¬propoxy, 3 bromopropoxy, 3,3,3-trifluoropropoxy, 3,3,3-trichloropropoxy, OCH₂-C₂F₅, OCF₂-C₂F₅, 1-fluoromethyl-2-fluoroethoxy, 1-chloromethyl-2-chloroethoxy, 1-bromomethyl-2-bromo¬ethoxy, 4-fluorobutoxy, 4-chlorobutoxy, 4-bromobutoxy or nonafluorobutoxy.

The term "C₁-C₄-alkoxy-C₁-C₄-alkyl" refers to alkyl having 1 to 4 carbon atoms (as defined above), wherein one hydrogen atom of the alkyl radical is replaced by a C₁-C₄-alkoxy group (as defined above). Likewise, the term "C₁-C₆-alkoxy-C₁-C₄-alkyl" refers to alkyl having 1 to 4 carbon atoms (as defined above), wherein one hydrogen atom of the alkyl radical is replaced by a C₁-C₆-alkoxy group (as defined above).

The term "C₁-C₆-alkylthio" as used herein refers to straight-chain or branched alkyl groups having 1 to 6 carbon atoms (as defined above) bonded via a sulfur atom. Accordingly, the term "C₁-C₆-haloalkylthio" as used herein refers to straight-chain or branched haloalkyl group having 1 to 6 carbon atoms (as defined above) bonded through a sulfur atom, at any position in the haloalkyl group.

The term "C₁-C₆-alkylsulfinyl" refers to straight-chain or branched alkyl groups having 1 to 6 carbon atoms (as defined above) bonded through a -S(=O)- moiety, at any position in the alkyl group, for example methylsulfinyl and ethylsulfinyl, and the like. Accordingly, the term "C₁-C₆-haloalkylsulfinyl" refers to straight-chain or branched haloalkyl group having 1 to 6 carbon atoms (as defined above), bonded through a -S(=O)- moiety, at any position in the haloalkyl group.

The term "C₁-C₆-alkylsulfonyl" refers to straight-chain or branched alkyl groups having 1 to 6 carbon atoms (as defined above), bonded through a -S(=O)₂- moiety, at any position in the alkyl group, for example methylsulfonyl. Accordingly, the term "C₁-C₆-haloalkylsulfonyl" refers to straight-chain or branched haloalkyl group having 1 to 6 carbon atoms (as defined above), bonded through a -S(=O)₂- moiety, at any position in the haloalkyl group.

The term "C₃-C₈-cycloalkyl-C₃-C₈-cycloalkyl" refers to a cycloalkyl radical having 3 to 8 carbon atoms (as defined above), which is substituted by a further cycloalkyl radical having 3 to 8 carbon atoms.

Agriculturally acceptable salts of the inventive compounds encompass especially the salts of those cations or the acid addition salts of those acids whose cations and anions, respectively, have no adverse effect on the fungicidal action of said compounds. Suitable cations are thus in particular the ions of the alkali metals, preferably sodium and potassium, of the alkaline earth metals, preferably calcium, magnesium and barium, of the transition metals, preferably manganese, copper, zinc and iron, and also the ammonium ion which, if desired, may carry one to four C₁-C₄-alkyl substituents and/or one phenyl or benzyl substituent, preferably diisopropylammonium, tetramethylammonium, tetrabutylammonium, trimethylbenzylammonium, furthermore phosphonium ions, sulfonium ions, preferably tri(C₁-C₄-alkyl)sulfonium, and sulfoxonium ions, preferably tri(C₁-C₄-alkyl)sulfoxonium. Anions of useful acid addition salts are primarily chloride, bromide, fluoride, hydrogensulfate, sulfate, dihydrogenphosphate, hydrogenphosphate, phosphate, nitrate, bicarbonate, carbonate, hexafluorosilicate, hexafluorophosphate, benzoate, and the anions of C₁-C₄-alkanoic acids, preferably formate, acetate, propionate and butyrate. They can be formed by reacting such inventive compound with an acid of the corresponding anion, preferably of hydrochloric acid, hydrobromic acid, sulfuric acid, phosphoric acid or nitric acid.

The inventive compounds can be present in atropisomers arising from restricted rotation about a single bond of asymmetric groups. They also form part of the subject matter of the present invention.

Depending on the substitution pattern, the compounds of formula I and their N-oxides may have one or more centers of chirality, in which case they are present as pure enantiomers or pure diastereomers or as enantiomer or diastereomer mixtures. Both, the pure enantiomers or diastereomers and their mixtures are subject matter of the present invention.

In the following, particular embodiments of the inventive compounds are described. Therein, specific meanings of the respective substituents are further detailled, wherein the meanings are in each case on their own but also in any combination with one another, particular embodiments of the present invention.

Furthermore, in respect of the variables, generally, the embodiments of the compounds I also apply to the intermediates.

A according to the invention is N or CH. According to one embodiment A is N. According to a further embodiment A is CH.

R¹ according to the invention is C₁-C₆-alkyl, C₂-C₆-alkenyl, C₂-C₆-alkynyl or C₃-C₆-cycloalkyl; wherein the aliphatic moieties of R¹ are not further substituted or carry one, two, three or up to the maximum possible number of identical or different groups R^{1a} which independently of one another are selected from halogen, OH, CN, C₁-C₄-alkoxy, C₃-C₈-Cycloalkyl, C₃-C₈-halocycloalkyl and C₁-C₄-halogenalkoxy; and wherein the cycloalkyl moieties of R¹ are not further substituted or carry one, two, three, four, five or up to the maximum number of identical or different groups R^{1b} which independently of one another are selected from halogen, OH, CN, C₁-C₄-alkyl, C₁-C₄-alkoxy, C₁-C₄-halogenalkyl, C₃-C₈-Cycloalkyl, C₃-C₈-halocycloalkyl and C₁-C₄-halogenalkoxy.

According to a further embodiment of the invention, R¹ is selected from C₁-C₆-alkyl, C₂-C₆-alkenyl, C₂-C₆-alkynyl, C₃-C₆-cycloalkyl and C₃-C₆-cycloalkyl-C₁-C₄-alkyl, wherein the R¹ are in each case unsubstituted or are substituted by R^{1a} and/or R^{1b} as defined and preferably defined herein. Specific embodiments thereof can be found in the below Table P1.

According to a further embodiment of the invention, R¹ is selected from C₁-C₆-alkyl, 1-(C₂-C₆)-alkenyl, 1-(C₂-C₆)-alkynyl and C₃-C₆-cycloalkyl, wherein the aliphatic moieties of R¹ are in each case unsubstituted or carry one, two, three, four or five R^{1a} independently selected from F, OH, CN, C₃-C₆-cycloalkyl, C₃-C₆-halogencycloalkyl and C₁-C₄-halogenalkoxy, in particular selected from F, OH, CN, C₃-C₆-cycloalkyl, C₃-C₆-halogencycloalkyl and C₁-C₂-halogenalkoxy, more specifically selected from F, OH, CN, C₃-C₆-cycloalkyl and C₃-C₆-halogencycloalkyl, and wherein the cycloalkyl moieties of R¹ are in each case unsubstituted or carry one, two, three, four or five R^{1b} independently selected from OH, C₁-C₄-alkyl, C₁-C₂-alkoxy, C₁-C₂-halogenalkyl, C₃-C₆-cycloalkyl, C₃-C₆-halogencycloalkyl and C₁-C₂-halogenalkoxy, in particular selected from OH, C₁-C₂-alkyl, C₃-C₆-cycloalkyl and C₃-C₆-halogencycloalkyl. Specific embodiments thereof can be found in the below Table P1.

According to one particular embodiment, R¹ is C₁-C₆-alkyl, in particular C₁-C₄-alkyl, such as CH₃, C₂H₅, CH(CH₃)₂ or C(CH₃)₃. A further embodiment relates to compounds, wherein R¹ is C₁-C₆-alkyl, in particular C₁-C₄-alkyl that is substituted by one, two or three or up to the maximum possible number of identical or different groups R^{1a}, as defined and preferably defined herein. According to a specific embodiment thereof, R¹ is C₁-C₆-haloalkyl, in particular C₁-C₄-haloalkyl, more particularly C₁-C₂-haloalkyl that is substituted by one or more F, such as CF₃ or CHF₂. Further specific embodiments thereof can be found in the below Table P1.

According to still another embodiment, R¹ is C₃-C₈-cycloalkyl-C₁-C₆-alkyl, in particular C₃-C₆-cycloalkyl-C₁-C₄-alkyl. A further embodiment relates to compounds, wherein R¹ is C₃-C₈-cycloalkyl-C₁-C₆-alkyl, in particular C₃-C₆-cycloalkyl-C₁-C₄-alkyl, that is substituted by one, two or three or up to the maximum possible number of identical or different groups R^{1a} in the alkyl moiety and/or substituted by one, two, three four or five or up to the maximum possible number of identical or different groups R^{1b} in the cycloalkyl moiety. R^{1a} are in each case as defined and preferably defined herein. Specific embodiments thereof can be found in the below Table P1.

According to another embodiment, R¹ is C₂-C₆-alkenyl, in particular C₂-C₄-alkenyl, such as CH=CH₂, CH₂CH=CH₂, CH=CHCH₃ or C(CH₃)=CH₂. A further embodiment relates to compounds, wherein R¹ is C₂-C₆-alkenyl, in particular C₂-C₄-alkenyl that is substituted by one, two or three or up to the maximum possible number of identical or different groups R^{1a} as defined and preferably defined herein. According to a specific embodiment thereof, R¹ is C₂-C₆-haloalkenyl, in particular C₂-C₄-haloalkenyl. Further specific embodiments thereof can be found in the below Table P1.

According to a further specific embodiment, R¹ is 1-(C₂-C₆)-alkenyl, in particular 1-(C₂-C₄)-alkenyl, more specifically 1-(C₂-C₃)-alkenyl, such as CH=CH₂, CH=CHCH₃ or C(CH₃)=CH₂. A further embodiment relates to compounds, wherein R¹ is 1-(C₂-C₆)-alkenyl, in particular 1-(C₂-C₄)-alkenyl, more specifically 1-(C₂-C₃)-alkenyl, that is substituted by one, two or three or up to the maximum possible number of identical or different groups R^{1a} as defined and preferably defined herein. Further specific embodiments thereof can be found in the below Table P1.

According to still another embodiment, R¹ is C₂-C₆-alkynyl, in particular C₂-C₄-alkynyl, such as C≡CH, C≡CCH₃, CH₂-C≡C-H or CH₂-C≡C-CH₃.

A further embodiment relates to compounds, wherein R¹ is C₂-C₆-alkynyl, in particular C₂-C₄-alkynyl that is substituted by one, two or three or up to the maximum possible number of identical or different groups R^{1a}, as defined and preferably defined herein. According to a specific embodiment thereof, R¹ is C₂-C₆-haloalkynyl, in particular C₂-C₄-haloalkynyl. According to a further specific embodiment thereof, R¹ is C₃-C₆-cycloalkyl-C₂-C₆-alkynyl or C₃-C₆-halocycloalkyl-C₂-C₆-alkynyl, in particular C₃-C₆-cycloalkyl-C₂-C₄-alkynyl or C₃-C₆-halocycloalkyl-C₂-C₄-alkynyl. Further specific embodiments thereof can be found in the below Table P1.

According to a further specific embodiment, R¹ is 1-(C₂-C₆)-alkynyl, in particular 1-(C₂-C₄)-alkynyl, more specifically 1-(C₂-C₃)-alkynyl, such as C≡CH or C≡CCH₃. A further embodiment relates to compounds, wherein R¹ is 1-(C₂-C₆)-alkynyl, in particular 1-(C₂-C₄)-alkynyl, more specifically 1-(C₂-C₃)-alkynyl, that is substituted by one, two or three or up to the maximum possible number of identical or different groups R^{1a} as defined and preferably defined herein. Further specific embodiments thereof can be found in the below Table P1.

According to still another embodiment, R¹ is C₃-C₆-cycloalkyl, such as C₃H₅ (cyclopropyl), C₄H₇ (cyclobutyl), cyclopentyl or cyclohexyl. A further embodiment relates to compounds, wherein R¹ is C₃-C₆-cycloalkyl, such as C₃H₅ (cyclopropyl) or C₄H₇ (cyclobutyl), that is substituted by one, two, three four or five or up to the maximum possible number of identical or different groups R^{1b} as defined and preferably defined herein, in particular selected from C₁-C₄-alkyl, C₁-C₄-alkoxy, C₁-C₄-halogenalkyl, C₃-C₆-cycloalkyl, C₃-C₆-halocycloalkyl and C₁-C₄-halogenalkoxy. According to a specific embodiment thereof, R¹ is C₃-C₆-halocycloalkyl, such as halocyclopropyl, in particular 1-F-cyclopropyl or 1-Cl-cyclopropyl. According to a further specific embodiment thereof, R¹ C₃-C₆-cycloalkyl-C₃-C₆-cycloalkyl, wherein each of said cycloalkyl-cycloalkyl moieties is unsubstituted or carries one, two or three R^{1b} as defined and preferably defined herein, such as 1-cyclopropyl-cyclopropyl or 2-cyclopropyl-cyclopropyl. Specific embodiments thereof can be found in the below Table P1.

Specifically, it may be preferred, if R¹ is selected from C₁-C₄-alkyl, such as methyl, ethyl, n-propyl, iso-propyl, tert-butyl, CH₂C(CH₃)₃ and CH₂CH(CH₃)₂, C₁-C₄-haloalkyl, in particular C₁-C₄-fluoroalkyl, such as CF₃, C₂-C₆-alkenyl, C₂-C₆-alkynyl, such as C≡CCH₃, and C₃-C₆-cycloalkyl, such as cyclopropyl.

Particularly preferred embodiments of R¹ according to the invention are in Table P1 below, wherein each line of lines P1-1 to P1-138 corresponds to one particular embodiment of the invention, wherein P1-1 to P1-138 are also in any combination a preferred embodiment of the present invention.

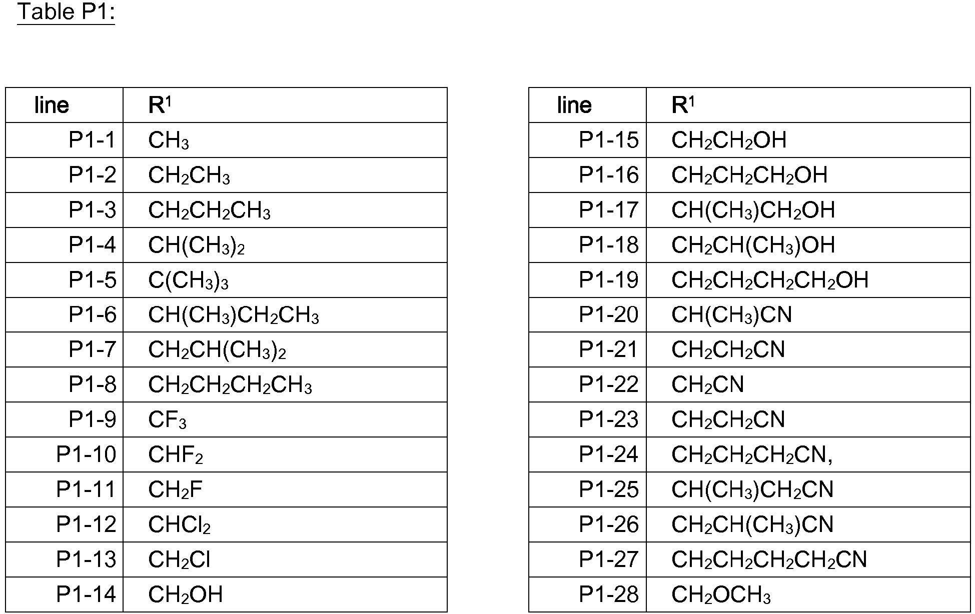

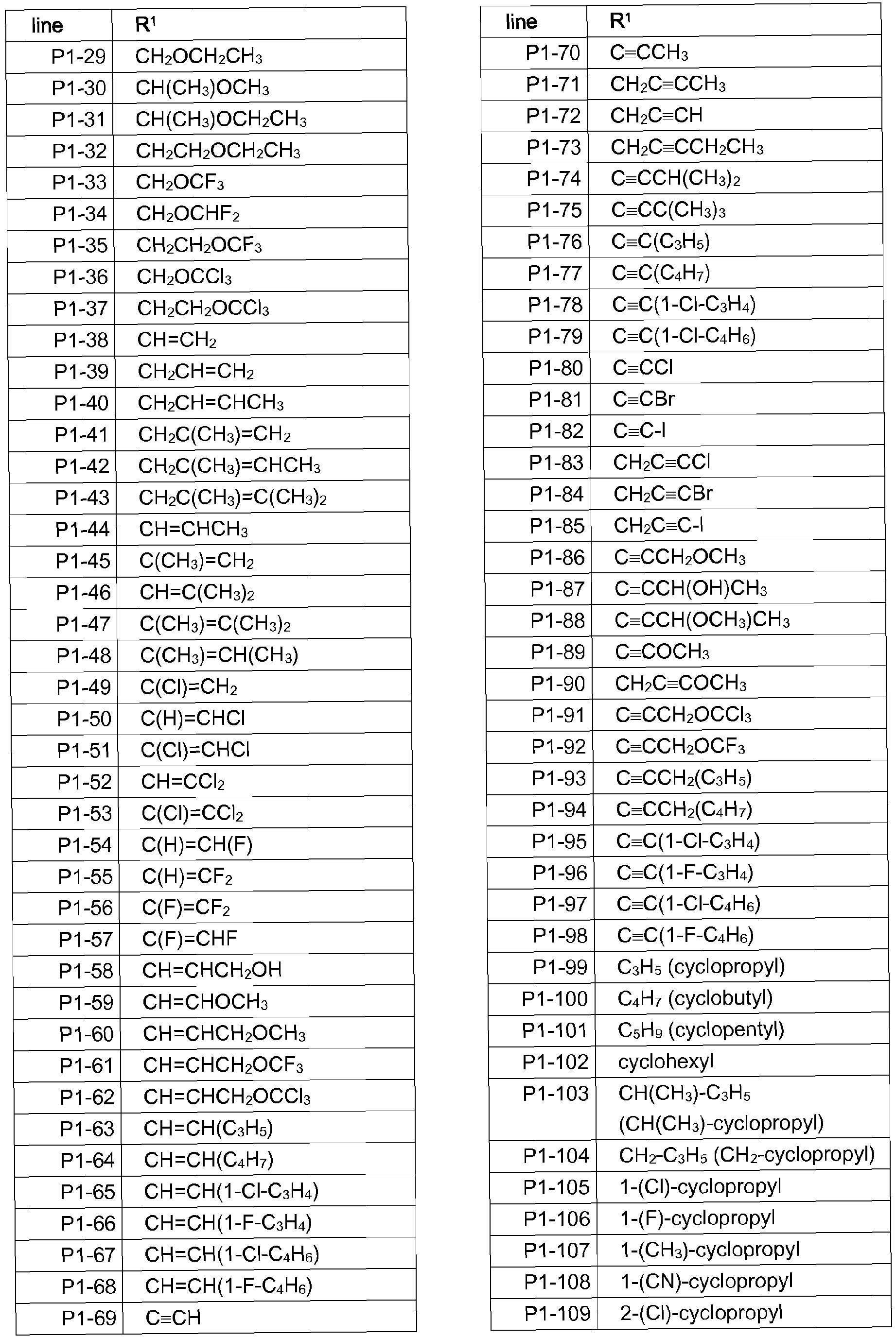

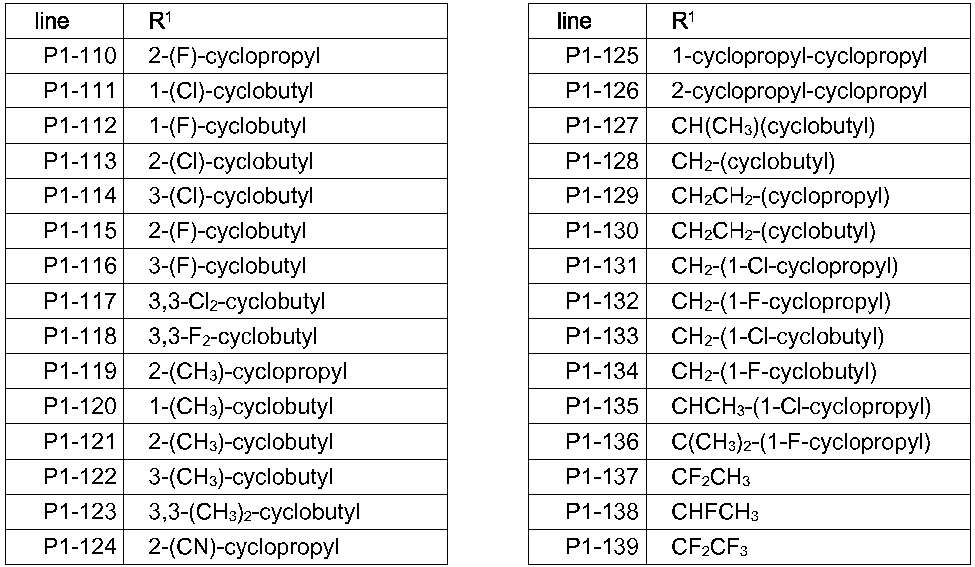

In a specifcally preferred embodiment, R¹ is selected from P1-1, P1-2, P1-3, P1-4, P1-5, P1-9, P1-10, P1-11, P1-12, P1-13, P1-14, P1-15, P1-22, P1-30, P1-33, P1-34, P1-38, P1-44, P1-45, P1-46, P1-47, P1-48, P1-49, P1-52, P1-54, P1-55, P1-56, P1-57, P1-59, P1-61, P1-63, P1-65, P1-69, P1-70, P1-74, P1-75, P1-76, P1-78, P1-81, P1-82, P1-86, P1-92, P1-99, P1-103, P1-104, P1-105, P1-109, P1-110, P1-119, P1-125, P1-126, P1-131, P1-132, P1-137, P1-138 and P1-139, more specifically selected from P1-1, P1-2, P1-3, P1-4, P1-5, P1-9, P1-10, P1-11, P1-14, P1-15, P1-22, P1-30, P1-33, P1-34, P1-38, P1-44, P1-45, P1-46, P1-47, P1-48, P1-59, P1-61, P1-63, P1-65, P1-69, P1-70, P1-74, P1-75, P1-76, P1-78, P1-86, P1-92, P1-99, P1-103, P1-104, P1-105, P1-109, P1-110, P1-119, P1-125, P1-126, P1-131, P1-132, P1-137, P1-138and P1-139.

R^{1a} are the possible substituents for the aliphatic moieties of R¹.

R^{1a} according to the invention is independently selected from halogen, OH, CN, C₁-C₄-alkoxy, C₃-C₆-cycloalkyl, C₃-C₆-halocycloalkyl and C₁-C₄-halogenalkoxy.

According to one embodiment R^{1a} is independently selected from halogen, OH, CN, C₁-C₂-alkoxy, C₃-C₆-cycloalkyl, C₃-C₆-halocycloalkyl and C₁-C₂-halogenalkoxy. Specifically, R^{1a} is independently selected from F, Cl, OH, CN, C₁-C₂-alkoxy, cyclopropyl, 1-F-cyclopropyl, 1-Cl-cyclopropyl and C₁-C₂-halogenalkoxy.

According to a further embodiment, R^{1a} is independently selected from OH, C₃-C₆-cycloalkyl, C₃-C₆-halocycloalkyl and C₁-C₂-halogenalkoxy. Specifically, R^{1a} is independently selected from OH, cyclopropyl and C₁-C₂-halogenalkoxy.

R^{1b} are the possible substituents for the cycloalkyl moieties of R¹.

R^{1b} according to the invention is independently selected from halogen, OH, CN, C₁-C₄-alkyl, C₁-C₄-alkoxy, C₁-C₄-halogenalkyl, C₃-C₆-cycloalkyl, C₃-C₆-halocycloalkyl and C₁-C₄-halogenalkoxy.

According to one embodiment thereof R^{1b} is independently selected from halogen, CN, C₁-C₂-alkyl, C₁-C₂-alkoxy, C₁-C₂-halogenalkyl, C₃-C₆-cycloalkyl, C₃-C₆-halocycloalkyl and C₁-C₂-halogenalkoxy. Specifically, R^{1b} is independently selected from F, Cl, OH, CN, CH₃, OCH₃, cyclopropyl, 1-F-cyclopropyl, 1-Cl-cyclopropyl and halogenmethoxy.

According to a further embodiment thereof R^{1b} is independently selected from C₁-C₂-alkyl, C₁-C₂-alkoxy, C₁-C₂-halogenalkyl, C₃-C₆-cycloalkyl, C₃-C₆-halocycloalkyl and C₁-C₂-halogenalkoxy. Specifically, R^{1b} is independently selected from OH, CH₃, OCH₃, cyclopropyl, 1-F-cyclopropyl, 1-Cl-cyclopropyl and halogenmethoxy, more specifically independently selected from OH, CH₃, OCH₃, cyclopropyl, 1-F-cyclopropyl, 1-Cl-cyclopropyl and OCHF₂.

According to the invention, R² is hydrogen, C₁-C₄-alkyl, C₂-C₄-alkenyl, C₂-C₄-alkynyl, wherein the aliphatic moieties of R² are not further substituted or carry one, two, three or up to the maximum possible number of identical or different groups R^{2a} which independently of one another are selected from halogen, OH, CN, C₁-C₄-alkoxy, C₃-C₆-cycloalkyl, C₃-C₆-halocycloalkyl and C₁-C₄-halogenalkoxy.

According to one embodiment, R² is H.

According to a further embodiment of the invention, R² is selected from C₁-C₄-alkyl, C₂-C₄-alkenyl and C₂-C₄-alkynyl.

According to a further embodiment of the invention, R² is selected from H, C₁-C₄-alkyl, in particular methyl or ethyl, C₂-C₄-alkenyl, in particular CH₂CH=CH₂, and C₂-C₄-alkynyl, in particular CH₂C≡CH. Specific embodiments thereof can be found in the below Table P2.

According to one particular embodiment, R² is C₁-C₄-alkyl, such as CH₃, C₂H₅, CH(CH₃)₂, CH₂CH₂CH₃, CH₂CH₂CH₂CH₃, CH₂CH(CH₃)₂.

A further embodiment relates to compounds, wherein R² is C₁-C₄-alkyl, that is substituted by one, two or three or up to the maximum possible number of identical or different groups R^{2a}, as defined and preferably defined herein. According to a specific embodiment thereof, R² is C₁-C₄-haloalkyl, more particularly C₁-C₂-haloalkyl. According to a further specific embodiment thereof, R² is C₁-C₄-alkoxy-C₁-C₄-alkyl, such as CH₂OCH₃ or CH₂CH₂OCH₃. According to still a further specific embodiment thereof, R² is hydroxyl-C₁-C₄-alkyl, such as CH₂CH₂OH. Further specific embodiments thereof can be found in the below Table P2.

According to still another embodiment, R² is C₃-C₆-cycloalkyl-C₁-C₄-alkyl. A further embodiment relates to compounds, wherein R² is C₃-C₆-cycloalkyl-C₁-C₄-alkyl, more particularly C₃-C₆-cycloalkyl-C₁-C₂-alkyl that is substituted by one, two or three or up to the maximum possible number of identical or different groups R^{2a}. A further embodiment relates to compounds, wherein R² is C₃-C₆-halocycloalkyl-C₁-C₄-alkyl, more particularly C₃-C₆-halocycloalkyl-C₁-C₂-alkyl. Specific embodiments thereof can be found in the below Table P2.

According to another embodiment, R² is C₂-C₄-alkenyl, such as CH₂CH=CH₂, CH₂C(CH₃)=CH₂ or CH₂CH=CHCH₃. A further embodiment relates to compounds, wherein R² is C₂-C₄-alkenyl that is substituted by one, two or three or up to the maximum possible number of identical or different groups R^{2a} as defined and preferably defined herein. According to a specific embodiment thereof, R² is C₂-C₄-haloalkenyl, such as CH₂C(Cl)=CH₂ and CH₂C(H)=CHCl. According to a further specific embodiment thereof, R² is C₃-C₆-cycloalkyl-C₂-C₄-alkenyl or C₃-C₆-halocycloalkyl-C₂-C₄-alkenyl. Further specific embodiments thereof can be found in the below Table P2.

According to still another embodiment, R² is C₂-C₄-alkynyl, such as CH₂C≡CH or CH₂C≡CCH₃. A further embodiment relates to compounds, wherein R² is C₂-C₄-alkynyl that is substituted by one, two or three or up to the maximum possible number of identical or different groups R^{2a}, as defined and preferably defined herein. According to a specific embodiment thereof, R² is C₂-C₄-haloalkynyl. According to a further specific embodiment thereof, R² is C₃-C₆-cycloalkyl-C₂-C₄-alkynyl or C₃-C₆-halocycloalkyl-C₂-C₄-alkynyl. Specific embodiments thereof can be found in the below Table P2.

Particularly preferred embodiments of R² according to the invention are in Table P2 below, wherein each line of lines P2-1 to P2-79 corresponds to one particular embodiment of the invention, wherein P2-1 to P2-79 are also in any combination a preferred embodiment of the present invention.

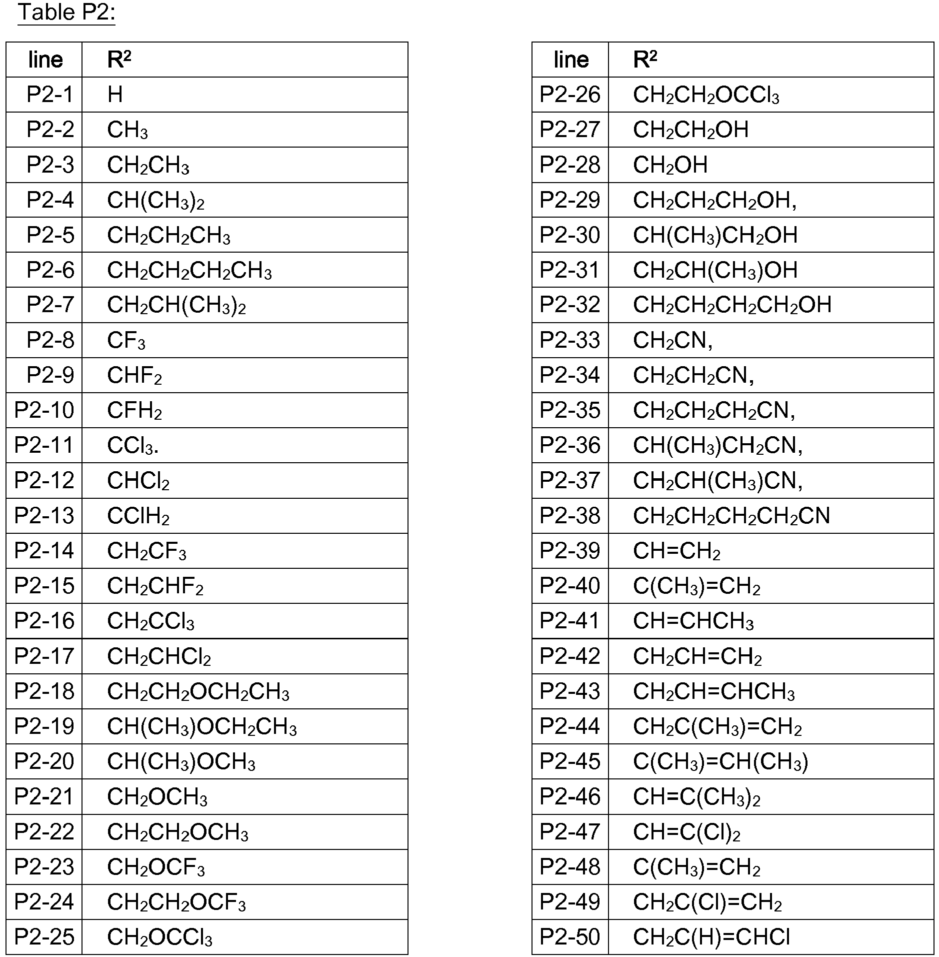

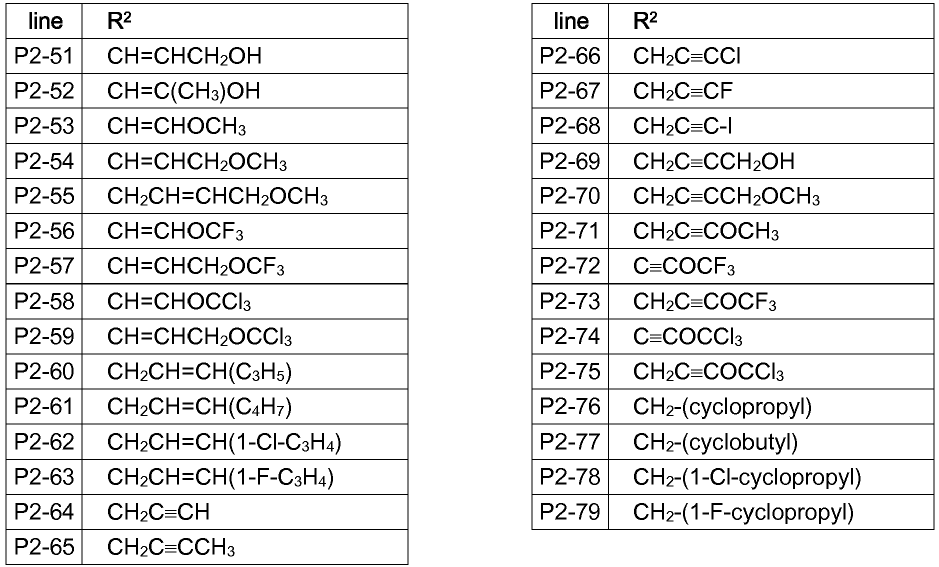

R³ according to the present invention is independently selected from halogen, CN, C₁-C₄-alkyl, C₁-C₄-alkoxy, C₂-C₄-alkenyl, C₂-C₄-alkynyl, C₃-C₆-cycloalkyl and S(O)ₚ(C₁-C₄-alkyl), wherein each of R³ is unsubstituted or further substituted by one, two, three or four R^{3a}; wherein R^{3a} is independently selected from halogen, CN, OH, C₁-C₄-alkyl, C₁-C₄-halogenalkyl, C₃-C₆-cycloalkyl, C₃-C₆-halogencycloalkyl, C₁-C₄-alkoxy and C₁-C₄-halogenalkoxy, and wherein p is 0, 1 or 2.

R³ according to one further embodiment is independently selected from halogen, CN, C₁-C₄-alkyl, C₁-C₄-alkoxy, C₂-C₄-alkenyl, C₂-C₄-alkynyl, C₃-C₆-cycloalkyl and S(O)ₚ(C₁-C₄-alkyl), wherein each of R³ is unsubstituted or further substituted by one, two, three or four R^{3a}; wherein R^{3a} is independently selected from halogen, CN, OH, C₁-C₄-alkyl, C₁-C₄-halogenalkyl, C₃-C₈-cycloalkyl, C₃-C₈-halogencycloalkyl, C₁-C₄-alkoxy and C₁-C₄-halogenalkoxy, and wherein p is 0, 1 or 2.

According to still a further embodiment, R³ is selected from F, Cl, Br, CN, C₁-C₄-alkyl, C₁-C₄-halogenalkyl, C₁-C₄-alkoxy, C₁-C₄-halogenalkoxy, S(C₁-C₄-alkyl), S(O)(C₁-C₄-alkyl) and S(O)₂(C₁-C₄-alkyl).

According to still a further embodiment, R³ is selected from Cl, F, Br, CN, C₁-C₂-alkyl, in particular CH₃, C₁-C₂-haloalkyl, in particular CF₃, C₁-C₂-alkoxy, in particular OCH₃, and C₁-C₂-halogenalkoxy, in particular OCF₃.

According to a further embodiment, R³ is selected from C₂-C₄-alkenyl, C₂-C₄-halogenalkenyl, C₂-C₄-alkynyl and C₂-C₄-halogenalkynyl. According to one particular embodiment, R³ is C₂-C₄-alkenyl or C₂-C₄-halogenalkenyl, such as CH=CH₂. According to a further particular embodiment, R³ is C₂-C₄-alkynyl or C₂-C₄-halogenalkynyl, such as CΞCH.

According to a further embodiment, R³ is selected from C₃-C₆-cycloalkyl and C₃-C₆-halogencycloalkyl.

According to a further embodiment, R³ is selected from S(C₁-C₂-alkyl), S(O)(C₁-C₂-alkyl) and S(O)₂(C₁-C₂-alkyl). According to a particular embodiment thereof, R³ is selected from SCH₃, S(O)(CH₃) and S(O)₂(CH₃).

According to one further specific embodiment, R³ is halogen, in particular Br, F or Cl, more specifically F or Cl.

According to a further specific embodiment, R³ is CN.

According to a further specific embodiment, R³ is C₁-C₄-alkyl, such as CH₃, or C₁-C₄-haloalkyl, such as CF₃, CHF₂, CH₂F, CCl₃, CHCl₂ or CH₂Cl.

According to a further specific embodiment, R³ is C₁-C₄-alkoxy, more specifically C₁-C₂-alkoxy such as OCH₃ or OCH₂CH₃, or C₁-C₄-haloalkoxy, more specifically C₁-C₂-haloalkoxy such as OCF₃, OCHF₂, OCH₂F, OCCl₃, OCHCl₂ or OCH₂Cl, in particular OCF₃, OCHF₂, OCCl₃ or OCHCl₂.

R^{3a} is selected from halogen, CN, OH, C₁-C₄-alkyl, C₁-C₄-haloalkyl, C₃-C₈-cycloalkyl, C₃-C₈-halocycloalkyl, C₁-C₄-alkoxy and C₁-C₄-halogenalkoxy, in particular selected from halogen, CN, C₁-C₂-alkyl, C₁-C₂-haloalkyl, C₃-C₆-cycloalkyl, C₃-C₆-halocycloalkyl, C₁-C₂-alkoxy and C₁-C₂-halogenalkoxy. Specifically, R^{3a} is independently selected from F, Cl, CN, OH, CH₃, halomethyl, cyclopropyl, halocyclopropyl, OCH₃ and halogenmethoxy.

Particularly preferred embodiments of R³ according to the invention are in Table P3 below, wherein each line of lines P3-1 to P3-15 corresponds to one particular embodiment of the invention, wherein P3-1 to P3-15 are also in any combination with one another a preferred embodiment of the present invention. Thereby, for every R³ that is present in the inventive compounds, these specific embodiments and preferences apply independently of the meaning of any other R³ that may be present in the phenyl ring:

**Table P3:**

| **No.** | **R³** |
|---|---|
| P3-1 | Cl |
| P3-2 | F |
| P3-3 | CN |
| P3-4 | CH₃ |
| P3-5 | CH₂CH₃ |
| P3-6 | CF₃ |
| P3-7 | CHF₂ |
| P3-8 | OCH₃ |
| P3-9 | OCH₂CH₃ |
| P3-10 | OCF₃ |
| P3-11 | OCHF₂ |
| P3-12 | SCH₃ |
| P3-13 | SOCH₃ |
| P3-14 | SO₂CH₃ |
| P3-15 | Br |

One embodiment of the invention relates to compounds I, wherein A is N (I.A).

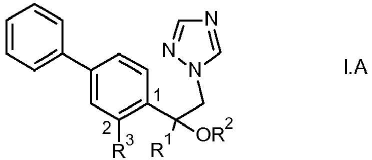

One particular embodiment thereof relates to compounds I.A, wherein R³ is Cl, corresponding to compounds I.A1. One further particular embodiment relates to compounds I.A, wherein R³ is F, corresponding to compounds I.A2. Still one further particular embodiment relates to compounds I.A, wherein R³ is Br, corresponding to compounds I.A3. Still one further particular embodiment relates to compounds I.A, wherein R³ is CF₃, corresponding to compounds I.A4. Still one further particular embodiment relates to compounds I.A, wherein R³ is CH₃, corresponding to compounds I.A5. Still one further particular embodiment relates to compounds I.A, wherein R³ is OCH₃, corresponding to compounds I.A6.

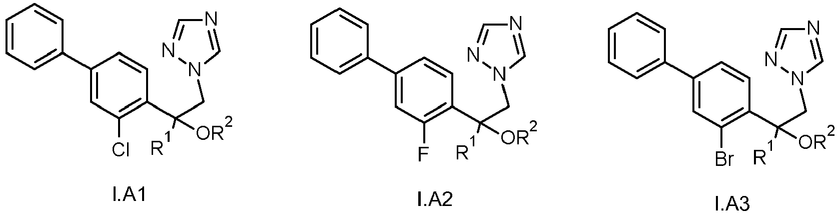

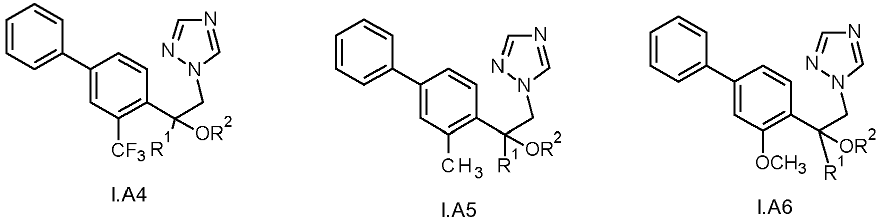

A further embodiment relates to compounds I, wherein A is CH (I.B).

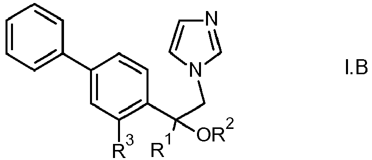

One particular embodiment thereof relates to compounds I.B, wherein R³ is Cl, corresponding to compounds I.B1. One further particular embodiment relates to compounds I.B, wherein R³ is F, corresponding to compounds I.B2. Still one further particular embodiment relates to compounds I.B, wherein R³ is Br, corresponding to compounds I.B3. Still one further particular embodiment relates to compounds I.B, wherein R³ is CF₃, corresponding to compounds I.B4. Still one further particular embodiment relates to compounds I.B, wherein R³ is CH₃, corresponding to compounds I.B5. Still one further particular embodiment relates to compounds I.B, wherein R³ is OCH₃, corresponding to compounds I.B6.

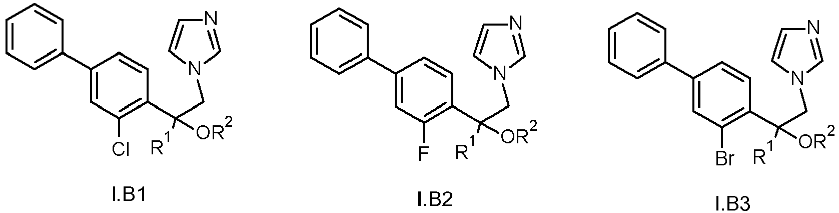

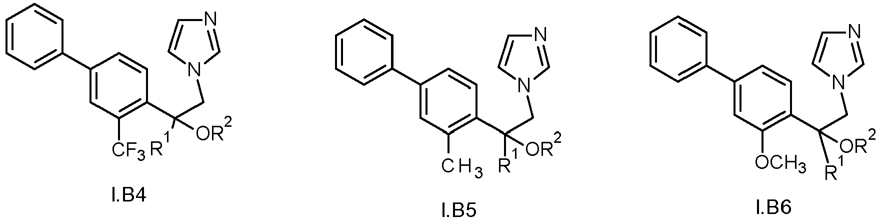

In particular with a view to their use, according to one embodiment, preference is given to the compounds of the formula I.A1, I.A2, I.A3, I.A4, I.A5 and I.A6 that are compiled in the Tables 1a, 1b, 1c, 1d, 1e and 1f. Each of the groups mentioned for a substituent in the tables is furthermore per se, independently of the combination in which it is mentioned, a particularly preferred aspect of the substituent in question.

Table 1a Compounds of the formula I.A1 in which the meaning for the combination of R¹ and R² for each individual compound corresponds in each case to one line of Table B (compounds I.A1.B-1 to I.A1.B-455).

Table 1b Compounds of the formula I.A2 in which the meaning for the combination of R¹ and R² for each individual compound corresponds in each case to one line of Table B (compounds I.A2.B-1 to I.A2.B-455).

Table 1c Compounds of the formula I.A3 in which the meaning for the combination of R¹ and R² for each individual compound corresponds in each case to one line of Table B (compounds I.A3.B-1 to I.A3.B-455).

Table 1d Compounds of the formula I.A4 in which the meaning for the combination of R¹ and R² for each individual compound corresponds in each case to one line of Table B (compounds I.A4.B-1 to I.A4.B-455).

Table 1e Compounds of the formula I.A5 in which the meaning for the combination of R¹ and R² for each individual compound corresponds in each case to one line of Table B (compounds I.A5.B-1 to I.A5.B-455).

Table 1f Compounds of the formula I.A6 in which the meaning for the combination of R¹ and R² for each individual compound corresponds in each case to one line of Table B (compounds I.A6.B-1 to I.A6.B-455).

In particular with a view to their use, according to a further embodiment, preference is given to the compounds of the formula I.B1, I.B2, I.B3, I.B4, I.B5 and I.B6 that are compiled in the Tables 2a, 2b, 2c, 2d, 2e and 2f. Each of the groups mentioned for a substituent in the tables is furthermore per se, independently of the combination in which it is mentioned, a particularly preferred aspect of the substituent in question.

Table 2a Compounds of the formula I.B1 in which the meaning for the combination of R¹ and R² for each individual compound corresponds in each case to one line of Table B (compounds I.B1.B-1 to I.B1.B-455).

Table 2b Compounds of the formula I.B2 in which the meaning for the combination of R¹ and R² for each individual compound corresponds in each case to one line of Table B (compounds I.B2.B-1 to I.B2.B-455).

Table 2c Compounds of the formula I.B3 in which the meaning for the combination of R¹ and R² for each individual compound corresponds in each case to one line of Table B (compounds I.B3.B-1 to I.B3.B-455).

Table 2d Compounds of the formula I.B4 in which the meaning for the combination of R¹ and R² for each individual compound corresponds in each case to one line of Table B (compounds I.B4.B-1 to I.B4.B-455).

Table 2e Compounds of the formula I.B5 in which the meaning for the combination of R¹ and R² for each individual compound corresponds in each case to one line of Table B (compounds I.B5.B-1 to I.B5.B-455).

Table 2f Compounds of the formula I.B6 in which the meaning for the combination of R¹ and R² for each individual compound corresponds in each case to one line of Table B (compounds I.B6.B-1 to I.B6.B-455).

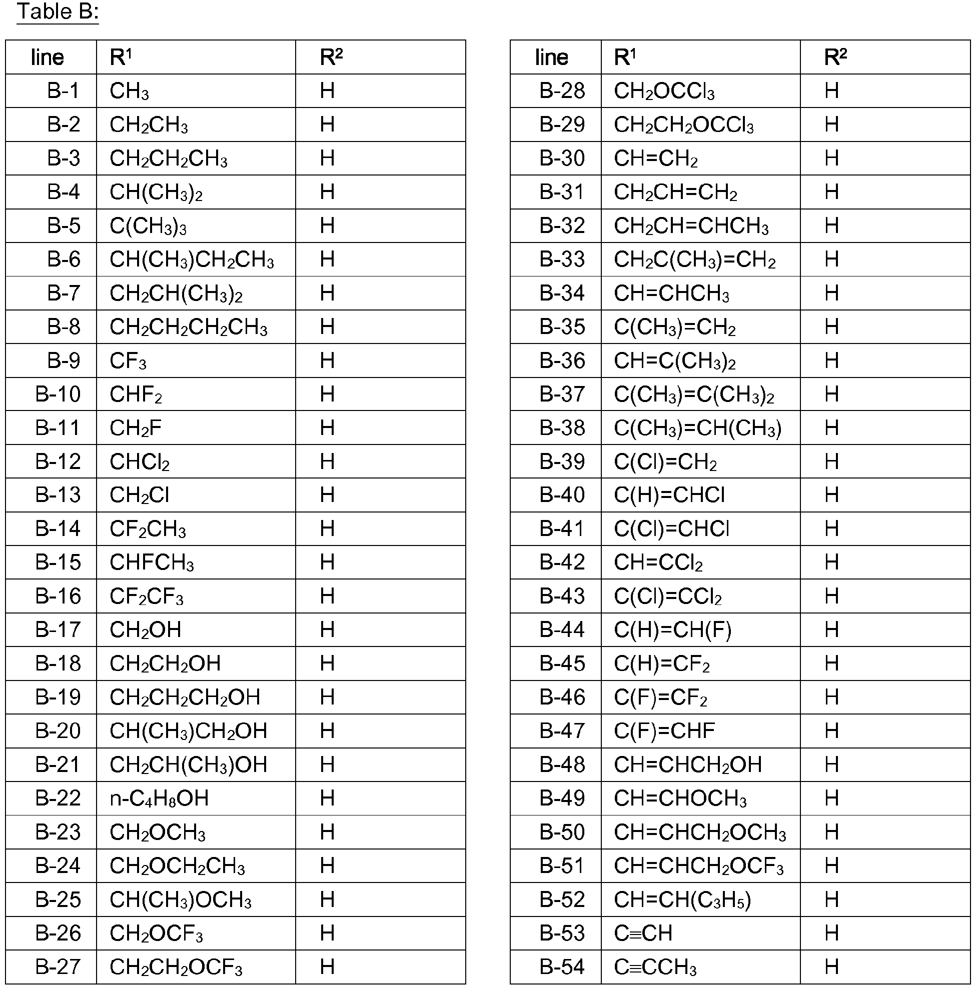

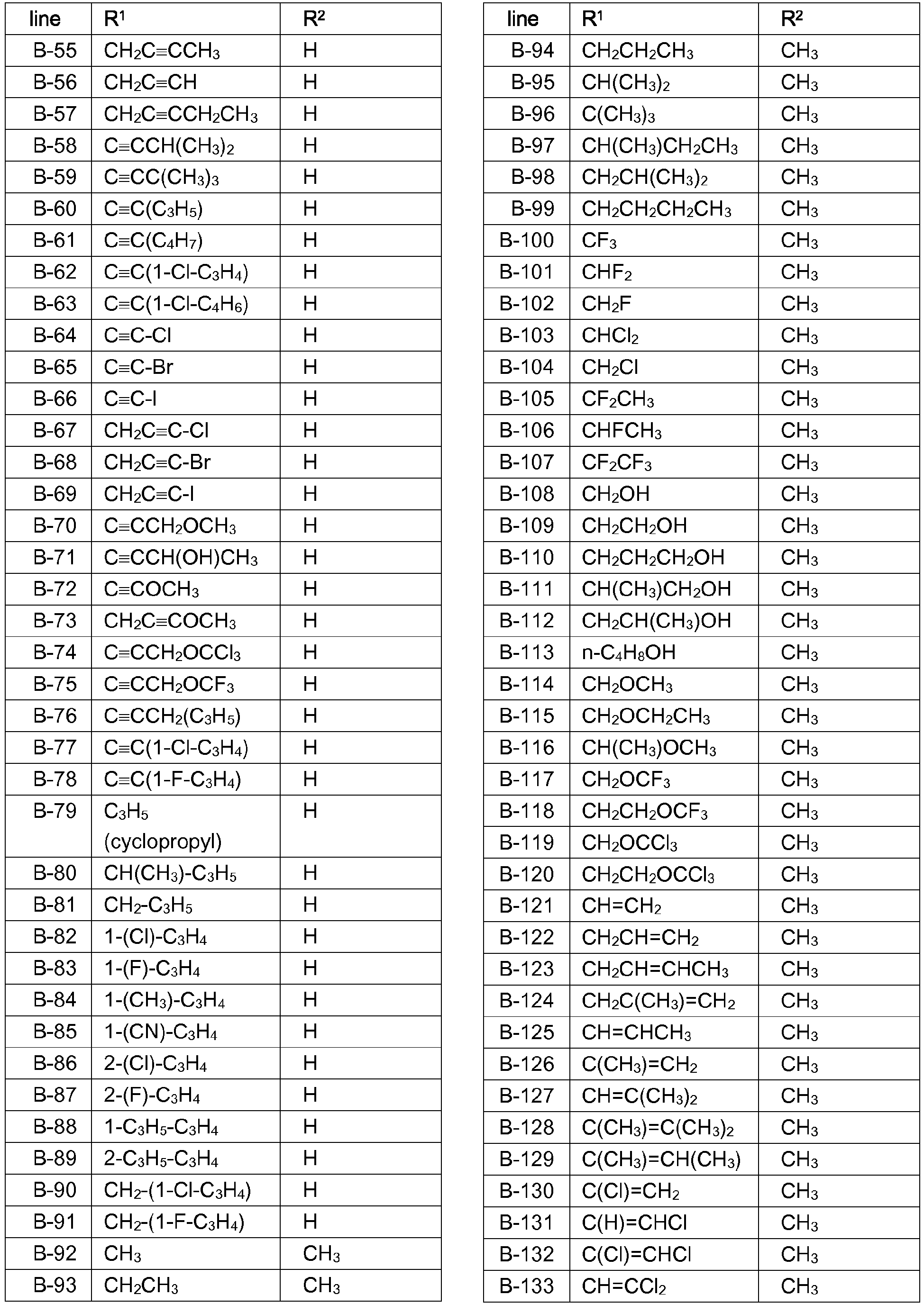

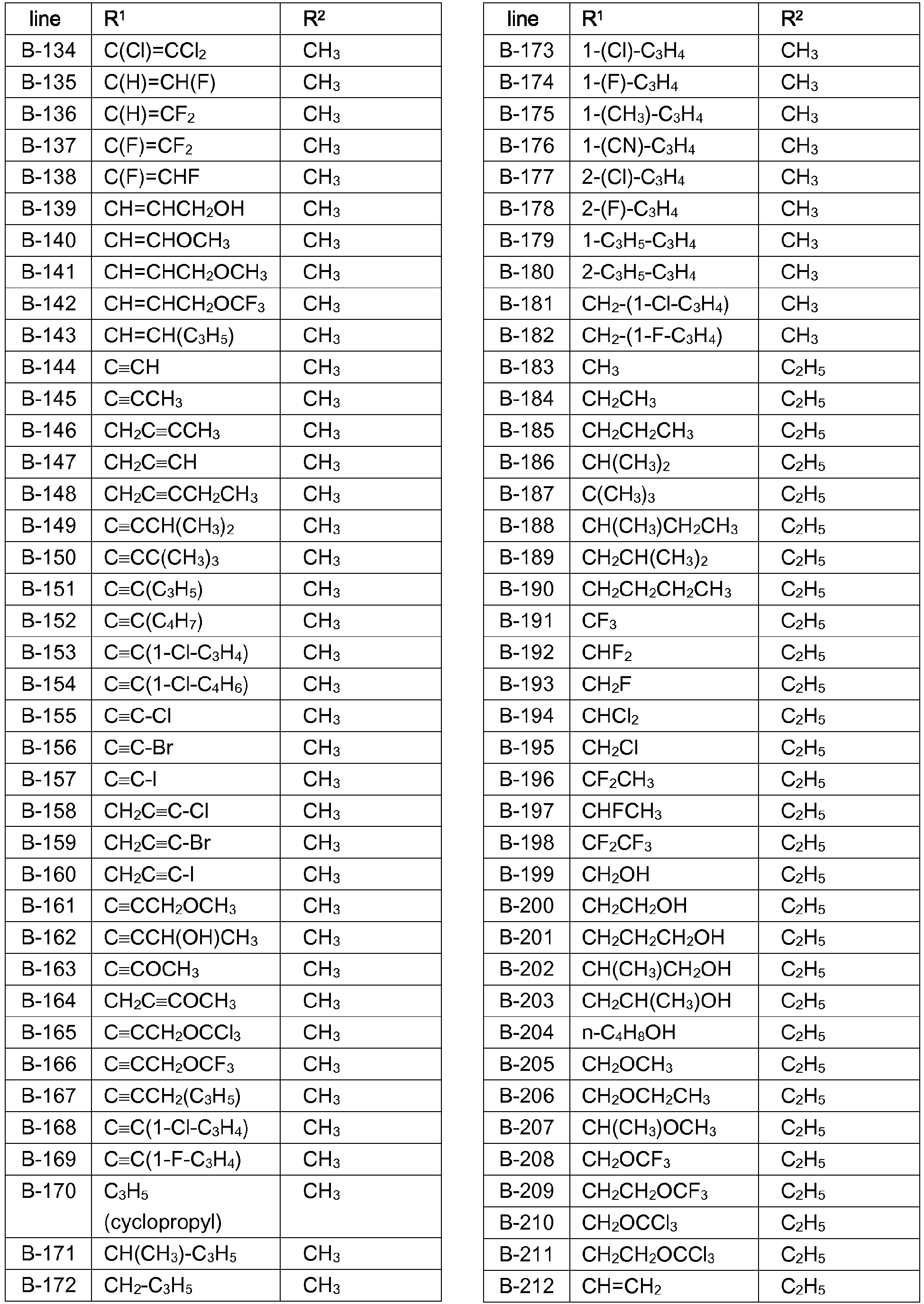

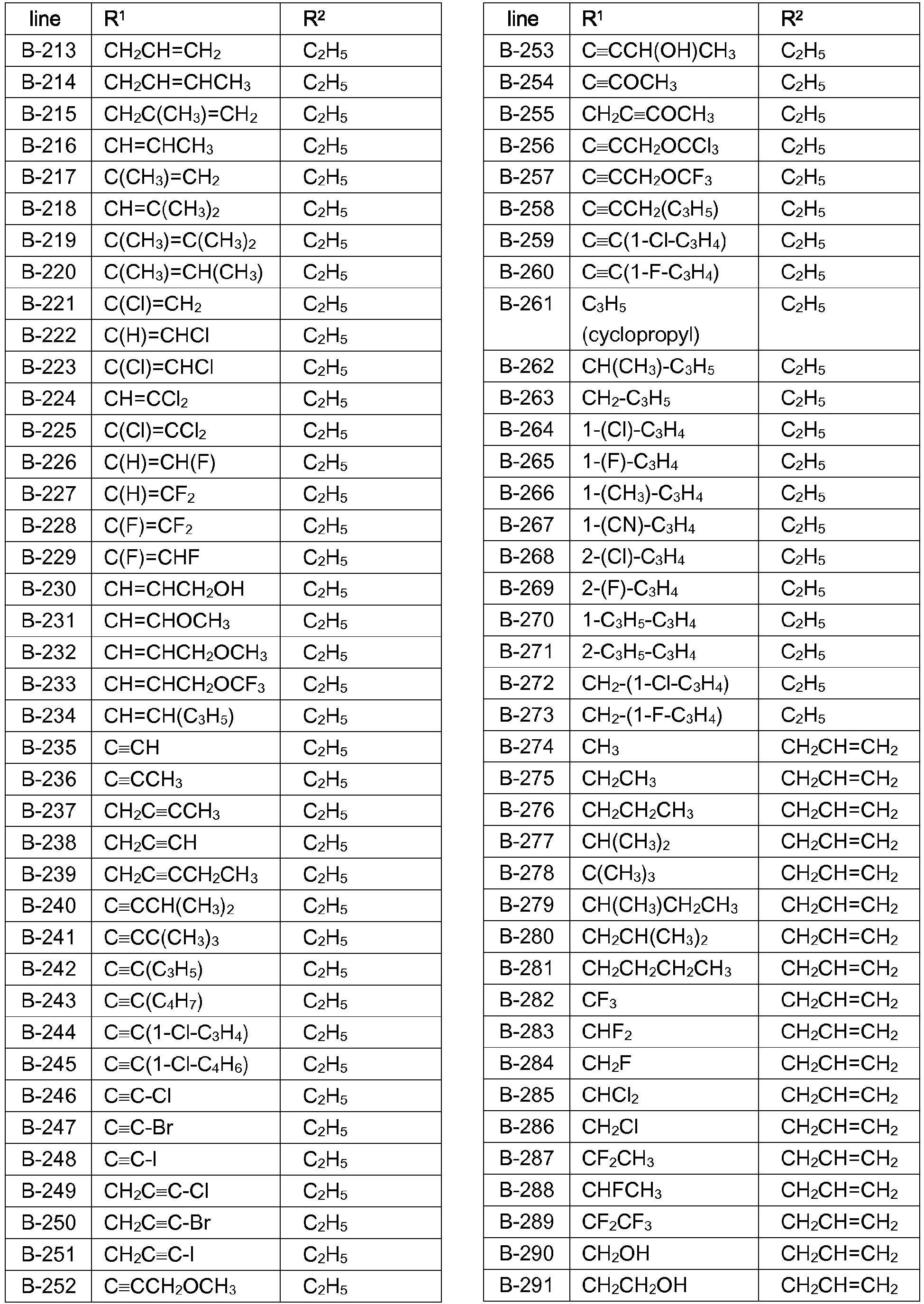

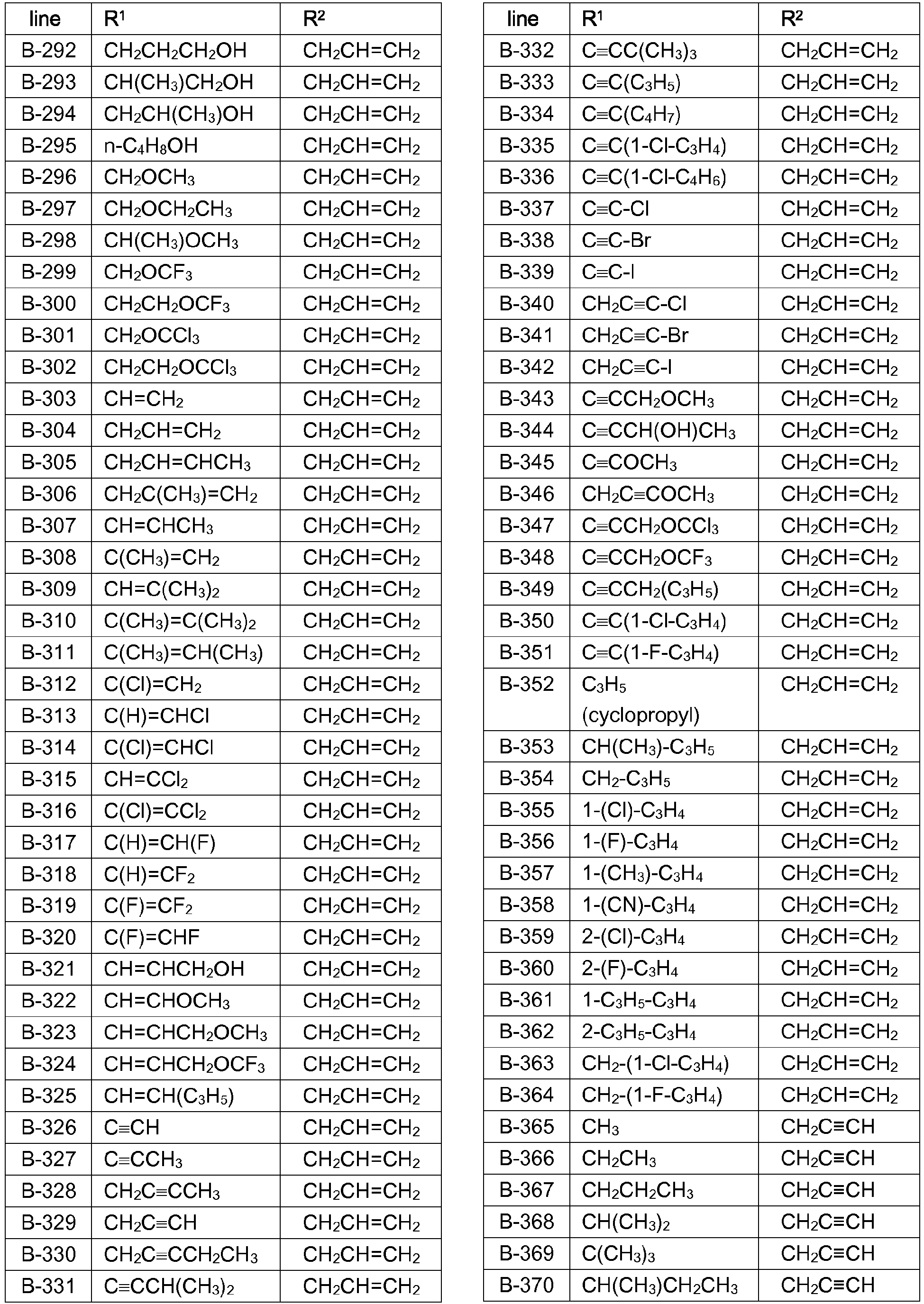

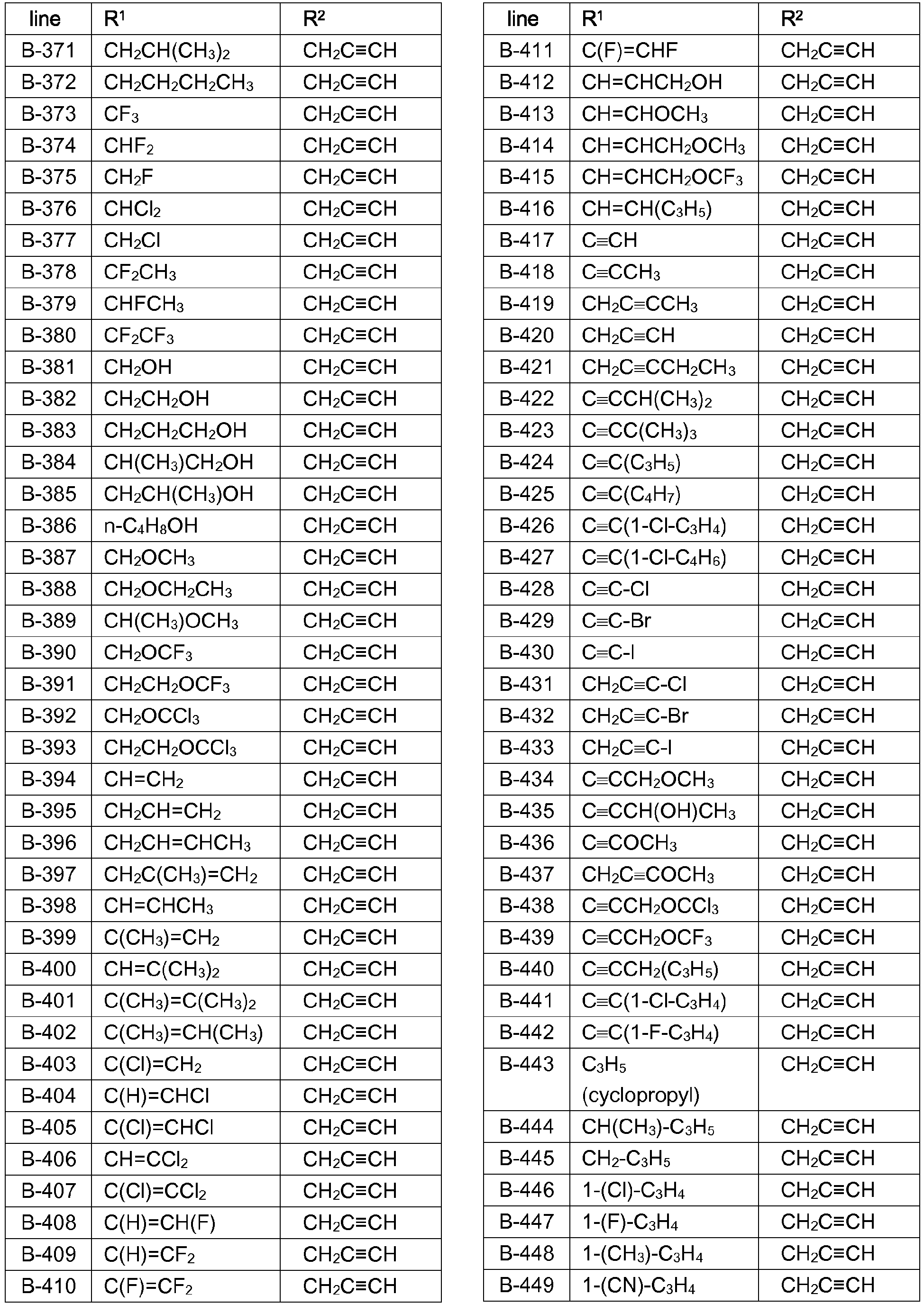

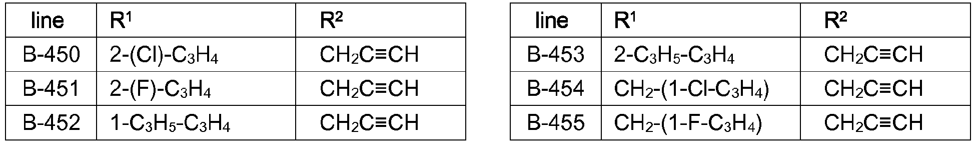

A further aspect are compounds I.C: 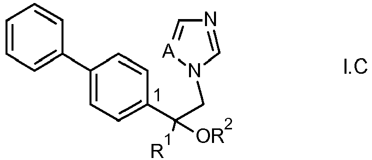 wherein A, R¹ and R² are asdefined and preferably defined for compounds I above.

According to one embodiment, A is N (compounds I.C1). According to a further embodiment, A is CH (compounds I.C2):

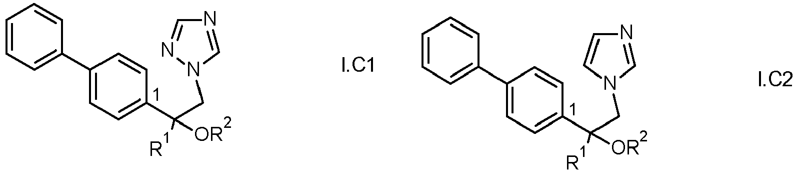

At last some of the compounds I.C, in particular I.C1 and I.C2, are novel and also an aspect of the invention.

In particular with a view to their use, according to a specific embodiment, preference is given to the compounds of the formula I.C1, in Table 3a. Each of the groups mentioned for a substituent in the tables is furthermore per se, independently of the combination in which it is mentioned, a particularly preferred aspect of the substituent in question.

Table 3a Compounds of the formula I.C1 in which the meaning for the combination of R¹ and R² for each individual compound corresponds in each case to one line of Table B (compounds I.C1.B-1 to I.C1.B-455).

In particular with a view to their use, according to a further specific embodiment, preference is given to the compounds of the formula I.C2, in Table 4a. Each of the groups mentioned for a substituent in the tables is furthermore per se, independently of the combination in which it is mentioned, a particularly preferred aspect of the substituent in question.

Table 4a Compounds of the formula I.C2 in which the meaning for the combination of R¹ and R² for each individual compound corresponds in each case to one line of Table B (compounds I.C2.B-1 to I.C2.B-455).

The compounds I.C, in particular I.C1 and I.C2, and the compositions comprising the same, respectively, are suitable as fungicides. The following description for compounds I also applies to compounds I.C, I.C1 and I.C2.

The compounds I and the compositions according to the invention, respectively, are suitable as fungicides.

They are distinguished by an outstanding effectiveness against a broad spectrum of phytopathogenic fungi, including soil-borne fungi, which derive especially from the classes of the Plasmodiophoromycetes, Peronosporomycetes (syn. Oomycetes), Chytridiomycetes, Zygomycetes, Ascomycetes, Basidiomycetes and Deuteromycetes (syn. Fungi imperfecti). Some are systemically effective and they can be used in crop protection as foliar fungicides, fungicides for seed dressing and soil fungicides. Moreover, they are suitable for controlling harmful fungi, which inter alia occur in wood or roots of plants.

The compounds I and the compositions according to the invention are particularly important in the control of a multitude of phytopathogenic fungi on various cultivated plants, such as cereals, e. g. wheat, rye, barley, triticale, oats or rice; beet, e. g. sugar beet or fodder beet; fruits, such as pomes, stone fruits or soft fruits, e. g. apples, pears, plums, peaches, almonds, cherries, strawberries, raspberries, blackberries or gooseberries; leguminous plants, such as lentils, peas, alfalfa or soybeans; oil plants, such as rape, mustard, olives, sunflowers, coconut, cocoa beans, castor oil plants, oil palms, ground nuts or soybeans; cucurbits, such as squashes, cucumber or melons; fiber plants, such as cotton, flax, hemp or jute; citrus fruit, such as oranges, lemons, grapefruits or mandarins; vegetables, such as spinach, lettuce, asparagus, cabbages, carrots, onions, tomatoes, potatoes, cucurbits or paprika; lauraceous plants, such as avocados, cinnamon or camphor; energy and raw material plants, such as corn, soybean, rape, sugar cane or oil palm; corn; tobacco; nuts; coffee; tea; bananas; vines (table grapes and grape juice grape vines); hop; turf; sweet leaf (also called Stevia); natural rubber plants or ornamental and forestry plants, such as flowers, shrubs, broad-leaved trees or evergreens, e. g. conifers; and on the plant propagation material, such as seeds, and the crop material of these plants.

Preferably, compounds I and compositions thereof, respectively are used for controlling a multitude of fungi on field crops, such as potatoes sugar beets, tobacco, wheat, rye, barley, oats, rice, corn, cotton, soybeans, rape, legumes, sunflowers, coffee or sugar cane; fruits; vines; ornamentals; or vegetables, such as cucumbers, tomatoes, beans or squashes.

The term "plant propagation material" is to be understood to denote all the generative parts of the plant such as seeds and vegetative plant material such as cuttings and tubers (e. g. potatoes), which can be used for the multiplication of the plant. This includes seeds, roots, fruits, tubers, bulbs, rhizomes, shoots, sprouts and other parts of plants, including seedlings and young plants, which are to be transplanted after germination or after emergence from soil. These young plants may also be protected before transplantation by a total or partial treatment by immersion or pouring.

Preferably, treatment of plant propagation materials with compounds I and compositions thereof, respectively, is used for controlling a multitude of fungi on cereals, such as wheat, rye, barley and oats; rice, corn, cotton and soybeans.

The term "cultivated plants" is to be understood as including plants which have been modified by breeding, mutagenesis or genetic engineering including but not limiting to agricultural biotech products on the market or in development (cf. http://cera-gmc.org/, see GM crop database therein). Genetically modified plants are plants, which genetic material has been so modified by the use of recombinant DNA techniques that under natural circumstances cannot readily be obtained by cross breeding, mutations or natural recombination. Typically, one or more genes have been integrated into the genetic material of a genetically modified plant in order to improve certain properties of the plant. Such genetic modifications also include but are not limited to targeted post-translational modification of protein(s), oligo- or polypeptides e. g. by glycosylation or polymer additions such as prenylated, acetylated or farnesylated moieties or PEG moieties.

Plants that have been modified by breeding, mutagenesis or genetic engineering, e. g. have been rendered tolerant to applications of specific classes of herbicides, such as auxin herbicides such as dicamba or 2,4-D; bleacher herbicides such as hydroxylphenylpyruvate dioxygenase (HPPD) inhibitors or phytoene desaturase (PDS) inhibitors; acetolactate synthase (ALS) inhibitors such as sulfonyl ureas or imidazolinones; enolpyruvylshikimate-3-phosphate synthase (EPSPS) inhibitors, such as glyphosate; glutamine synthetase (GS) inhibitors such as glufosinate; protoporphyrinogen-IX oxidase inhibitors; lipid biosynthesis inhibitors such as acetyl CoA carboxylase (ACCase) inhibitors; or oxynil (i. e. bromoxynil or ioxynil) herbicides as a result of conventional methods of breeding or genetic engineering. Furthermore, plants have been made resistant to multiple classes of herbicides through multiple genetic modifications, such as resistance to both glyphosate and glufosinate or to both glyphosate and a herbicide from another class such as ALS inhibitors, HPPD inhibitors, auxin herbicides, or ACCase inhibitors. These herbicide resistance technologies are e. g. described in Pest Managem. Sci. 61, 2005, 246; 61, 2005, 258; 61, 2005, 277; 61, 2005, 269; 61, 2005, 286; 64, 2008, 326; 64, 2008, 332; Weed Sci. 57, 2009, 108; Austral. J. Agricult. Res. 58, 2007, 708; Science 316, 2007, 1185; and references quoted therein. Several cultivated plants have been rendered tolerant to herbicides by conventional methods of breeding (mutagenesis), e. g. Clearfield® summer rape (Canola, BASF SE, Germany) being tolerant to imidazolinones, e. g. imazamox, or ExpressSun® sunflowers (DuPont, USA) being tolerant to sulfonyl ureas, e. g. tribenuron. Genetic engineering methods have been used to render cultivated plants such as soybean, cotton, corn, beets and rape, tolerant to herbicides such as glyphosate and glufosinate, some of which are commercially available under the trade names RoundupReady® (glyphosate-tolerant, Monsanto, U.S.A.), Cultivance® (imidazolinone tolerant, BASF SE, Germany) and LibertyLink® (glufosinate-tolerant, Bayer CropScience, Germany).

Furthermore, plants are also covered that are by the use of recombinant DNA techniques capable to synthesize one or more insecticidal proteins, especially those known from the bacterial genus *Bacillus*, particularly from *Bacillus thuringiensis*, such as δ-endotoxins, e. g. CryIA(b), CryIA(c), CryIF, CryIF(a2), CryIIA(b), CryIIIA, CryIIIB(b1) or Cry9c; vegetative insecticidal proteins (VIP), e. g. VIP1, VIP2, VIP3 or VIP3A; insecticidal proteins of bacteria colonizing nematodes, e. g. *Photorhabdus* spp. or *Xenorhabdus* spp.; toxins produced by animals, such as scorpion toxins, arachnid toxins, wasp toxins, or other insect-specific neurotoxins; toxins produced by fungi, such Streptomycetes toxins, plant lectins, such as pea or barley lectins; agglutinins; proteinase inhibitors, such as trypsin inhibitors, serine protease inhibitors, patatin, cystatin or papain inhibitors; ribosome-inactivating proteins (RIP), such as ricin, maize-RIP, abrin, luffin, saporin or bryodin; steroid metabolism enzymes, such as 3-hydroxysteroid oxidase, ecdysteroid-IDP-glycosyl-transferase, cholesterol oxidases, ecdysone inhibitors or HMG-CoA-reductase; ion channel blockers, such as blockers of sodium or calcium channels; juvenile hormone esterase; diuretic hormone receptors (helicokinin receptors); stilbene synthase, bibenzyl synthase, chitinases or glucanases. In the context of the present invention these insecticidal proteins or toxins are to be understood expressly also as pre-toxins, hybrid proteins, truncated or otherwise modified proteins. Hybrid proteins are characterized by a new combination of protein domains, (see, e. g. WO 02/015701). Further examples of such toxins or genetically modified plants capable of synthesizing such toxins are disclosed, e. g., in EP-A 374 753, WO 93/007278,

WO 95/34656, EP-A 427 529, EP-A 451 878, WO 03/18810 und WO 03/52073. The methods for producing such genetically modified plants are generally known to the person skilled in the art and are described, e. g. in the publications mentioned above. These insecticidal proteins contained in the genetically modified plants impart to the plants producing these proteins tolerance to harmful pests from all taxonomic groups of arthropods, especially to beetles (Coeloptera), two-winged insects (Diptera), and moths (Lepidoptera) and to nematodes (Nematoda). Genetically modified plants capable to synthesize one or more insecticidal proteins are, e. g., described in the publications mentioned above, and some of which are commercially available such as YieldGard® (corn cultivars producing the Cry1Ab toxin), YieldGard® Plus (corn cultivars producing Cry1Ab and Cry3Bb1 toxins), Starlink® (corn cultivars producing the Cry9c toxin), Herculex® RW (corn cultivars producing Cry34Ab1, Cry35Ab1 and the enzyme phosphinothricin-N-acetyltransferase [PAT]); NuCOTN® 33B (cotton cultivars producing the Cry1Ac toxin), Bollgard® I (cotton cultivars producing the Cry1Ac toxin), Bollgard® II (cotton cultivars producing Cry1Ac and Cry2Ab2 toxins); VIPCOT® (cotton cultivars producing a VIP-toxin); NewLeaf® (potato cultivars producing the Cry3A toxin); Bt-Xtra®, NatureGard®, KnockOut®, BiteGard®, Protecta®, Bt11 (e. g. Agrisure® CB) and Bt176 from Syngenta Seeds SAS, France, (corn cultivars producing the Cry1Ab toxin and PAT enyzme), MIR604 from Syngenta Seeds SAS, France (corn cultivars producing a modified version of the Cry3A toxin, c.f. WO 03/018810), MON 863 from Monsanto Europe S.A., Belgium (corn cultivars producing the Cry3Bb1 toxin), IPC 531 from Monsanto Europe S.A., Belgium (cotton cultivars producing a modified version of the Cry1Ac toxin) and 1507 from Pioneer Overseas Corporation, Belgium (corn cultivars producing the Cry1F toxin and PAT enzyme).

Furthermore, plants are also covered that are by the use of recombinant DNA techniques capable to synthesize one or more proteins to increase the resistance or tolerance of those plants to bacterial, viral or fungal pathogens. Examples of such proteins are the so-called "pathogenesis-related proteins" (PR proteins, see, e. g. EP-A 392 225), plant disease resistance genes (e. g. potato cultivars, which express resistance genes acting against *Phytophthora infestans* derived from the Mexican wild potato *Solanum bulbocastanum*) or T4-lysozym (e. g. potato cultivars capable of synthesizing these proteins with increased resistance against bacteria such as *Erwinia amylvora*). The methods for producing such genetically modified plants are generally known to the person skilled in the art and are described, e. g. in the publications mentioned above.

Furthermore, plants are also covered that are by the use of recombinant DNA techniques capable to synthesize one or more proteins to increase the productivity (e. g. bio mass production, grain yield, starch content, oil content or protein content), tolerance to drought, salinity or other growth-limiting environmental factors or tolerance to pests and fungal, bacterial or viral pathogens of those plants.

Furthermore, plants are also covered that contain by the use of recombinant DNA techniques a modified amount of substances of content or new substances of content, specifically to improve human or animal nutrition, e. g. oil crops that produce health-promoting long-chain omega-3 fatty acids or unsaturated omega-9 fatty acids (e. g. Nexera® rape, DOW Agro Sciences, Canada).

Furthermore, plants are also covered that contain by the use of recombinant DNA techniques a modified amount of substances of content or new substances of content, specifically to improve raw material production, e. g. potatoes that produce increased amounts of amylopectin (e. g. Amflora® potato, BASF SE, Germany).

The compounds I and compositions thereof, respectively, are particularly suitable for controlling the following plant diseases:
*Albugo* spp. (white rust) on ornamentals, vegetables (e. g. *A. candid*a) and sunflowers (e. g. *A. tragopogonis*); *Alternaria* spp. (Alternaria leaf spot) on vegetables, rape (*A. brassicola* or *brassicae*), sugar beets (*A. tenuis*), fruits, rice, soybeans, potatoes (e. g. *A. solani* or *A. alternata*), tomatoes (e. g. *A. solani* or *A. alternata*) and wheat; *Aphanomyces* spp. on sugar beets and vegetables; *Ascochyta* spp. on cereals and vegetables, e. g. *A. tritici* (anthracnose) on wheat and *A. hordei* on barley; *Bipolaris* and *Drechslera* spp. (teleomorph: *Cochliobolus* spp.), e. g. Southern leaf blight (*D. maydis)* or Northern leaf blight (*B. zeicola*) on corn, e. g. spot blotch (*B*. *sorokiniana*) on cereals and e. g. *B. oryzae* on rice and turfs; *Blumeria* (formerly *Erysiphe*) *graminis* (powdery mildew) on cereals (e. g. on wheat or barley); *Botrytis cinerea* (teleomorph: *Botryotinia fuckeliana*: grey mold) on fruits and berries (e. g. strawberries), vegetables (e. g. lettuce, carrots, celery and cabbages), rape, flowers, vines, forestry plants and wheat; *Bremia lactucae* (downy mildew) on lettuce; *Ceratocystis* (syn. *Ophiostoma*) spp. (rot or wilt) on broad-leaved trees and evergreens, e. g. *C. ulmi* (Dutch elm disease) on elms; *Cercospora* spp. (Cercospora leaf spots) on corn (e. g. Gray leaf spot: *C. zeae-maydis*), rice, sugar beets (e. g. *C. beticola*), sugar cane, vegetables, coffee, soybeans (e. g. *C. sojina* or *C. kikuchii*) and rice; *Cladosporium* spp. on tomatoes (e. g. *C. fulvum*: leaf mold) and cereals, e. g. *C. herbarum* (black ear) on wheat; *Claviceps purpurea* (ergot) on cereals; *Cochliobolus* (anamorph: *Helminthosporium* of *Bipolaris*) spp. (leaf spots) on corn (*C. carbonum*), cereals (e. g. *C. sativus*, anamorph: *B. sorokiniana*) and rice (e. g. *C. miyabeanus*, anamorph: *H. oryzae*); *Colletotrichum* (teleomorph: *Glomerella*) spp. (anthracnose) on cotton (e. g. *C. gossypii*), corn (e. g. *C. graminicola*: Anthracnose stalk rot), soft fruits, potatoes (e. g. *C. coccodes*: black dot), beans (e. g. *C. lindemuthianum*) and soybeans (e. g. *C. truncatum* or *C. gloeosporioides*); *Corticium* spp., e. g. *C. sasakii* (sheath blight) on rice; *Corynespora cassiicola* (leaf spots) on soybeans and ornamentals; *Cycloconium* spp., e. g. *C. oleaginum* on olive trees; *Cylindrocarpon* spp. (e. g. fruit tree canker or young vine decline, teleomorph: *Nectria* or *Neonectria* spp.) on fruit trees, vines (e. g. *C. liriodendri,* teleomorph: *Neonectria liriodendri*: Black Foot Disease) and ornamentals; *Dematophora* (teleomorph: *Rosellinia*) *necatrix* (root and stem rot) on soybeans; *Diaporthe* spp., e. g. *D. phaseolorum* (damping off) on soybeans; *Drechslera* (syn. *Helminthosporium*, teleomorph: *Pyrenophora*) spp. on corn, cereals, such as barley (e. g. *D. teres*, net blotch) and wheat (e. g. *D. tritici-repentis*: tan spot), rice and turf; Esca (dieback, apoplexy) on vines, caused by *Formitiporia* (syn. *Phellinus*) *punctata*, *F. mediterranea*, *Phaeomoniella chlamydospora* (earlier *Phaeoacremonium chlamydosporum*), *Phaeoacremonium aleophilum* and/or *Botryosphaeria obtusa*; *Elsinoe* spp. on pome fruits (*E. pyri*), soft fruits (*E. veneta*: anthracnose) and vines (*E. ampelina*: anthracnose); *Entyloma oryzae* (leaf smut) on rice; *Epicoccum* spp. (black mold) on wheat; *Erysiphe* spp. (powdery mildew) on sugar beets (*E. betae*), vegetables (e. g. *E. pisi*), such as cucurbits (e. g. *E. cichoracearum*), cabbages, rape (e. g. *E. cruciferarum*); *Eutypa lata* (Eutypa canker or dieback, anamorph: *Cytosporina lata*, syn. *Libertella blepharis*) on fruit trees, vines and ornamental woods; *Exserohilum* (syn. *Helminthosporium)* spp. on corn (e. g. *E. turcicum*); *Fusarium* (teleomorph: *Gibberella*) spp. (wilt, root or stem rot) on various plants, such as *F. graminearum* or *F. culmorum* (root rot, scab or head blight) on cereals (e. g. wheat or barley), *F. oxysporum* on tomatoes, *F. solani* (f. sp. *glycines* now syn. *F. virguliforme*) and *F. tucumaniae* and *F. brasiliense* each causing sudden death syndrome on soybeans, and *F. verticillioides* on corn; *Gaeumannomyces graminis* (take-all) on cereals (e. g. wheat or barley) and corn; *Gibberella* spp. on cereals (e. g. *G. zeae*) and rice (e. g. *G. fujikuroi*: Bakanae disease); *Glomerella cingulata* on vines, pome fruits and other plants and *G. gossypii* on cotton; Grainstaining complex on rice; *Guignardia bidwellii* (black rot) on vines; *Gymnosporangium* spp. on rosaceous plants and junipers, e. g. *G. sabinae* (rust) on pears; *Helminthosporium* spp. (syn. *Drechslera*, teleomorph: *Cochliobolus*) on corn, cereals and rice; *Hemileia* spp., e. g. *H. vastatrix* (coffee leaf rust) on coffee; *Isariopsis clavispora* (syn. *Cladosporium vitis*) on vines; *Macrophomina phaseolina* (syn. *phaseoli*) (root and stem rot) on soybeans and cotton; *Microdochium* (syn. *Fusarium*) *nivale* (pink snow mold) on cereals (e. g. wheat or barley); *Microsphaera diffusa* (powdery mildew) on soybeans; *Monilinia* spp., e. g. *M. laxa*, *M. fructicola* and *M. fructigena* (bloom and twig blight, brown rot) on stone fruits and other rosaceous plants; *Mycosphaerella* spp. on cereals, bananas, soft fruits and ground nuts, such as e. g. *M. graminicola* (anamorph: *Septoria tritici*, Septoria blotch) on wheat or *M. fijiensis* (black Sigatoka disease) on bananas; *Peronospora* spp. (downy mildew) on cabbage (e. g. *P. brassicae*), rape (e. g. *P. parasitica*), onions (e. g. *P. destructor*), tobacco (*P. tabacina*) and soybeans (e. g. *P. manshurica*); *Phakopsora pachyrhizi* and *P. meibomiae* (soybean rust) on soybeans; *Phialophora* spp. e. g. on vines (e. g. *P. tracheiphila* and *P. tetraspora*) and soybeans (e. g. *P. gregata*: stem rot); *Phoma lingam* (root and stem rot) on rape and cabbage and *P. betae* (root rot, leaf spot and damping-off) on sugar beets; *Phomopsis* spp. on sunflowers, vines (e. g. *P. viticola*: can and leaf spot) and soybeans (e. g. stem rot: *P. phaseoli*, teleomorph: *Diaporthe phaseolorum*); *Physoderma maydis* (brown spots) on corn; *Phytophthora* spp. (wilt, root, leaf, fruit and stem root) on various plants, such as paprika and cucurbits (e. g. *P. capsici*), soybeans (e. g. *P. megasperma*, syn. *P. sojae*), potatoes and tomatoes (e. g. *P. infestans*: late blight) and broad-leaved trees (e. g. *P. ramorum*: sudden oak death); *Plasmodiophora brassicae* (club root) on cabbage, rape, radish and other plants; *Plasmopara* spp., e. g. *P. viticola* (grapevine downy mildew) on vines and *P. halstedii* on sunflowers; *Podosphaera* spp. (powdery mildew) on rosaceous plants, hop, pome and soft fruits, e. g. *P. leucotricha* on apples; *Polymyxa* spp., e. g. on cereals, such as barley and wheat (*P. graminis*) and sugar beets (*P. betae*) and thereby transmitted viral diseases; *Pseudocercosporella herpotrichoides* (eyespot, teleomorph: *Tapesia yallundae*) on cereals, e. g. wheat or barley; *Pseudoperonospora* (downy mildew) on various plants, e. g. *P. cubensis* on cucurbits or *P. humili* on hop; *Pseudopezicula tracheiphila* (red fire disease or ,rotbrenner', anamorph: *Phialophora*) on vines; *Puccinia* spp. (rusts) on various plants, e. g. *P. triticina* (brown or leaf rust), *P. striiformis* (stripe or yellow rust), *P. hordei* (dwarf rust), *P. graminis* (stem or black rust) or *P. recondita* (brown or leaf rust) on cereals, such as e. g. wheat, barley or rye, *P. kuehnii* (orange rust) on sugar cane and *P. asparagi* on asparagus; *Pyrenophora* (anamorph: *Drechslera*) *tritici-repentis* (tan spot) on wheat or *P. teres* (net blotch) on barley; *Pyricularia* spp., e. g. *P. oryzae* (teleomorph: *Magnaporthe grisea,* rice blast) on rice and *P. grisea* on turf and cereals; *Pythium* spp. (damping-off) on turf, rice, corn, wheat, cotton, rape, sunflowers, soybeans, sugar beets, vegetables and various other plants (e. g. *P. ultimum* or *P. aphanidermatum*); *Ramularia* spp., e. g. *R. collo-cygni* (Ramularia leaf spots, Physiological leaf spots) on barley and *R. beticola* on sugar beets; *Rhizoctonia* spp. on cotton, rice, potatoes, turf, corn, rape, potatoes, sugar beets, vegetables and various other plants, e. g. *R. solani* (root and stem rot) on soybeans, *R. solani* (sheath blight) on rice or *R. cerealis* (Rhizoctonia spring blight) on wheat or barley; *Rhizopus stolonifer* (black mold, soft rot) on strawberries, carrots, cabbage, vines and tomatoes; *Rhynchosporium secalis* (scald) on barley, rye and triticale; *Sarocladium oryzae* and *S. attenuatum* (sheath rot) on rice; *Sclerotinia* spp. (stem rot or white mold) on vegetables and field crops, such as rape, sunflowers (e. g. *S. sclerotiorum*) and soybeans (e. g. *S. rolfsii* or *S. sclerotiorum*); *Septoria* spp. on various plants, e. g. *S. glycines* (brown spot) on soybeans, *S. tritici* (Septoria blotch) on wheat and *S.* (syn. *Stagonospora*) nodorum (Stagonospora blotch) on cereals; *Uncinula* (syn. *Erysiphe*) *necator* (powdery mildew, anamorph: *Oidium tuckeri*) on vines; *Setospaeria* spp. (leaf blight) on corn (e. g. *S. turcicum,* syn. *Helminthosporium turcicum*) and turf; *Sphacelotheca* spp. (smut) on corn, (e. g. *S. reiliana*: head smut), sorghum und sugar cane; *Sphaerotheca fuliginea* (powdery mildew) on cucurbits; *Spongospora subterranea* (powdery scab) on potatoes and thereby transmitted viral diseases; *Stagonospora* spp. on cereals, e. g. *S. nodorum* (Stagonospora blotch, teleomorph: *Leptosphaeria* [syn. *Phaeosphaeria*] *nodorum*) on wheat; *Synchytrium endobioticum* on potatoes (potato wart disease); *Taphrina* spp., e. g. *T. deformans* (leaf curl disease) on peaches and *T. pruni* (plum pocket) on plums; *Thielaviopsis* spp. (black root rot) on tobacco, pome fruits, vegetables, soybeans and cotton, e. g. *T. basicola* (syn. *Chalara elegans*); *Tilletia* spp. (common bunt or stinking smut) on cereals, such as e. g. *T. tritici* (syn. *T. caries*, wheat bunt) and *T. controversa* (dwarf bunt) on wheat; *Typhula incarnata* (grey snow mold) on barley or wheat; *Urocystis* spp., e. g. *U. occulta* (stem smut) on rye; *Uromyces* spp. (rust) on vegetables, such as beans (e. g. *U. appendiculatus,* syn. *U. phaseoli*) and sugar beets (e. g. *U. betae*); *Ustilago* spp. (loose smut) on cereals (e. g. *U. nuda* and *U. avaenae*), corn (e. g. *U. maydis*: corn smut) and sugar cane; *Venturia* spp. (scab) on apples (e. g. V. *inaequalis*) and pears; and *Verticillium* spp. (wilt) on various plants, such as fruits and ornamentals, vines, soft fruits, vegetables and field crops, e. g. *V. dahliae* on strawberries, rape, potatoes and tomatoes.

The compounds I and compositions thereof, respectively, are also suitable for controlling harmful fungi in the protection of stored products or harvest and in the protection of materials.

The term "protection of materials" is to be understood to denote the protection of technical and non-living materials, such as adhesives, glues, wood, paper and paperboard, textiles, leather, paint dispersions, plastics, cooling lubricants, fiber or fabrics, against the infestation and destruction by harmful microorganisms, such as fungi and bacteria. As to the protection of wood and other materials, the particular attention is paid to the following harmful fungi: Ascomycetes such as *Ophiostoma* spp., *Ceratocystis* spp., *Aureobasidium pullulans, Sclerophoma* spp., *Chaetomium* spp., *Humicola* spp., *Petriella* spp., *Trichurus* spp.; Basidiomycetes such as *Coniophora* spp., *Coriolus* spp., *Gloeophyllum* spp., *Lentinus* spp., *Pleurotus* spp., *Poria* spp., *Serpula* spp. and *Tyromyces* spp., Deuteromycetes such as *Aspergillus* spp., *Cladosporium* spp., *Penicillium* spp., *Trichoderma* spp., *Alternaria* spp., *Paecilomyces* spp. and Zygomycetes such as *Mucor* spp., and in addition in the protection of stored products and harvest the following yeast fungi are worthy of note: *Candida* spp. and *Saccharomyces cerevisae.*

The method of treatment according to the invention can also be used in the field of protecting stored products or harvest against attack of fungi and microorganisms. According to the present invention, the term "stored products" is understood to denote natural substances of plant or animal origin and their processed forms, which have been taken from the natural life cycle and for which long-term protection is desired. Stored products of crop plant origin, such as plants or parts thereof, for example stalks, leafs, tubers, seeds, fruits or grains, can be protected in the freshly harvested state or in processed form, such as pre-dried, moistened, comminuted, ground, pressed or roasted, which process is also known as post-harvest treatment. Also falling under the definition of stored products is timber, whether in the form of crude timber, such as construction timber, electricity pylons and barriers, or in the form of finished articles, such as furniture or objects made from wood. Stored products of animal origin are hides, leather, furs, hairs and the like. The combinations according the present invention can prevent disadvantageous effects such as decay, discoloration or mold. Preferably "stored products" is understood to denote natural substances of plant origin and their processed forms, more preferably fruits and their processed forms, such as pomes, stone fruits, soft fruits and citrus fruits and their processed forms.

The compounds I and compositions thereof, respectively, may be used for improving the health of a plant. The invention also relates to a method for improving plant health by treating a plant, its propagation material and/or the locus where the plant is growing or is to grow with an effective amount of compounds I and compositions thereof, respectively.

The term "plant health" is to be understood to denote a condition of the plant and/or its products which is determined by several indicators alone or in combination with each other such as yield (e. g. increased biomass and/or increased content of valuable ingredients), plant vigor (e. g. improved plant growth and/or greener leaves ("greening effect")), quality (e. g. improved content or composition of certain ingredients) and tolerance to abiotic and/or biotic stress. The above identified indicators for the health condition of a plant may be interdependent or may result from each other.

The compounds of formula I can be present in different crystal modifications whose biological activity may differ. They are likewise subject matter of the present invention.

The compounds I are employed as such or in form of compositions by treating the fungi or the plants, plant propagation materials, such as seeds, soil, surfaces, materials or rooms to be protected from fungal attack with a fungicidally effective amount of the active substances. The application can be carried out both before and after the infection of the plants, plant propagation materials, such as seeds, soil, surfaces, materials or rooms by the fungi.

Plant propagation materials may be treated with compounds I as such or a composition comprising at least one compound I prophylactically either at or before planting or transplanting.

The invention also relates to agrochemical compositions comprising an auxiliary and at least one compound I according to the invention.

An agrochemical composition comprises a fungicidally effective amount of a compound I. The term "effective amount" denotes an amount of the composition or of the compounds I, which is sufficient for controlling harmful fungi on cultivated plants or in the protection of materials and which does not result in a substantial damage to the treated plants. Such an amount can vary in a broad range and is dependent on various factors, such as the fungal species to be controlled, the treated cultivated plant or material, the climatic conditions and the specific compound I used.

The compounds I, their N-oxides and salts can be converted into customary types of agrochemical compositions, e. g. solutions, emulsions, suspensions, dusts, powders, pastes, granules, pressings, capsules, and mixtures thereof. Examples for composition types are suspensions (e. g. SC, OD, FS), emulsifiable concentrates (e. g. EC), emulsions (e. g. EW, EO, ES, ME), capsules (e. g. CS, ZC), pastes, pastilles, wettable powders or dusts (e. g. WP, SP, WS, DP, DS), pressings (e. g. BR, TB, DT), granules (e. g. WG, SG, GR, FG, GG, MG), insecticidal articles (e. g. LN), as well as gel formulations for the treatment of plant propagation materials such as seeds (e. g. GF). These and further compositions types are defined in the "Catalogue of pesticide formulation types and international coding system", Technical Monograph No. 2, 6th Ed. May 2008, CropLife International.

The compositions are prepared in a known manner, such as described by Mollet and Grubemann, Formulation technology, Wiley VCH, Weinheim, 2001; or Knowles, New developments in crop protection product formulation, Agrow Reports DS243, T&F Informa, London, 2005.

Suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, such as mineral oil fractions of medium to high boiling point, e. g. kerosene, diesel oil; oils of vegetable or animal origin; aliphatic, cyclic and aromatic hydrocarbons, e. g. toluene, paraffin, tetrahydronaphthalene, alkylated naphthalenes; alcohols, e. g. ethanol, propanol, butanol, benzyl alcohol, cyclohexanol; glycols; DMSO; ketones, e. g. cyclohexanone; esters, e. g. lactates, carbonates, fatty acid esters, gamma-butyrolactone; fatty acids; phosphonates; amines; amides, e. g. N-methyl pyrrolidone, fatty acid dimethyl amides; and mixtures thereof.

Suitable solid carriers or fillers are mineral earths, e. g. silicates, silica gels, talc, kaolins, limestone, lime, chalk, clays, dolomite, diatomaceous earth, bentonite, calcium sulfate, magnesium sulfate, magnesium oxide; polysaccharides, e. g. cellulose, starch; fertilizers, e. g. ammonium sulfate, ammonium phosphate, ammonium nitrate, ureas; products of vegetable origin, e. g. cereal meal, tree bark meal, wood meal, nutshell meal, and mixtures thereof.

Suitable surfactants are surface-active compounds, such as anionic, cationic, nonionic and amphoteric surfactants, block polymers, polyelectrolytes, and mixtures thereof. Such surfactants can be used as emulsifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International Ed. or North American Ed.).

Suitable anionic surfactants are alkali, alkaline earth or ammonium salts of sulfonates, sulfates, phosphates, carboxylates, and mixtures thereof. Examples of sulfonates are alkylaryl sulfonates, diphenyl sulfonates, alpha-olefin sulfonates, lignin sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkyl naphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, N-substituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-substituted fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are home- or copolymers of vinyl pyrrolidone, vinyl alcohols, or vinyl acetate.

Suitable cationic surfactants are quaternary surfactants, for example quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinyl amines or polyethylene amines.

Suitable adjuvants are compounds, which have a negligible or even no pesticidal activity themselves, and which improve the biological performance of the compound I on the target. Examples are surfactants, mineral or vegetable oils, and other auxiliaries. Further examples are listed by Knowles, Adjuvants and additives, Agrow Reports DS256, T&F Informa UK, 2006, chapter 5.

Suitable thickeners are polysaccharides (e. g. xanthan gum, carboxymethyl cellulose), inorganic clays (organically modified or unmodified), polycarboxylates, and silicates.

Suitable bactericides are bronopol and isothiazolinone derivatives such as alkylisothiazolinones and benzisothiazolinones.

Suitable anti-freezing agents are ethylene glycol, propylene glycol, urea and glycerin.

Suitable anti-foaming agents are silicones, long chain alcohols, and salts of fatty acids.

Suitable colorants (e. g. in red, blue, or green) are pigments of low water solubility and water-soluble dyes. Examples are inorganic colorants (e. g. iron oxide, titan oxide, iron hexacyanoferrate) and organic colorants (e. g. alizarin-, azo- and phthalocyanine colorants).

Suitable tackifiers or binders are polyvinyl pyrrolidones, polyvinyl acetates, polyvinyl alcohols, polyacrylates, biological or synthetic waxes, and cellulose ethers.

Examples for composition types and their preparation are:
i) Water-soluble concentrates (SL, LS)
   10-60 wt% of a compound I and 5-15 wt% wetting agent (e. g. alcohol alkoxylates) are dissolved in water and/or in a water-soluble solvent (e. g. alcohols) ad 100 wt%. The active substance dissolves upon dilution with water.
ii) Dispersible concentrates (DC)
   5-25 wt% of a compound I and 1-10 wt% dispersant (e. g. polyvinyl pyrrolidone) are dissolved in organic solvent (e. g. cyclohexanone) ad 100 wt%. Dilution with water gives a dispersion.
iii) Emulsifiable concentrates (EC)
   15-70 wt% of a compound I and 5-10 wt% emulsifiers (e. g. calcium dodecylbenzenesulfonate and castor oil ethoxylate) are dissolved in water-insoluble organic solvent (e. g. aromatic hydrocarbon) ad 100 wt%. Dilution with water gives an emulsion.
iv) Emulsions (EW, EO, ES)
   5-40 wt% of a compound I and 1-10 wt% emulsifiers (e. g. calcium dodecylbenzenesulfonate and castor oil ethoxylate) are dissolved in 20-40 wt% water-insoluble organic solvent (e. g. aromatic hydrocarbon). This mixture is introduced into water ad 100 wt% by means of an emulsifying machine and made into a homogeneous emulsion. Dilution with water gives an emulsion.
v) Suspensions (SC, OD, FS)
   In an agitated ball mill, 20-60 wt% of a compound I are comminuted with addition of 2-10 wt% dispersants and wetting agents (e. g. sodium lignosulfonate and alcohol ethoxylate), 0.1-2 wt% thickener (e. g. xanthan gum) and water ad 100 wt% to give a fine active substance suspension. Dilution with water gives a stable suspension of the active substance. For FS type composition up to 40 wt% binder (e. g. polyvinyl alcohol) is added.
vi) Water-dispersible granules and water-soluble granules (WG, SG)
   50-80 wt% of a compound I are ground finely with addition of dispersants and wetting agents (e. g. sodium lignosulfonate and alcohol ethoxylate) ad 100 wt% and prepared as water-dispersible or water-soluble granules by means of technical appliances (e. g. extrusion, spray tower, fluidized bed). Dilution with water gives a stable dispersion or solution of the active substance.
vii) Water-dispersible powders and water-soluble powders (WP, SP, WS)
   50-80 wt% of a compound I are ground in a rotor-stator mill with addition of 1-5 wt% dispersants (e. g. sodium lignosulfonate), 1-3 wt% wetting agents (e. g. alcohol ethoxylate) and solid carrier (e. g. silica gel) ad 100 wt%. Dilution with water gives a stable dispersion or solution of the active substance.
viii) Gel (GW, GF)
   In an agitated ball mill, 5-25 wt% of a compound I are comminuted with addition of 3-10 wt% dispersants (e. g. sodium lignosulfonate), 1-5 wt% thickener (e. g. carboxymethyl cellulose) and water ad 100 wt% to give a fine suspension of the active substance. Dilution with water gives a stable suspension of the active substance.
ix) Microemulsion (ME)
   5-20 wt% of a compound I are added to 5-30 wt% organic solvent blend (e. g. fatty acid dimethyl amide and cyclohexanone), 10-25 wt% surfactant blend (e. g. alcohol ethoxylate and arylphenol ethoxylate), and water ad 100 %. This mixture is stirred for 1 h to produce spontaneously a thermodynamically stable microemulsion.
x) Microcapsules (CS)
   An oil phase comprising 5-50 wt% of a compound I, 0-40 wt% water insoluble organic solvent (e. g. aromatic hydrocarbon), 2-15 wt% acrylic monomers (e. g. methylmethacrylate, methacrylic acid and a di- or triacrylate) are dispersed into an aqueous solution of a protective colloid (e. g. polyvinyl alcohol). Radical polymerization results in the formation of poly(meth)acrylate microcapsules. Alternatively, an oil phase comprising 5-50 wt% of a compound I according to the invention, 0-40 wt% water insoluble organic solvent (e. g. aromatic hydrocarbon), and an isocyanate monomer (e. g. diphenylmethene-4,4'-diisocyanatae) are dispersed into an aqueous solution of a protective colloid (e. g. polyvinyl alcohol). The addition of a polyamine (e. g. hexamethylenediamine) results in the formation of polyurea microcapsules. The monomers amount to 1-10 wt%. The wt% relate to the total CS composition.
xi) Dustable powders (DP, DS)
   1-10 wt% of a compound I are ground finely and mixed intimately with solid carrier (e. g. finely divided kaolin) ad 100 wt%.
xii) Granules (GR, FG)
   0.5-30 wt% of a compound I is ground finely and associated with solid carrier (e. g. silicate) ad 100 wt%. Granulation is achieved by extrusion, spray-drying or fluidized bed.
xiii) Ultra-low volume liquids (UL)
   1-50 wt% of a compound I are dissolved in organic solvent (e. g. aromatic hydrocarbon) ad 100 wt%.

The compositions types i) to xiii) may optionally comprise further auxiliaries, such as 0.1-1 wt% bactericides, 5-15 wt% anti-freezing agents, 0.1-1 wt% anti-foaming agents, and 0.1-1 wt% colorants.

The agrochemical compositions generally comprise between 0.01 and 95%, preferably between 0.1 and 90%, and in particular between 0.5 and 75%, by weight of active substance. The active substances are employed in a purity of from 90% to 100%, preferably from 95% to 100% (according to NMR spectrum).

For the purposes of treatment of plant propagation materials, particularly seeds, solutions for seed treatment (LS), Suspoemulsions (SE), flowable concentrates (FS), powders for dry treatment (DS), water-dispersible powders for slurry treatment (WS), water-soluble powders (SS), emulsions (ES), emulsifiable concentrates (EC), and gels (GF) are usually employed. The compositions in question give, after two-to-tenfold dilution, active substance concentrations of from 0.01 to 60% by weight, preferably from 0.1 to 40%, in the ready-to-use preparations. Application can be carried out before or during sowing. Methods for applying compound I and compositions thereof, respectively, onto plant propagation material, especially seeds, include dressing, coating, pelleting, dusting, and soaking as well as in-furrow application methods. Preferably, compound I or the compositions thereof, respectively, are applied on to the plant propagation material by a method such that germination is not induced, e. g. by seed dressing, pelleting, coating and dusting.

When employed in plant protection, the amounts of active substances applied are, depending on the kind of effect desired, from 0.001 to 2 kg per ha, preferably from 0.005 to 2 kg per ha, more preferably from 0.05 to 0.9 kg per ha, and in particular from 0.1 to 0.75 kg per ha.

In treatment of plant propagation materials such as seeds, e. g. by dusting, coating or drenching seed, amounts of active substance of from 0.1 to 1000 g, preferably from 1 to 1000 g, more preferably from 1 to 100 g and most preferably from 5 to 100 g, per 100 kilogram of plant propagation material (preferably seeds) are generally required.

When used in the protection of materials or stored products, the amount of active substance applied depends on the kind of application area and on the desired effect. Amounts customarily applied in the protection of materials are 0.001 g to 2 kg, preferably 0.005 g to 1 kg, of active substance per cubic meter of treated material.

Various types of oils, wetters, adjuvants, fertilizer, or micronutrients, and further pesticides (e. g. herbicides, insecticides, fungicides, growth regulators, safeners, biopesticides) may be added to the active substances or the compositions comprising them as premix or, if appropriate not until immediately prior to use (tank mix). These agents can be admixed with the compositions according to the invention in a weight ratio of 1:100 to 100:1, preferably 1:10 to 10:1.

A pesticide is generally a chemical or biological agent (such as pestidal active ingredient, compound, composition, virus, bacterium, antimicrobial or disinfectant) that through its effect deters, incapacitates, kills or otherwise discourages pests. Target pests can include insects, plant pathogens, weeds, mollusks, birds, mammals, fish, nematodes (roundworms), and microbes that destroy property, cause nuisance, spread disease or are vectors for disease. The term "pesticide" includes also plant growth regulators that alter the expected growth, flowering, or reproduction rate of plants; defoliants that cause leaves or other foliage to drop from a plant, usually to facilitate harvest; desiccants that promote drying of living tissues, such as unwanted plant tops; plant activators that activate plant physiology for defense of against certain pests; safeners that reduce unwanted herbicidal action of pesticides on crop plants; and plant growth promoters that affect plant physiology e.g. to increase plant growth, biomass, yield or any other quality parameter of the harvestable goods of a crop plant.

Biopesticides have been defined as a form of pesticides based on micro-organisms (bacteria, fungi, viruses, nematodes, etc.) or natural products (compounds, such as metabolites, proteins, or extracts from biological or other natural sources) (U.S. Environmental Protection Agency: http://www.epa.gov/pesticides/biopesticides/). Biopesticides fall into two major classes, microbial and biochemical pesticides:
(1) Microbial pesticides consist of bacteria, fungi or viruses (and often include the metabolites that bacteria and fungi produce). Entomopathogenic nematodes are also classed as microbial pesticides, even though they are multi-cellular.
(2) Biochemical pesticides are naturally occurring substances that control pests or provide other crop protection uses as defined below, but are relatively non-toxic to mammals.

The user applies the composition according to the invention usually from a predosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

According to one embodiment, individual components of the composition according to the invention such as parts of a kit or parts of a binary or ternary mixture may be mixed by the user himself in a spray tank or any other kind of vessel used for applications (e. g. seed treater drums, seed pelleting machinery, knapsack sprayer) and further auxiliaries may be added, if appropriate.

When living microorganisms, such as microbial pesticides from groups L1), L3) and L5), form part of such kit, it must be taken care that choice and amounts of the components (e. g. chemical pesticides) and of the further auxiliaries should not influence the viability of the microbial pesticides in the composition mixed by the user. Especially for bactericides and solvents, compatibility with the respective microbial pesticide has to be taken into account.

Consequently, one embodiment of the invention is a kit for preparing a usable pesticidal composition, the kit comprising a) a composition comprising component 1) as defined herein and at least one auxiliary; and b) a composition comprising component 2) as defined herein and at least one auxiliary; and optionally c) a composition comprising at least one auxiliary and optionally a further active component 3) as defined herein.

Mixing the compounds I or the compositions comprising them in the use form as fungicides with other fungicides results in many cases in an expansion of the fungicidal spectrum of activity being obtained or in a prevention of fungicide resistance development. Furthermore, in many cases, synergistic effects are obtained.

The following list of pesticides II (e. g. pesticidally-active substances and biopesticides), in conjunction with which the compounds I can be used, is intended to illustrate the possible combinations but does not limit them:
A) Respiration inhibitors
   - Inhibitors of complex III at Qₒ site (e. g. strobilurins): azoxystrobin (A.1.1), coumethoxystrobin (A.1.2), coumoxystrobin (A.1.3), dimoxystrobin (A.1.4), enestroburin (A.1.5), fenaminstrobin (A.1.6), fenoxystrobin/flufenoxystrobin (A.1.7), fluoxastrobin (A.1.8), kresoxim-methyl (A.1.9), mandestrobin (A.1.10), metominostrobin (A.1.11), orysastrobin (A.1.12), picoxystrobin (A.1.13), pyraclostrobin (A.1.14), pyrametostrobin (A.1.15), pyraoxystrobin (A.1.16), tri-floxystrobin (A.1.17), 2-(2-(3-(2,6-dichlorophenyl)-1-methyl-allylideneaminooxymethyl)-phenyl)-2-methoxyimino-N-methyl-acetamide (A.1.18), pyribencarb (A.1.19), triclopyricarb/chlorodincarb (A.1.20), famoxadone (A.1.21), fenamidone (A.1.21), methyl-*N*-[2-[(1,4-dimethyl-5-phenyl-pyrazol-3-yl)oxylmethyl]phenyl]-N-methoxy-carbamate (A.1.22), 1-[3-chloro-2-[[[1-(4-chlorophenyl)-1H-pyrazol-3-yl]oxy]methyl]phenyl]-1,4-dihydro-4-methyl-5H-tetrazol-5-one (A.1.23), (*Z*,2*E*)-5-[1-(2,4-dichlorophenyl)pyrazol-3-yl]-oxy-2-methoxyimino-*N*,3-dimethyl-pent-3-enamide (A.1.24), (*Z*,2*E*)-5-[1-(4-chlorophenyl)pyrazol-3-yl]oxy-2-methoxyimino-*N*,3-dimethyl-pent-3-enamide (A.1.25), (*Z*,2*E*)-5-[1-(4-chloro-2-fluoro-phenyl)pyrazol-3-yl]oxy-2-methoxyimino-*N*,3-dimethyl-pent-3-enamide (A.1.26); 1-[3-bromo-2-[[1-(4-chlorophenyl)pyrazol-3-yl]oxymethyl]phenyl]-4-methyl-tetrazol-5-one (A.1.27), 1-[2-[[1-(4-chlorophenyl)pyrazol-3-yl]oxymethyl]-3-methyl-phenyl]-4-methyl-tetrazol-5-one (A.1.28), 1-[2-[[1-(4-chlorophenyl)pyrazol-3-yl]oxymethyl]-3-fluoro-phenyl]-4-methyl-tetrazol-5-one (A.1.29), 1-[2-[[1-(2,4-dichlorophenyl)pyrazol-3-yl]oxymethyl]-3-fluoro-phenyl]-4-methyl-tetrazol-5-one (A.1.30), 1-[2-[[4-(4-chlorophenyl)thiazol-2-yl]oxymethyl]-3-methyl-phenyl]-4-methyl-tetrazol-5-one (A.1.31), 1-[3-chloro-2-[[4-(p-tolyl)thiazol-2-yl]oxymethyl]phenyl]-4-methyl-tetrazol-5-one (A.1.32), 1-[3-cyclopropyl-2-[[2-methyl-4-(1-methylpyrazol-3-yl)phenoxy]methyl]phenyl]-4-methyl-tetrazol-5-one (A.1.33), 1-[3-(difluoromethoxy)-2-[[2-methyl-4-(1-methylpyrazol-3-yl)phenoxy]methyl]phenyl]-4-methyl-tetrazol-5-one (A.1.34), 1-methyl-4-[3-methyl-2-[[2-methyl-4-(1-methylpyrazol-3-yl)phenoxy]methyl]phenyl]tetrazol-5-one (A.1.35), 1-methyl-4-[3-methyl-2-[[1-[3-(trifluoromethyl)phenyl]-ethylideneamino]oxymethyl]phenyl]tetrazol-5-one (A.1.36), (*Z*,2*E*)-5-[1-(2,4-dichlorophenyl)pyrazol-3-yl]-oxy-2-methoxyimino-*N*,3-dimethyl-pent-3-enamide (A.1.37), (*Z*,2*E*)-5-[1-(4-chlorophenyl)pyrazol-3-yl]oxy-2-methoxyimino-*N*,3-dimethyl-pent-3-enamide (A.1.38), (*Z*,2*E*)-5-[1-(4-chloro-2-fluoro-phenyl)pyrazol-3-yl]oxy-2-methoxyimino-*N*,3-dimethyl-pent-3-enamide (A.1.39);
   - inhibitors of complex III at Qᵢ site: cyazofamid (A.2.1), amisulbrom (A.2.2), [(3S,6S,7R,8R)-8-benzyl-3-[(3-acetoxy-4-methoxy-pyridine-2-carbonyl)amino]-6-methyl-4,9-dioxo-1,5-dioxonan-7-yl] 2-methylpropanoate (A.2.3), [(3S,6S,7R,8R)-8-benzyl-3-[[3-(acetoxymethoxy)-4-methoxy-pyridine-2-carbonyl]amino]-6-methyl-4,9-dioxo-1,5-dioxonan-7-yl] 2-methylpropanoate (A.2.4), [(3S,6S,7R,8R)-8-benzyl-3-[(3-isobutoxycarbonyloxy-4-methoxy-pyridine-2-carbonyl)amino]-6-methyl-4,9-dioxo-1,5-dioxonan-7-yl] 2-methylpropanoate (A.2.5), [(3S,6S,7R,8R)-8-benzyl-3-[[3-(1,3-benzodioxol-5-ylmethoxy)-4-methoxy-pyridine-2-carbonyl]amino]-6-methyl-4,9-dioxo-1,5-dioxonan-7-yl] 2-methylpropanoate (A.2.6); (3S,6S,7R,8R)-3-[[(3-hydroxy-4-methoxy-2-pyridinyl)carbonyl]amino]-6-methyl-4,9-dioxo-8-(phenylmethyl)-1,5-dioxonan-7-yl 2-methylpropanoate (A.2.7); (3S,6S,7R,8R)-8-benzyl-3-[3-[(isobutyryloxy)methoxy]-4-methoxypicolinamido]-6-methyl-4,9-dioxo-1,5-dioxonan-7-yl isobutyrate (A.2.8);
   - inhibitors of complex II (e. g. carboxamides): benodanil (A.3.1), benzovindiflupyr (A.3.2), bixafen (A.3.3), boscalid (A.3.4), carboxin (A.3.5), fenfuram (A.3.6), fluopyram (A.3.7), flutolanil (A.3.8), fluxapyroxad (A.3.9), furametpyr (A.3.10), isofetamid (A.3.11), isopyrazam (A.3.12), mepronil (A.3.13), oxycarboxin (A.3.14), penflufen (A.3.14), penthiopyrad (A.3.15), sedaxane (A.3.16), tecloftalam (A.3.17), thifluzamide (A.3.18), N-(4'-trifluoromethylthiobiphenyl-2-yl)-3-difluoromethyl-1-methyl-1H-pyrazole-4-carboxamide (A.3.19), N-(2-(1,3,3-trimethyl-butyl)-phenyl)-1,3-dimethyl-5-fluoro-1H-pyrazole-4-carboxamide (A.3.20), 3-(difluoromethyl)-1-methyl-N-(1,1,3-trimethylindan-4-yl)pyrazole-4-carboxamide (A.3.21), 3-(trifluoromethyl)-1-methyl-N-(1,1,3-trimethylindan-4-yl)pyrazole-4-carboxamide (A.3.22), 1,3-dimethyl-N-(1,1,3-trimethylindan-4-yl)pyrazole-4-carboxamide (A.3.23), 3-(trifluoromethyl)-1,5-dimethyl-N-(1,1,3-trimethylindan-4-yl)pyrazole-4-carboxamide (A.3.24), 1,3,5-trimethyl-N-(1,1,3-trimethylindan-4-yl)pyrazole-4-carboxamide (A.3.25), N-(7-fluoro-1,1,3-trimethyl-indan-4-yl)-1,3-dimethylpyrazole-4-carboxamide (A.3.26), N-[2-(2,4-dichlorophenyl)-2-methoxy-1-methyl-ethyl]-3-(difluoromethyl)-1-methyl-pyrazole-4-carboxamide (A.3.27);
   - other respiration inhibitors (e. g. complex I, uncouplers): diflumetorim (A.4.1), (5,8-difluoro-quinazolin-4-yl)-{2-[2-fluoro-4-(4-trifluoromethylpyridin-2-yloxy)-phenyl]-ethyl}-amine (A.4.2); nitrophenyl derivates: binapacryl (A.4.3), dinobuton (A.4.4), dinocap (A.4.5), fluazinam (A.4.6); ferimzone (A.4.7); organometal compounds: fentin salts, such as fentin-acetate (A.4.8), fentin chloride (A.4.9) or fentin hydroxide (A.4.10); ametoctradin (A.4.11); and silthiofam (A.4.12);
B) Sterol biosynthesis inhibitors (SBI fungicides)
   - C14 demethylase inhibitors (DMI fungicides): triazoles: azaconazole (B.1.1), bitertanol (B.1.2), bromuconazole (B.1.3), cyproconazole (B.1.4), difenoconazole (B.1.5), diniconazole (B.1.6), diniconazole-M (B.1.7), epoxiconazole (B.1.8), fenbuconazole (B.1.9), fluquinconazole (B.1.10), flusilazole (B.1.11), flutriafol (B.1.12), hexaconazole (B.1.13), imibenconazole (B.1.14), ipconazole (B.1.15), metconazole (B.1.17), myclobutanil (B.1.18), oxpoconazole (B.1.19), paclobutrazole (B.1.20), penconazole (B.1.21), propiconazole (B.1.22), prothioconazole (B.1.23), simeconazole (B.1.24), tebuconazole (B.1.25), tetraconazole (B.1.26), triadimefon (B.1.27), triadimenol (B.1.28), triticonazole (B.1.29), uniconazole (B.1.30), 1-[*rel*-(2*S*;3*R*)-3-(2-chlorophenyl)-2-(2,4-difluorophenyl)-oxiranylmethyl]-5-thiocyanato-1H-[1,2,4]triazolo (B.1.31), 2-[*rel*-(2*S*;3*R*)-3-(2-chlorophenyl)-2-(2,4-difluorophenyl)-oxiranylmethyl]-2H-[1,2,4]triazole-3-thiol (B.1.32), 2-[2-chloro-4-(4-chlorophenoxy)phenyl]-1-(1,2,4-triazol-1-yl)pentan-2-ol (B.1.33), 1-[4-(4-chlorophenoxy)-2-(trifluoromethyl)phenyl]-1-cyclopropyl-2-(1,2,4-triazol-1-yl)ethanol (B.1.34), 2-[4-(4-chlorophenoxy)-2-(trifluoromethyl)phenyl]-1-(1,2,4-triazol-1-yl)butan-2-ol (B.1.35), 2-[2-chloro-4-(4-chlorophenoxy)phenyl]-1-(1,2,4-triazol-1-yl)butan-2-ol (B.1.36), 2-[4-(4-chlorophenoxy)-2-(trifluoromethyl)phenyl]-3-methyl-1-(1,2,4-triazol-1-yl)butan-2-ol (B.1.37), 2-[4-(4-chlorophenoxy)-2-(trifluoromethyl)phenyl]-1-(1,2,4-triazol-1-yl)propan-2-ol (B.1.38), 2-[2-chloro-4-(4-chlorophenoxy)phenyl]-3-methyl-1-(1,2,4-triazol-1-yl)butan-2-ol (B.1.39), 2-[4-(4-chlorophenoxy)-2-(trifluoromethyl)phenyl]-1-(1,2,4-triazol-1-yl)pentan-2-ol (B.1.40), 2-[4-(4-fluorophenoxy)-2-(trifluoromethyl)phenyl]-1-(1,2,4-triazol-1-yl)propan-2-ol (B.1.41), 2-[2-chloro-4-(4-chlorophenoxy)phenyl]-1-(1,2,4-triazol-1-yl)pent-3-yn-2-ol (B.1.51); imidazoles: imazalil (B.1.42), pefurazoate (B.1.43), prochloraz (B.1.44), triflumizol (B.1.45); pyrimidines, pyridines and piperazines: fenarimol (B.1.46), nuarimol (B.1.47), pyrifenox (B.1.48), triforine (B.1.49), [3-(4-chloro-2-fluoro-phenyl)-5-(2,4-difluorophenyl)isoxazol-4-yl]-(3-pyridyl)methanol (B.1.50);
   - Delta14-reductase inhibitors: aldimorph (B.2.1), dodemorph (B.2.2), dodemorph-acetate (B.2.3), fenpropimorph (B.2.4), tridemorph (B.2.5), fenpropidin (B.2.6), piperalin (B.2.7), spirox-amine (B.2.8);
   - Inhibitors of 3-keto reductase: fenhexamid (B.3.1);
C) Nucleic acid synthesis inhibitors
   - phenylamides or acyl amino acid fungicides: benalaxyl (C.1.1), benalaxyl-M (C.1.2), kiralaxyl (C.1.3), metalaxyl (C.1.4), metalaxyl-M (mefenoxam, C.1.5), ofurace (C.1.6), oxadixyl (C.1.7);
   - others: hymexazole (C.2.1), octhilinone (C.2.2), oxolinic acid (C.2.3), bupirimate (C.2.4), 5-fluorocytosine (C.2.5), 5-fluoro-2-(p-tolylmethoxy)pyrimidin-4-amine (C.2.6), 5-fluoro-2-(4-fluorophenylmethoxy)pyrimidin-4-amine (C.2.7);
D) Inhibitors of cell division and cytoskeleton
   - tubulin inhibitors, such as benzimidazoles, thiophanates: benomyl (D1.1), carbendazim (D1.2), fuberidazole (D1.3), thiabendazole (D1.4), thiophanate-methyl (D1.5); triazolopyrimidines: 5-chloro-7-(4-methylpiperidin-1-yl)-6-(2,4,6-trifluorophenyl)-[1,2,4]tri-azolo[1,5-a]pyrimidine (D1.6);
   - other cell division inhibitors: diethofencarb (D2.1), ethaboxam (D2.2), pencycuron (D2.3), fluopicolide (D2.4), zoxamide (D2.5), metrafenone (D2.6), pyriofenone (D2.7);
E) Inhibitors of amino acid and protein synthesis
   - methionine synthesis inhibitors (anilino-pyrimidines): cyprodinil (E.1.1), mepanipyrim (E.1.2), pyrimethanil (E.1.3);
   - protein synthesis inhibitors: blasticidin-S (E.2.1), kasugamycin (E.2.2), kasugamycin hydrochloride-hydrate (E.2.3), mildiomycin (E.2.4), streptomycin (E.2.5), oxytetracyclin (E.2.6), polyoxine (E.2.7), validamycin A (E.2.8);
F) Signal transduction inhibitors
   - MAP / histidine kinase inhibitors: fluoroimid (F.1.1), iprodione (F.1.2), procymidone (F.1.3), vinclozolin (F.1.4), fenpiclonil (F.1.5), fludioxonil (F.1.6);
   - G protein inhibitors: quinoxyfen (F.2.1);
G) Lipid and membrane synthesis inhibitors
   - Phospholipid biosynthesis inhibitors: edifenphos (G.1.1), iprobenfos (G.1.2), pyrazophos (G.1.3), isoprothiolane (G.1.4);
   - lipid peroxidation: dicloran (G.2.1), quintozene (G.2.2), tecnazene (G.2.3), tolclofos-methyl (G.2.4), biphenyl (G.2.5), chloroneb (G.2.6), etridiazole (G.2.7);
   - phospholipid biosynthesis and cell wall deposition: dimethomorph (G.3.1), flumorph (G.3.2), mandipropamid (G.3.3), pyrimorph (G.3.4), benthiavalicarb (G.3.5), iprovalicarb (G.3.6), valifenalate (G.3.7) and N-(1-(1-(4-cyano-phenyl)ethanesulfonyl)-but-2-yl) carbamic acid-(4-fluorophenyl) ester (G.3.8);
   - compounds affecting cell membrane permeability and fatty acides: propamocarb (G.4.1);
   - fatty acid amide hydrolase inhibitors: oxathiapiprolin (G.5.1), 2-{3-[2-(1-{[3,5-bis(difluoromethyl-1H-pyrazol-1-yl]acetyl}piperidin-4-yl)-1,3-thiazol-4-yl]-4,5-dihydro-1,2-oxazol-5-yl}phenyl methanesulfonate (G.5.2), 2-{3-[2-(1-{[3,5-bis(difluoromethyl)-1H-pyrazol-1-yl]acetyl}piperidin-4-yl) 1,3-thiazol-4-yl]-4,5-dihydro-1,2-oxazol-5-yl}-3-chlorophenyl methanesulfonate (G.5.3);
H) Inhibitors with Multi Site Action
   - inorganic active substances: Bordeaux mixture (H.1.1), copper acetate (H.1.2), copper hydroxide (H.1.3), copper oxychloride (H.1.4), basic copper sulfate (H.1.5), sulfur (H.1.6);
   - thio- and dithiocarbamates: ferbam (H.2.1), mancozeb (H.2.2), maneb (H.2.3), metam (H.2.4), metiram (H.2.5), propineb (H.2.6), thiram (H.2.7), zineb (H.2.8), ziram (H.2.9);
   - organochlorine compounds (e. g. phthalimides, sulfamides, chloronitriles): anilazine (H.3.1), chlorothalonil (H.3.2), captafol (H.3.3), captan (H.3.4), folpet (H.3.5), dichlofluanid (H.3.6), dichlorophen (H.3.7), hexachlorobenzene (H.3.8), pentachlorphenole (H.3.9) and its salts, phthalide (H.3.10), tolylfluanid (H.3.11), N-(4-chloro-2-nitro-phenyl)-N-ethyl-4-methyl-benzenesulfonamide (H.3.12);
   - guanidines and others: guanidine (H.4.1), dodine (H.4.2), dodine free base (H.4.3), guazatine (H.4.4), guazatine-acetate (H.4.5), iminoctadine (H.4.6), iminoctadine-triacetate (H.4.7), iminoctadine-tris(albesilate) (H.4.8), dithianon (H.4.9), 2,6-dimethyl-1H,5H-[1,4]dithiino[2,3-c:5,6-c']dipyrrole-1,3,5,7(2H,6H)-tetraone (H.4.10);
I) Cell wall synthesis inhibitors
   - inhibitors of glucan synthesis: validamycin (I.1.1), polyoxin B (I.1.2);
   - melanin synthesis inhibitors: pyroquilon (I.2.1), tricyclazole (I.2.2), carpropamid (I.2.3), dicyclomet (I.2.4), fenoxanil (I.2.5);
J) Plant defence inducers
   - acibenzolar-S-methyl (J.1.1), probenazole (J.1.2), isotianil (J.1.3), tiadinil (J.1.4), prohexadione-calcium (J.1.5); phosphonates: fosetyl (J.1.6), fosetyl-aluminum (J.1.7), phosphorous acid and its salts (J.1.8), potassium or sodium bicarbonate (J.1.9);
K) Unknown mode of action
   - bronopol (K.1.1), chinomethionat (K.1.2), cyflufenamid (K.1.3), cymoxanil (K.1.4), dazomet (K.1.5), debacarb (K.1.6), diclomezine (K.1.7), difenzoquat (K.1.8), difenzoquat-methylsulfate (K.1.9), diphenylamin (K.1.10), fenpyrazamine (K.1.11), flumetover (K.1.12), flusulfamide (K.1.13), flutianil (K.1.14), methasulfocarb (K.1.15), nitrapyrin (K.1.16), nitrothal-isopropyl (K.1.18), oxathiapiprolin (K.1.19), tolprocarb (K.1.20), oxin-copper (K.1.21), proquinazid (K.1.22), tebufloquin (K.1.23), tecloftalam (K.1.24), triazoxide (K.1.25), 2-butoxy-6-iodo-3-propylchromen-4-one (K.1.26), 2-[3,5-bis(difluoromethyl)-1H-pyrazol-1-yl]-1-[4-(4-{5-[2-(prop-2-yn-1-yloxy)phenyl]-4,5-dihydro-1,2-oxazol-3-yl}-1,3-thiazol-2-yl)piperidin-1-yl]ethanone (K.1.27), 2-[3,5-bis(difluoromethyl)-1H-pyrazol-1-yl]-1-[4-(4-{5-[2-fluoro-6-(prop-2-yn-1-yl-oxy)phenyl]-4,5-dihydro-1,2-oxazol-3-yl}-1,3-thiazol-2-yl)piperidin-1-yl]ethanone (K.1.28), 2-[3,5-bis(difluoromethyl)-1H-pyrazol-1-yl]-1-[4-(4-{5-[2-chloro-6-(prop-2-yn-1-yloxy)phenyl]-4,5-dihydro-1,2-oxazol-3-yl}-1,3-thiazol-2-yl)piperidin-1-yl]ethanone (K.1.29), N-(cyclopropylmethoxyimino-(6-difluoro-methoxy-2,3-difluoro-phenyl)-methyl)-2-phenyl acetamide (K.1.30), N'-(4-(4-chloro-3-trifluoromethyl-phenoxy)-2,5-dimethyl-phenyl)-N-ethyl-N-methyl formamidine (K.1.31), N'-(4-(4-fluoro-3-trifluoromethyl-phenoxy)-2,5-dimethyl-phenyl)-N-ethyl-N-methyl formamidine (K.1.32), N'-(2-methyl-5-trifluoromethyl-4-(3-trimethylsilanyl-propoxy)-phenyl)-N-ethyl-N-methyl formamidine (K.1.33), N'-(5-difluoromethyl-2-methyl-4-(3-tri-methylsilanyl-propoxy)-phenyl)-N-ethyl-N-methyl formamidine (K.1.34), methoxy-acetic acid 6-tert-butyl-8-fluoro-2,3-dimethyl-quinolin-4-yl ester (K.1.35), 3-[5-(4-methylphenyl)-2,3-dimethyl-isoxazolidin-3-yl]-pyridine (K.1.36), 3-[5-(4-chloro-phenyl)-2,3-dimethyl-isoxazolidin-3-yl]-pyridine (pyrisoxazole) (K.1.37), N-(6-methoxy-pyridin-3-yl) cyclopropanecarboxylic acid amide (K.1.38), 5-chloro-1-(4,6-dimethoxy-pyrimidin-2-yl)-2-methyl-1H-benzoimidazole (K.1.39), 2-(4-chloro-phenyl)-N-[4-(3,4-dimethoxy-phenyl)-isoxazol-5-yl]-2-prop-2-ynyloxy-acetamide, ethyl (Z)-3-amino-2-cyano-3-phenyl-prop-2-enoate (K.1.40), picarbutrazox (K.1.41), pentyl N-[6-[[(Z)-[(1-methyltetrazol-5-yl)-phenyl-methylene]amino]oxymethyl]-2-pyridyl]carbamate (K.1.42), 2-[2-[(7,8-difluoro-2-methyl-3-quinolyl)oxy]-6-fluoro-phenyl]propan-2-ol (K.1.43), 2-[2-fluoro-6-[(8-fluoro-2-methyl-3-quinolyl)oxy]phen-yl]propan-2-ol (K.1.44), 3-(5-fluoro-3,3,4,4-tetramethyl-3,4-dihydroisoquinolin-1-yl)quinoline (K.1.45), 3-(4,4-difluoro-3,3-dimethyl-3,4-dihydroisoquinolin-1-yl)quinoline (K.1.46), 3-(4,4,5-trifluoro-3,3-dimethyl-3,4-dihydroisoquinolin-1-yl)quinoline (K.1.47), 9-fluoro-2,2-dimethyl-5-(3-quinolyl)-3H-1,4-benzoxazepine (K.1.48);
M) Growth regulators
   abscisic acid (M.1.1), amidochlor, ancymidol, 6-benzylaminopurine, brassinolide, butralin, chlormequat, chlormequat chloride, choline chloride, cyclanilide, daminozide, dikegulac, dimethipin, 2,6-dimethylpuridine, ethephon, flumetralin, flurprimidol, fluthiacet, forchlorfenuron, gibberellic acid, inabenfide, indole-3-acetic acid, maleic hydrazide, mefluidide, mepiquat, mepiquat chloride, naphthaleneacetic acid, N-6-benzyladenine, paclobutrazol, prohexadione, prohexadione-calcium, prohydrojasmon, thidiazuron, triapenthenol, tributyl phosphorotrithioate, 2,3,5-tri-iodobenzoic acid, trinexapac-ethyl and uniconazole;
N) Herbicides
   - acetamides: acetochlor (N.1.1), alachlor, butachlor, dimethachlor, dimethenamid (N.1.2), flufenacet (N.1.3), mefenacet (N.1.4), metolachlor (N.1.5), metazachlor (N.1.6), napropamide, naproanilide, pethoxamid, pretilachlor, propachlor, thenylchlor;
   - amino acid derivatives: bilanafos, glyphosate (N.2.1), glufosinate (N.2.2), sulfosate (N.2.3);
   - aryloxyphenoxypropionates: clodinafop (N.3.1), cyhalofop-butyl, fenoxaprop (N.3.2), fluazifop (N.3.3), haloxyfop (N.3.4), metamifop, propaquizafop, quizalofop, quizalofop-P-tefuryl;
   - Bipyridyls: diquat, paraquat (N.4.1);
   - (thio)carbamates: asulam, butylate, carbetamide, desmedipham, dimepiperate, eptam (EPTC), esprocarb, molinate, orbencarb, phenmedipham (N.5.1), prosulfocarb, pyributicarb, thiobencarb, triallate;
   - cyclohexanediones: butroxydim, clethodim (N.6.1), cycloxydim (N.6.2), profoxydim (N.6.3), sethoxydim (N.6.4), tepraloxydim (N.6.5), tralkoxydim;
   - dinitroanilines: benfluralin, ethalfluralin, oryzalin, pendimethalin (N.7.1), prodiamine (N.7.2), trifluralin (N.7.3);
   - diphenyl ethers: acifluorfen (N.8.1), aclonifen, bifenox, diclofop, ethoxyfen, fomesafen, lactofen, oxyfluorfen;
   - hydroxybenzonitriles: bomoxynil (N.9.1), dichlobenil, ioxynil;
   - imidazolinones: imazamethabenz, imazamox (N.10.1), imazapic (N.10.2), imazapyr (N.10.3), imazaquin (N.10.4), imazethapyr (N.10.5);
   - phenoxy acetic acids: clomeprop, 2,4-dichlorophenoxyacetic acid (2,4-D) (N.11.1), 2,4-DB, dichlorprop, MCPA, MCPA-thioethyl, MCPB, Mecoprop;
   - pyrazines: chloridazon (N.11.1), flufenpyr-ethyl, fluthiacet, norflurazon, pyridate;
   - pyridines: aminopyralid, clopyralid (N.12.1), diflufenican, dithiopyr, fluridone, fluroxypyr (N.12.2), picloram (N.12.3), picolinafen (N.12.4), thiazopyr;
   - sulfonyl ureas: amidosulfuron, azimsulfuron, bensulfuron (N.13.1), chlorimuron-ethyl (N.13.2), chlorsulfuron, cinosulfuron, cyclosulfamuron (N.13.3), ethoxysulfuron, flazasulfuron, flucetosulfuron, flupyrsulfuron, foramsulfuron, halosulfuron, imazosulfuron, iodosulfuron (N.13.4), mesosulfuron (N.13.5), metazosulfuron, metsulfuron-methyl (N.13.6), nicosulfuron (N.13.7), oxasulfuron, primisulfuron, prosulfuron, pyrazosulfuron, rimsulfuron (N.13.8), sulfometuron, sulfosulfuron, thifensulfuron, triasulfuron, tribenuron, trifloxysulfuron, triflusulfuron (N.13.9), tritosulfuron, 1-((2-chloro-6-propyl-imidazo[1,2-b]pyridazin-3-yl)sulfonyl)-3-(4,6-dimethoxy-pyrimidin-2-yl)urea;
   - triazines: ametryn, atrazine (N.14.1), cyanazine, dimethametryn, ethiozin, hexazinone (N.14.2), metamitron, metribuzin, prometryn, simazine, terbuthylazine, terbutryn, triaziflam, trifludimoxazin (N14.3);
   - ureas: chlorotoluron, daimuron, diuron (N.15.1), fluometuron, isoproturon, linuron, methabenzthiazuron, tebuthiuron;
   - other acetolactate synthase inhibitors: bispyribac-sodium, cloransulam-methyl, diclosulam, florasulam (N.16.1), flucarbazone, flumetsulam, metosulam, ortho-sulfamuron, penoxsulam, propoxycarbazone, pyribambenz-propyl, pyribenzoxim, pyriftalid, pyriminobac-methyl, pyrimisulfan, pyrithiobac, pyroxasulfone (N.16.2), pyroxsulam;
   - others: amicarbazone, aminotriazole, anilofos, beflubutamid, benazolin, bencarbazone,benfluresate, benzofenap, bentazone (N.17.1), benzobicyclon, bicyclopyrone, bromacil, bromobutide, butafenacil, butamifos, cafenstrole, carfentrazone, cinidon-ethyl (N.17.2), chlorthal, cinmethylin (N.17.3), clomazone (N.17.4), cumyluron, cyprosulfamide, dicamba (N.17.5), difenzoquat, diflufenzopyr (N.17.6), *Drechslera monoceras*, endothal, ethofumesate, etobenzanid, fenoxasulfone, fentrazamide, flumiclorac-pentyl, flumioxazin, flupoxam, flurochloridone, flurtamone, indanofan, isoxaben, isoxaflutole, lenacil, propanil, propyzamide, quinclorac (N.17.7), quinmerac (N.17.8), mesotrione (N.17.9), methyl arsonic acid, naptalam, oxadiargyl, oxadiazon, oxaziclomefone, pentoxazone, pinoxaden, pyraclonil, pyraflufen-ethyl, pyrasulfotole, pyrazoxyfen, pyrazolynate, quinoclamine, saflufenacil (N.17.10), sulcotrione (N.17.11), sulfentrazone, terbacil, tefuryltrione, tembotrione, thiencarbazone, topramezone (N.17.12), (3-[2-chloro-4-fluoro-5-(3-methyl-2,6-dioxo-4-trifluoromethyl-3,6-dihydro-2H-pyrimidin-1-yl)-phenoxy]-pyridin-2-yloxy)-acetic acid ethyl ester, 6-amino-5-chloro-2-cyclopropyl-pyrimidine-4-carboxylic acid methyl ester, 6-chloro-3-(2-cyclopropyl-6-methyl-phenoxy)-pyridazin-4-ol, 4-amino-3-chloro-6-(4-chloro-phenyl)-5-fluoro-pyridine-2-carboxylic acid, 4-amino-3-chloro-6-(4-chloro-2-fluoro-3-methoxy-phenyl)-pyridine-2-carboxylic acid methyl ester, and 4-amino-3-chloro-6-(4-chloro-3-dimethylamino-2-fluoro-phenyl)-pyridine-2-carboxylic acid methyl ester;
O) Insecticides
   - organo(thio)phosphates: acephate (O.1.1), azamethiphos (O.1.2), azinphos-methyl (O.1.3), chlorpyrifos (O.1.4), chlorpyrifos-methyl (O.1.5), chlorfenvinphos (O.1.6), diazinon (O.1.7), dichlorvos (O.1.8), dicrotophos (O.1.9), dimethoate (O.1.10), disulfoton (O.1.11), ethion (O.1.12), fenitrothion (O.1.13), fenthion (O.1.14), isoxathion (O.1.15), malathion (O.1.16), methamidophos (O.1.17), methidathion (O.1.18), methyl-parathion (O.1.19), mevinphos (O.1.20), monocrotophos (O.1.21), oxydemeton-methyl (O.1.22), paraoxon (O.1.23), parathion (O.1.24), phenthoate (O.1.25), phosalone (O.1.26), phosmet (O.1.27), phosphamidon (O.1.28), phorate (O.1.29), phoxim (O.1.30), pirimiphos-methyl (O.1.31), profenofos (O.1.32), prothiofos (O.1.33), sulprophos (O.1.34), tetrachlorvinphos (O.1.35), terbufos (O.1.36), triazophos (O.1.37), trichlorfon (O.1.38);
   - carbamates: alanycarb (O.2.1), aldicarb (O.2.2), bendiocarb (O.2.3), benfuracarb (O.2.4), carbaryl (O.2.5), carbofuran (O.2.6), carbosulfan (O.2.7), fenoxycarb (O.2.8), furathiocarb (O.2.9), methiocarb (O.2.10), methomyl (O.2.11), oxamyl (O.2.12), pirimicarb (O.2.13), propoxur (O.2.14), thiodicarb (O.2.15), triazamate (O.2.16);
   - pyrethroids: allethrin (0.3.1), bifenthrin (O.3.2), cyfluthrin (O.3.3), cyhalothrin (O.3.4), cyphenothrin (O.3.5), cypermethrin (O.3.6), alpha-cypermethrin (O.3.7), beta-cypermethrin (O.3.8), zeta-cypermethrin (O.3.9), deltamethrin (0.3.10), esfenvalerate (0.3.11), etofenprox (0.3.11), fenpropathrin (0.3.12), fenvalerate (0.3.13), imiprothrin (0.3.14), lambda-cyhalothrin (0.3.15), permethrin (0.3.16), prallethrin (0.3.17), pyrethrin I and II (0.3.18), resmethrin (0.3.19), silafluofen (O.3.20), tau-fluvalinate (0.3.21), tefluthrin (O.3.22), tetramethrin (O.3.23), tralomethrin (O.3.24), transfluthrin (O.3.25), profluthrin (O.3.26), dimefluthrin (O.3.27);
   - insect growth regulators: a) chitin synthesis inhibitors: benzoylureas: chlorfluazuron (O.4.1), cyramazin (O.4.2), diflubenzuron (O.4.3), flucycloxuron (O.4.4), flufenoxuron (O.4.5), hexaflumuron (O.4.6), lufenuron (O.4.7), novaluron (O.4.8), teflubenzuron (O.4.9), triflumuron (O.4.10); buprofezin (0.4.11), diofenolan (O.4.12), hexythiazox (O.4.13), etoxazole (O.4.14), clofentazine (O.4.15); b) ecdysone antagonists: halofenozide (O.4.16), methoxyfenozide (O.4.17), tebufenozide (O.4.18), azadirachtin (O.4.19); c) juvenoids: pyriproxyfen (O.4.20), methoprene (O.4.21), fenoxycarb (O.4.22); d) lipid biosynthesis inhibitors: spirodiclofen (O.4.23), spiromesifen (O.4.24), spirotetramat (O.4.24);
   - nicotinic receptor agonists/antagonists compounds: clothianidin (0.5.1), dinotefuran (O.5.2), flupyradifurone (O.5.3), imidacloprid (O.5.4), thiamethoxam (O.5.5), nitenpyram (O.5.6), acetamiprid (O.5.7), thiacloprid (O.5.8), 1-2-chloro-thiazol-5-ylmethyl)-2-nitrimino-3,5-dimethyl-[1,3,5]triazinane (O.5.9);
   - GABA antagonist compounds: endosulfan (0.6.19, ethiprole (O.6.2), fipronil (O.6.3), vaniliprole (O.6.4), pyrafluprole (O.6.5), pyriprole (O.6.6), 5-amino-1-(2,6-dichloro-4-methylphenyl)-4-sulfinamoyl-1H-pyrazole-3-carbothioic acid amide (O.6.7);
   - macrocyclic lactone insecticides: abamectin (0.7.1), emamectin (O.7.2), milbemectin (O.7.3), lepimectin (O.7.4), spinosad (O.7.5), spinetoram (O.7.6);
   - mitochondrial electron transport inhibitor (METI) I acaricides: fenazaquin (0.8.1), pyridaben (O.8.2), tebufenpyrad (O.8.3), tolfenpyrad (O.8.4), flufenerim (O.8.5);
   - METI II and III compounds: acequinocyl (0.9.1), fluacyprim (O.9.2), hydramethylnon (O.9.3);
   - Uncouplers: chlorfenapyr (O.10.1);
   - oxidative phosphorylation inhibitors: cyhexatin (O.11.1), diafenthiuron (O.11.2), fenbutatin oxide (O.11.3), propargite (O.11.4);
   - moulting disruptor compounds: cryomazine (O.12.1);
   - mixed function oxidase inhibitors: piperonyl butoxide (O.13.1);
   - sodium channel blockers: indoxacarb (O.14.1), metaflumizone (O.14.2);
   - ryanodine receptor inhibitors: chlorantraniliprole (O.15.1), cyantraniliprole (O.15.2), flubendiamide (O.15.3), N-[4,6-dichloro-2-[(diethyl-lambda-4-sulfanylidene)carbamoyl]-phenyl]-2-(3-chloro-2-pyridyl)-5-(trifluoromethyl)pyrazole-3-carboxamide (O.15.4); N-[4-chloro-2-[(diethyl-lambda-4-sulfanylidene)carbamoyl]-6-methyl-phenyl]-2-(3-chloro-2-pyridyl)-5-(trifluoromethyl)pyrazole-3-carboxamide (O.15.5); N-[4-chloro-2-[(di-2-propyl-lambda-4-sulfanylidene)carbamoyl]-6-methyl-phenyl]-2-(3-chloro-2-pyridyl)-5-(trifluoromethyl)pyrazole-3-carboxamide (O.15.6); N-[4,6-dichloro-2-[(di-2-propyl-lambda-4-sulfanylidene)carbamoyl]-phenyl]-2-(3-chloro-2-pyridyl)-5-(trifluoromethyl)pyrazole-3-carboxamide (O.15.7); N-[4,6-dichloro-2-[(diethyl-lambda-4-sulfanylidene)carbamoyl]-phenyl]-2-(3-chloro-2-pyridyl)-5-(difluoromethyl)pyrazole-3-carboxamide (O.15.8); N-[4,6-dibromo-2-[(di-2-propyl-lambda-4-sulfanylidene)carbamoyl]-phenyl]-2-(3-chloro-2-pyridyl)-5-(trifluoromethyl)pyrazole-3-carboxamide (O.15.9); N-[4-chloro-2-[(di-2-propyl-lambda-4-sulfanylidene)carbamoyl]-6-cyano-phenyl]-2-(3-chloro-2-pyridyl)-5-(trifluoromethyl)pyrazole-3-carboxamide (O.15.10); N-[4,6-dibromo-2-[(diethyl-lambda-4-sulfanylidene)carbamoyl]-phenyl]-2-(3-chloro-2-pyridyl)-5-(trifluoromethyl)pyrazole-3-carboxamide (O.15.11);
   - others: benclothiaz (O.16.1), bifenazate (O.16.2), artap (O.16.3), flonicamid (O.16.4), pyridalyl (O.16.5), pymetrozine (O.16.6), sulfur (O.16.7), thiocyclam (O.16.8), cyenopyrafen (O.16.9), flupyrazofos (O.16.10), cyflumetofen (O.16.11), amidoflumet (O.16.12), imicyafos (O.16.13), bistrifluron (O.16.14), pyrifluquinazon (O.16.15) and 1,1'-[(3S,4R,4aR,6S,6aS,12R,12aS,12bS)-4-[[(2-cyclopropylacetyl)oxy]methyl]-1,3,4,4a,5,6,6a,12,12a,12b-decahydro-12-hydroxy-4,6a,12b-trimethyl-11-oxo-9-(3-pyridinyl)-2H,11H-naphtho[2,1-b]pyrano[3,4-e]pyran-3,6-diyl] cyclopropaneacetic acid ester (O.16.16); tioxazafen (O.16.17).

The active substances referred to as component 2, their preparation and their activity e. g. against harmful fungi is known (cf.: http://www.alanwood.net/pesticides/); these substances are commercially available. The compounds described by IUPAC nomenclature, their preparation and their pesticidal activity are also known (cf. Can. J. Plant Sci. 48(6), 587-94, 1968; EP-A 141 317; EP-A 152 031; EP-A 226 917; EP-A 243 970; EP-A 256 503; EP-A 428 941; EP-A 532 022; EP-A 1 028 125; EP-A 1 035 122; EP-A 1 201 648; EP-A 1 122 244, JP 2002316902; DE 19650197; DE 10021412; DE 102005009458; US 3,296,272; US 3,325,503; WO 98/46608; WO 99/14187; WO 99/24413; WO 99/27783; WO 00/29404; WO 00/46148; WO 00/65913; WO 01/54501; WO 01/56358; WO 02/22583; WO 02/40431; WO 03/10149; WO 03/11853; WO 03/14103; WO 03/16286; WO 03/53145; WO 03/61388; WO 03/66609; WO 03/74491; WO 04/49804; WO 04/83193; WO 05/120234; WO 05/123689; WO 05/123690; WO 05/63721; WO 05/87772; WO 05/87773; WO 06/15866; WO 06/87325; WO 06/87343; WO 07/82098; WO 07/90624, WO 11/028657, WO2012/168188, WO 2007/006670, WO 2011/77514; WO13/047749, WO 10/069882, WO 13/047441, WO 03/16303, WO 09/90181, WO 13/007767, WO 13/010862, WO 13/127704, WO 13/024009, WO 13/024010 and WO 13/047441, WO 13/162072, WO 13/092224, WO 11/135833).

The present invention furthermore relates to agrochemical compositions comprising a mixture of at least one compound I (component 1) and at least one further active substance useful for plant protection, e. g. selected from the groups A) to O) (component 2), in particular one further fungicide, e. g. one or more fungicide from the groups A) to K), as described above, and if desired one suitable solvent or solid carrier. Those mixtures are of particular interest, since many of them at the same application rate show higher efficiencies against harmful fungi. Furthermore, combating harmful fungi with a mixture of compounds I and at least one fungicide from groups A) to K), as described above, is more efficient than combating those fungi with individual compounds I or individual fungicides from groups A) to K).

By applying compounds I together with at least one active substance from groups A) to O) a synergistic effect can be obtained, i.e. more then simple addition of the individual effects is obtained (synergistic mixtures).

This can be obtained by applying the compounds I and at least one further active substance simultaneously, either jointly (e. g. as tank-mix) or seperately, or in succession, wherein the time interval between the individual applications is selected to ensure that the active substance applied first still occurs at the site of action in a sufficient amount at the time of application of the further active substance(s). The order of application is not essential for working of the present invention.

When applying compound I and a pesticide II sequentially the time between both applications may vary e. g. between 2 hours to 7 days. Also a broader range is possible ranging from 0.25 hour to 30 days, preferably from 0.5 hour to 14 days, particularly from 1 hour to 7 days or from 1.5 hours to 5 days, even more preferred from 2 hours to 1 day.

In the binary mixtures and compositions according to the invention the weight ratio of the component 1) and the component 2) generally depends from the properties of the active components used, usually it is in the range of from 1:100 to 100:1, regularly in the range of from 1:50 to 50:1, preferably in the range of from 1:20 to 20:1, more preferably in the range of from 1:10 to 10:1, even more preferably in the range of from 1:4 to 4:1 and in particular in the range of from 1:2 to 2:1.

According to further embodiments of the binary mixtures and compositions, the weight ratio of the component 1) and the component 2) usually is in the range of from 1000:1 to 1:1, often in the range of from 100: 1 to 1:1, regularly in the range of from 50:1 to 1:1, preferably in the range of from 20:1 to 1:1, more preferably in the range of from 10:1 to 1:1, even more preferably in the range of from 4:1 to 1:1 and in particular in the range of from 2:1 to 1:1.

According to a further embodiments of the binary mixtures and compositions, the weight ratio of the component 1) and the component 2) usually is in the range of from 1:1 to 1:1000, often in the range of from 1:1 to 1:100, regularly in the range of from 1:1 to 1:50, preferably in the range of from 1:1 to 1:20, more preferably in the range of from 1:1 to 1:10, even more preferably in the range of from 1:1 to 1:4 and in particular in the range of from 1:1 to 1:2.

According to further embodiments of the mixtures and compositions, the weight ratio of the component 1) and the component 2) generally depends from the properties of the active components used, usually it is in the range of from 1:10,000 to 10,000:1, regularly in the range of from 1:100 to 10,000:1, preferably in the range of from 1:100 to 5,000:1, more preferably in the range of from 1:1 to 1,000:1, even more preferably in the range of from 1:1 to 500:1 and in particular in the range of from 10:1 to 300:1.

According to further embodiments of the mixtures and compositions, the weight ratio of the component 1) and the component 2) usually is in the range of from 20,000:1 to 1:10, often in the range of from 10,000:1 to 1:1, regularly in the range of from 5,000:1 to 5:1, preferably in the range of from 5,000:1 to 10:1, more preferably in the range of from 2,000:1 to 30:1, even more preferably in the range of from 2,000:1 to 100:1 and in particular in the range of from 1,000:1 to 100:1.

According to further embodiments of the mixtures and compositions, the weight ratio of the component 1) and the component 2) usually is in the range of from 1:20,000 to 10:1, often in the range of from 1:10,000 to 1:1, regularly in the range of from 1:5,000 to 1:5, preferably in the range of from 1:5,000 to 1:10, more preferably in the range of from 1:2,000 to 1:30, even more preferably in the range of from 1:2,000 to 1:100 and in particular in the range of from 1:1,000 to 1:100.

In the ternary mixtures, i.e. compositions according to the invention comprising the component 1) and component 2) and a compound III (component 3), the weight ratio of component 1) and component 2) depends from the properties of the active substances used, usually it is in the range of from 1:100 to 100:1, regularly in the range of from 1:50 to 50:1, preferably in the range of from 1:20 to 20:1, more preferably in the range of from 1:10 to 10:1 and in particular in the range of from 1:4 to 4:1, and the weight ratio of component 1) and component 3) usually it is in the range of from 1:100 to 100:1, regularly in the range of from 1:50 to 50:1, preferably in the range of from 1:20 to 20:1, more preferably in the range of from 1:10 to 10:1 and in particular in the range of from 1:4 to 4:1.

Any further active components are, if desired, added in a ratio of from 20:1 to 1:20 to the component 1).

These ratios are also suitable for inventive mixtures applied by seed treatment.

When mixtures comprising microbial pesticides are employed in crop protection, the application rates preferably range from about 1 x 10⁶ to 5 x 10¹⁵ (or more) CFU/ha, preferably from about 1 x 10⁸ to about 1 x 10¹³ CFU/ha, and even more preferably from about 1 x 10⁹ to about 1 x 10¹² CFU/ha. In the case of (entomopathogenic) nematodes as microbial pesticides (e. g. Steinernema feltiae), the application rates preferably range inform about 1 x 10⁵ to 1 x 10¹² (or more), more preferably from 1 x 10⁸ to 1 x 10¹¹, even more preferably from 5 x 10³ to 1 x 10¹⁰ individuals (e. g. in the form of eggs, juvenile or any other live stages, preferably in an infetive juvenile stage) per ha.

When mixtures comprising microbial pesticides are employed in seed treatment, the application rates with respect to plant propagation material preferably range from about 1 x 10⁶ to 1 x 10¹² (or more) CFU/seed. Preferably, the concentration is about 1 x 10⁶ to about 1 x 10⁹ CFU/seed. In the case of the microbial pesticides II, the application rates with respect to plant propagation material also preferably range from about 1 x 10⁷ to 1 x 10¹⁴ (or more) CFU per 100 kg of seed, preferably from 1 x 10⁹ to about 1 x 10¹² CFU per 100 kg of seed.

Preference is also given to mixtures comprising as component 2) at least one active substance selected from group A), which is particularly selected from (A.1.1), (A.1.4), (A.1.8), (A.1.9), (A.1.12), (A.1.13), (A.1.14), (A.1.17), (A.1.19), (A.1.21), (A.2.1), (A.2.2), (A.3.2), (A.3.3), (A.3.4), (A.3.7), (A.3.8), (A.3.9), (A.3.12), (A.3.14), (A.3.15), (A.3.16), (A.3.19), (A.3.20), (A.3.21), (A.3.22), (A.3.23), (A.3.24), (A.3.25), (A.3.26), (A.3.27); (A.4.5), (A.4.6), (A.4.8), (A.4.9), (A.4.11), (A.1.23), (A.1.24) and (A.1.25).

Preference is given to mixtures as component 2) at least one active substance selected from group B), which is particularly selected from (B.1.4), (B.1.5), diniconazole (B.1.6), (B.1.8), (B.1.10), (B.1.11), (B.1.12), (B.1.17), (B.1.18), (B.1.21), (B.1.22), (B.1.23), (B.1.25), (B.1.26), (B.1.27), (B.1.28), (B.1.29), uni (B.1.31), (B.1.32), (B.1.33), (B.1.34), (B.1.35), (B.1.36), (B.1.37), (B.1.38), (B.1.39), (B.1.40), (B.1.41), (B.1.42), (B.1.44), (B.1.46), (B.1.49) and (B.1.50; (B.2.2), (B.2.4), (B.2.5), (B.2.6), piperalin (B.2.7), (B.2.8); and (B.3.1).

Preference is given to mixtures comprising as component 2) at least one active substance selected from group C), which is particularly selected from (C.1.4), C.1.5), (C.1.6), and (C.2.4).

Preference is given to mixtures comprising as component 2) at least one active substance selected from group D), which is particularly selected from (D1.1), (D1.2), (D1.4), (D1.5); (D2.2), (D2.4), (D2.5), (D2.6) and (D2.7);

Preference is also given to mixtures comprising as component 2) at least one active substance selected from group E), which is particularly selected from (E.1.1), (E.1.2), and (E.1.3);

Preference is also given to mixtures comprising as component 2) at least one active substance selected from group F), which is particularly selected from (F.1.2), (F.1.4), (F.1.5), (F.1.6) and (F.2.1).

Preference is also given to mixtures as component 2) at least one active substance selected from group G), which is particularly selected from (G.3.1), (G.3.2), (G.3.3), (G.3.4), (G.3.5), (G.3.6), (G.4.1) and (G.5.1).

Preference is also given to mixtures comprising as component 2) at least one active substance selected from group H), which is and particularly selected from (H.1.2), (H.1.3), copper oxychloride (H.1.4), (H.1.5), (H.1.6); (H.2.2), (H.2.5), (H.2.7), (H.3.2), (H.3.3), (H.3.4), (H.3.5), (H.3.6), (H.3.12); (H.4.2), (H.4.6), dithianon (H.4.9) and (H.4.10).

Preference is also given to mixtures comprising as component 2) at least one active substance selected from group I), which is particularly selected from (1.2.3) and (1.2.5).

Preference is also given to mixtures comprising as component 2) at least one active substance selected from group J), which is particularly selected from (J.1.1), (J.1.2), (J.1.3), (J.1.4), (J.1.6), (J.1.7), (J.1.8) and (J.1.9).

Preference is also given to mixtures comprising as component 2) at least one active substance selected from group K), which is particularly selected from (K.1.4), (K.1.5), (K.1.8), (K.1.12), (K.1.14), (K.1.15), (K.1.19) and(K.1.22).

Accordingly, the present invention furthermore relates to compositions comprising one compound I (component 1) and one pesticide II (component 2), which pesticide II is selected from the column "Co. 2" of the lines C-1 to C-597 of Table C.

A further embodiment relates to the compositions C-1 to C-597 listed in Table C, where a row of Table C corresponds in each case to a fungicidal composition comprising as active components one of the in the present specification individualized compounds of formula I (component 1) and the respective pesticide II from groups A) to O) (component 2) stated in the row in question. Preferably, the compositions described comprise the active components in synergistically effective amounts.

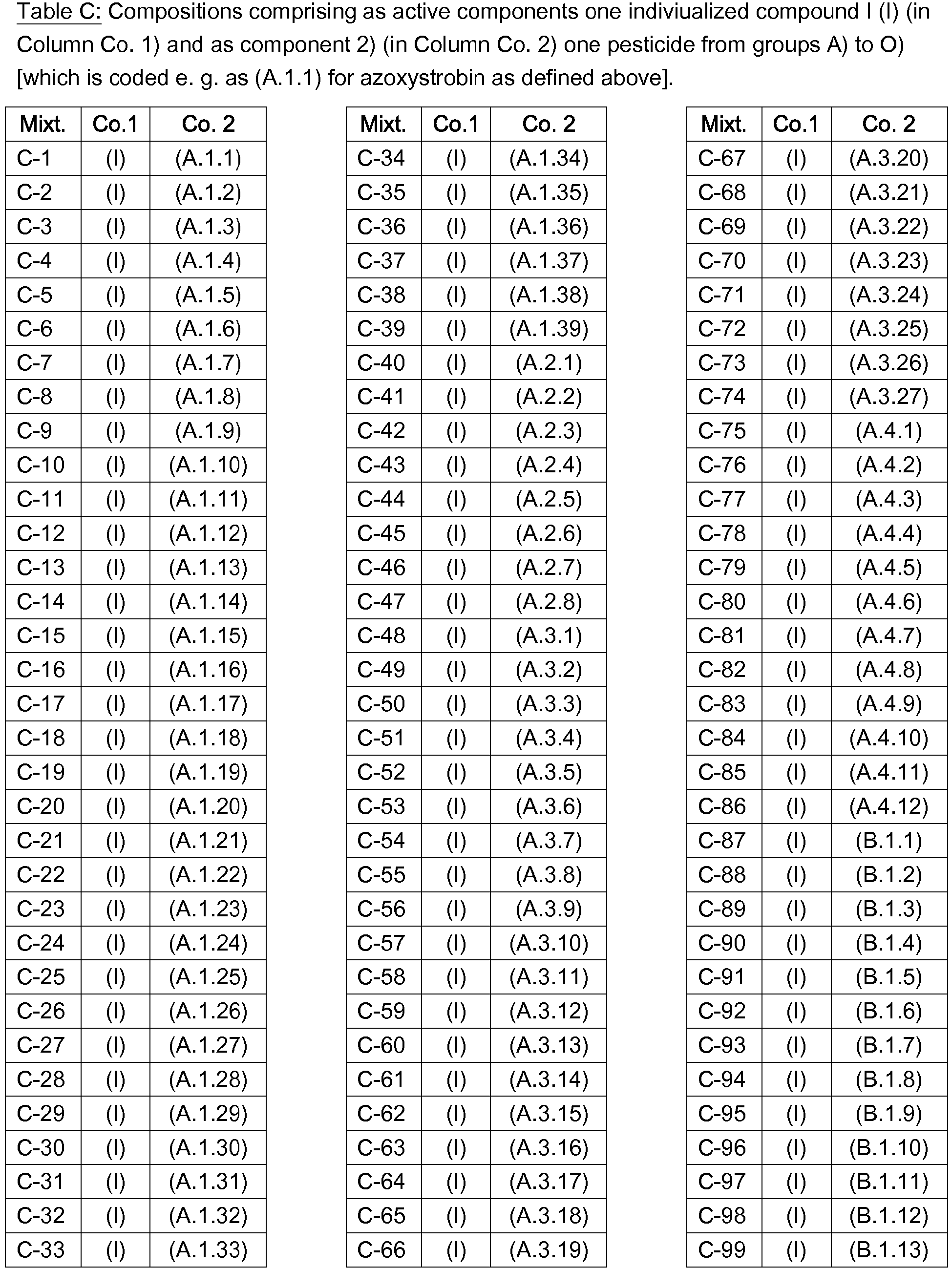

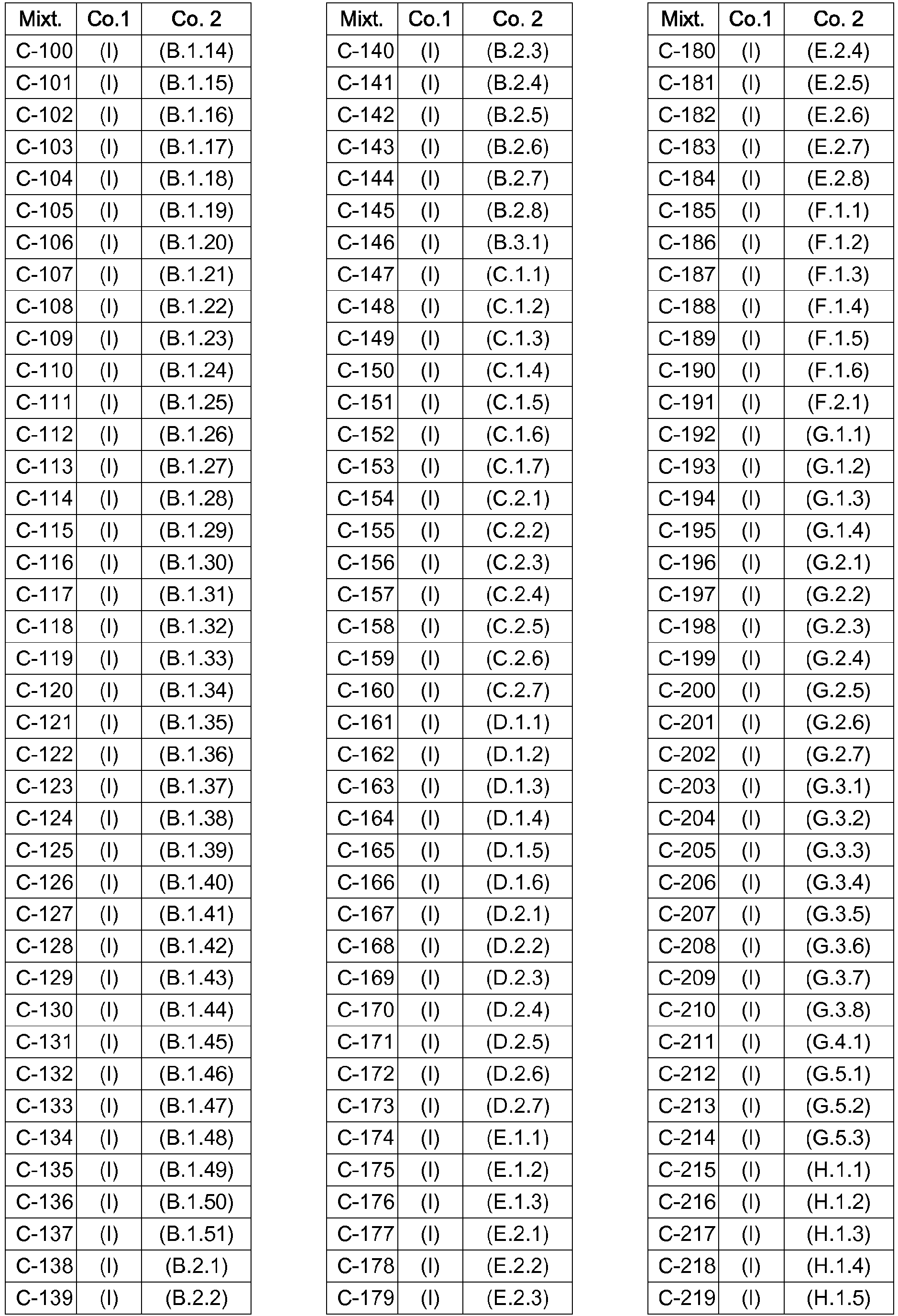

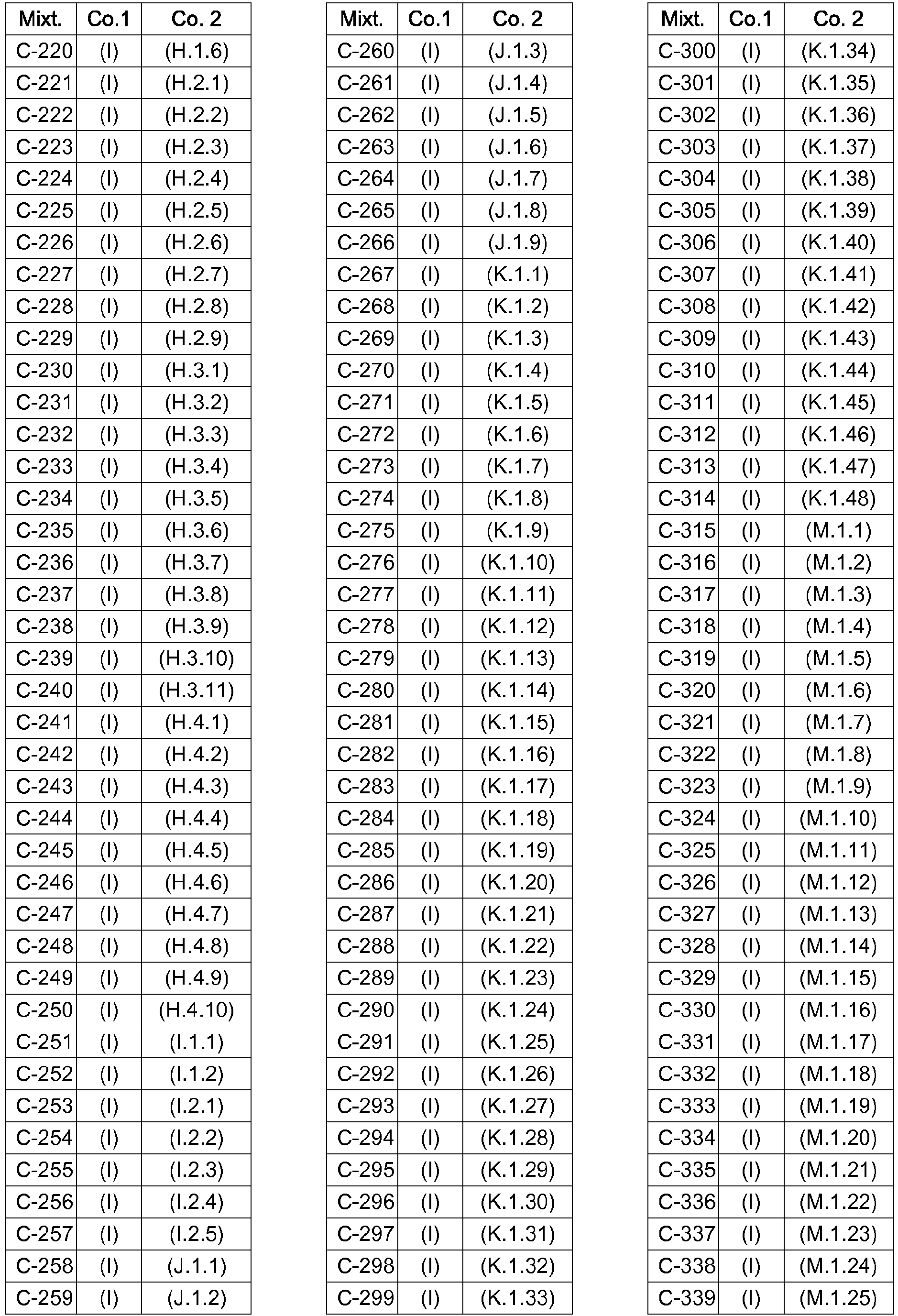

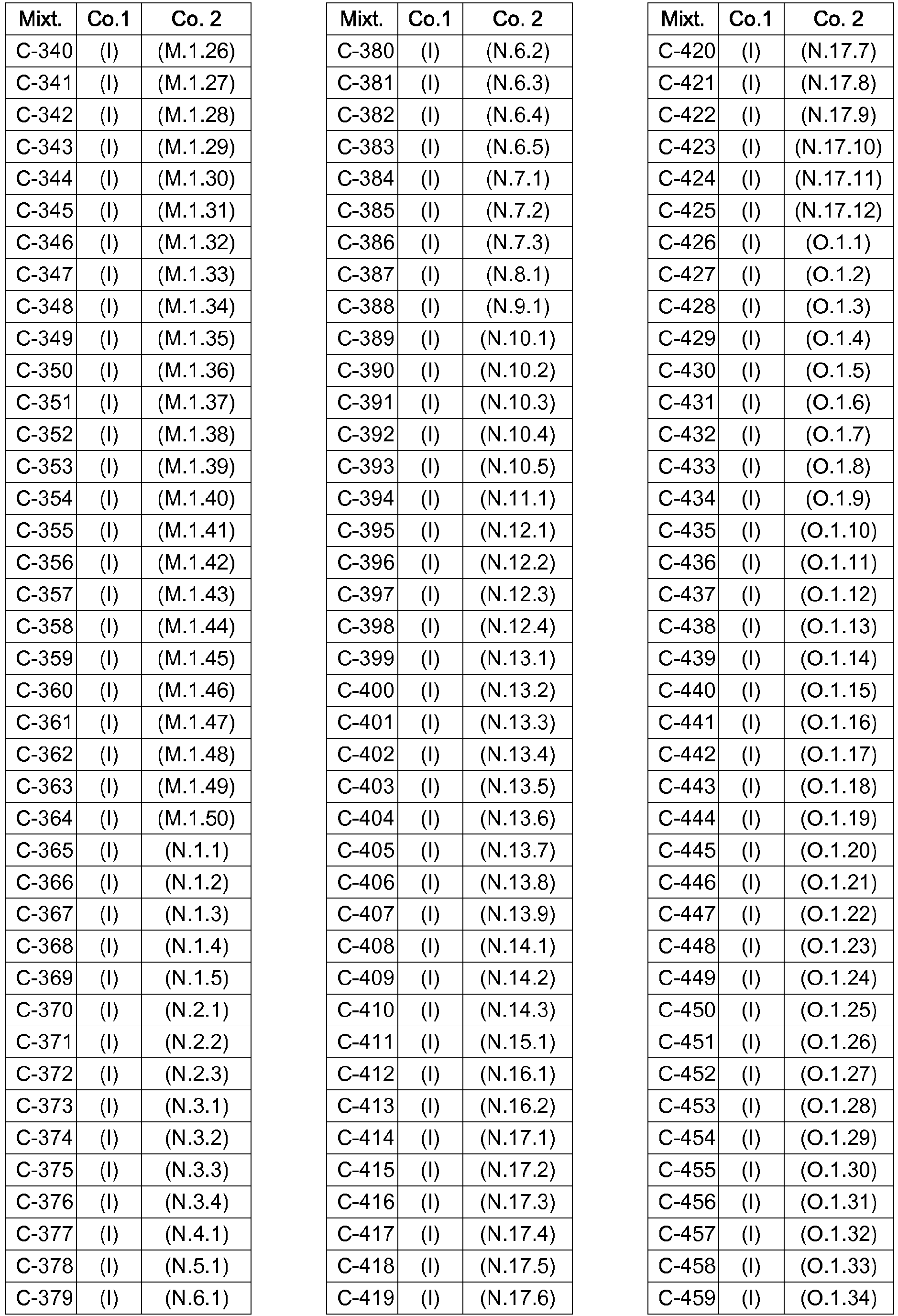

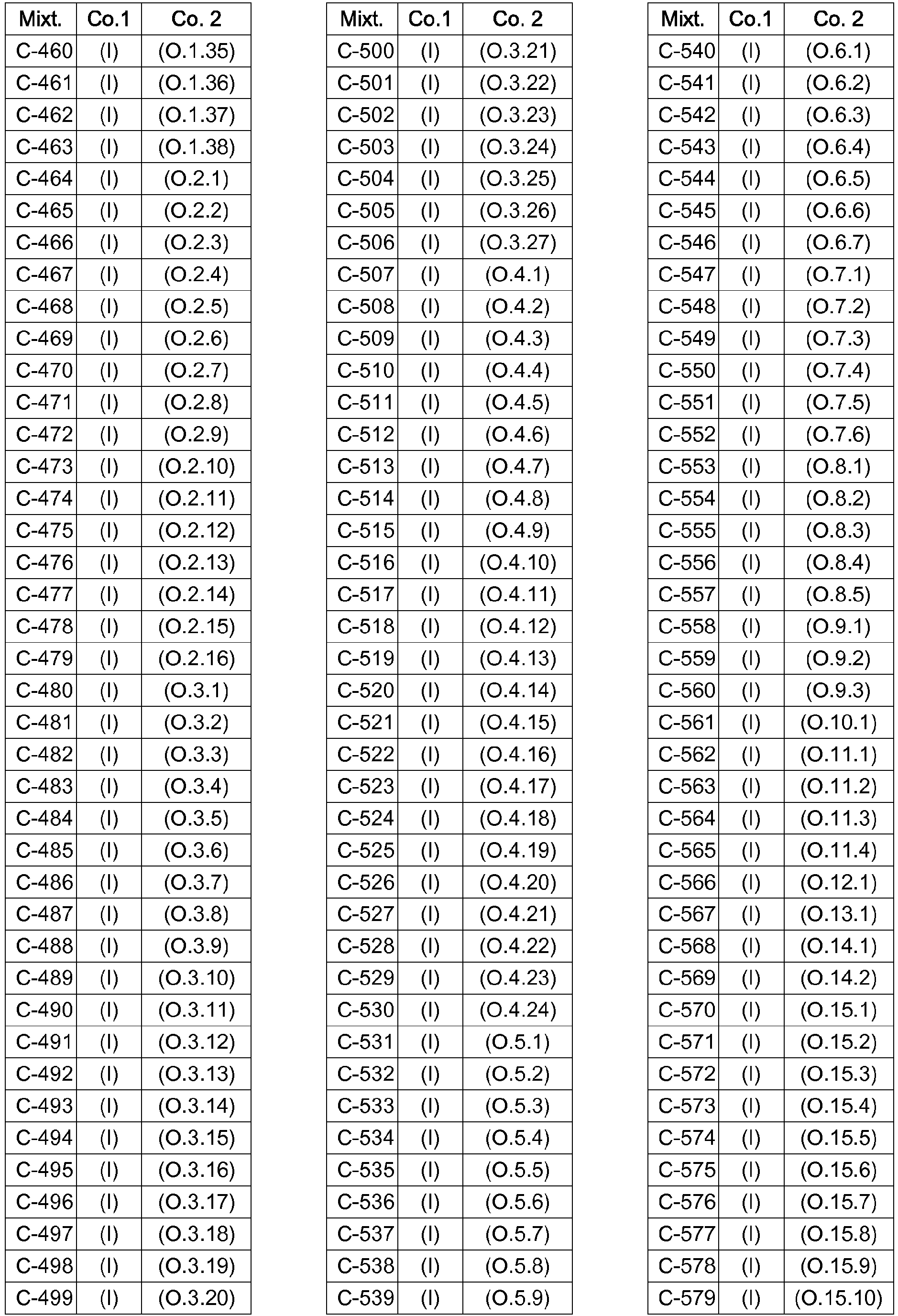

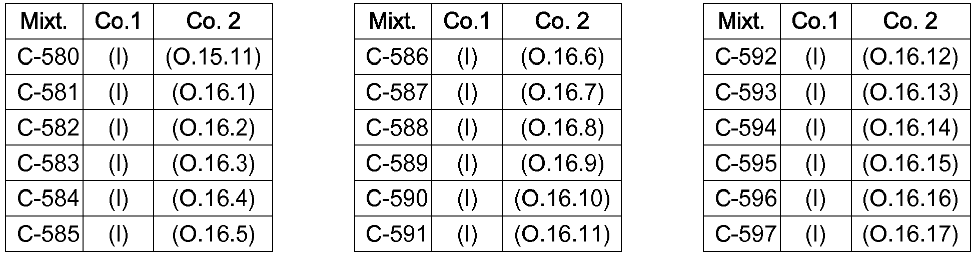

The mixtures of active substances can be prepared as compositions comprising besides the active ingredients at least one inert ingredient (auxiliary) by usual means, e. g. by the means given for the compositions of compounds I.

Concerning usual ingredients of such compositions reference is made to the explanations given for the compositions containing compounds I.

The mixtures of active substances according to the present invention are suitable as fungicides, as are the compounds of formula I. They are distinguished by an outstanding effectiveness against a broad spectrum of phytopathogenic fungi, especially from the classes of the Ascomy-cetes, Basidiomycetes, Deuteromycetes and Peronosporomycetes (syn. Oomycetes). In addition, it is refered to the explanations regarding the fungicidal activity of the compounds and the compositions containing compounds I, respectively.

### Synthesis examples

With due modification of the starting compounds, the procedures shown in the synthesis examples below were used to obtain further compounds I. The resulting compounds, together with physical data, are listed in Table I below.

### Example 1: Compound I-1

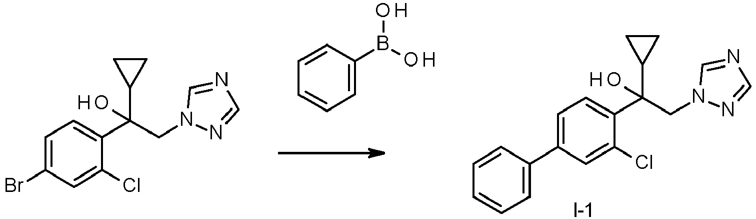

A round-bottom flask was charged with Pd(PPh₃)₄ (168 mg, 10 mol%), Pd(dppf)Cl2 (106 mg, 10 mol%) and solutions of 1-(4-bromo-2-chloro-phenyl)-1- cyclopropyl-2-(1,2,4-triazol-1-yl)ethanol 500 mg) and phenyl boronic acid (267 mg, 1.5 eq) in 1,2-dimethoxyethane (5 mL each) were added. A solution of NaCO₃ (387 mg, 2.5 eq) in water (2 mL) was added and the mixture was warmed to 90 °C. When HPLC indicated complete conversion, the mixture was cooled to ambient temperature and quenched by the addition of a saturated solution of NH₄Cl. The product was extracted into MTBE, the combined organic extracts were dried over MgSO₄ and concentrated under reduced pressure. Purification of the residue by column chromatography yielded the title compound as oil (370 mg, 74%). HPLC-MS: t_{R} = 1.159 min; ¹H NMR (400 MHz, CDCl₃): δ [ppm] = 0.20-0.30 (1H), 0.35-0.55 (2H), 0.60-0.70 (1H), 1.75-1.85 (1H), 4.50 (1H), 4.62 (1H), 5.40 (1H), 7.30-7.45 (5H), 7.50-7.60 (2H), 7.70 (1H), 7.85 (1H), 8.10 (1H).

| Compound No. | R¹ | R² | R³ | A | HPLC-MS: |
|---|---|---|---|---|---|
| | | | | | t_{R} = [min.]**; |
| | | | | | ¹H NMR (400 MHz, CDCl₃): δ [ppm] |
| I-1 | C₃H₅ (cyclopropyl) | H | Cl | N | 1.159; |
| | | | | | 0.20-0.30 (1H), 0.35-0.55 (2H), 0.60-0.70 (1H), 1.75-1.85 (1H), 4.50 (1H), 4.62 (1H), 5.40 (1H), 7.30-7.45 (5H), 7.50-7.60 (2H), 7.70 (1H), 7.85(1 H), 8.10 (1H) |
| I-2 | C₃H₅ (cyclopropyl) | H | CF₃ | N | 1.173 |
| I-3 | CH₃ | H | CF₃ | N | 1.072 |
| I-4 | CH(CH₃)₂ | H | CF₃ | N | 1.207 |
| I-5 | C(CH₃)₃ | H | CF₃ | N | 1.300 |
| I-6 | C≡CCH₃ | H | CF₃ | N | 1.100 |
| I-7 | C(CH₃)₃ | H | Cl | N | 1.316 |
| I-8 | CH₃ | H | Cl | N | 1.067 |
| I-9 | CH₂CH₂CH=CH₂ | H | Cl | N | 1.197 |
| I-10 | C≡CCH₃ | H | Cl | N | 1.067 |
| I-11 | C≡CH | H | Cl | N | 1.024 |
| I-12 | C≡C-I | H | Cl | N | 1.114 |
| I-13 | C₂H₅ | H | Cl | N | 1.139 |
| I-14 | CF₂CH₃ | H | Cl | N | 1.141 |

| | | | | | |
|---|---|---|---|---|---|
| ** :HPLC method Data: | | | | | |

Mobile Phase: A: Water + 0.1% TFA; B: acetonitrile; Gradient: 5% B to 100% B in 1.5min; Temperature: 60 °C; MS-Method: ESI positive; mass area (m/z): 100-700; Flow: 0.8ml/min to 1.0ml/min in 1.5min; Column: Kinetex XB C18 1.7µ 50 x 2.1mm; Aparatus: Shimadzu Nexera LC-30 LCMS-2020.

### II. Examples of the action against harmful fungi

The fungicidal action of the compounds of the formula I was demonstrated by the following experiments:

### Green House

**The spray solutions were prepared in several steps:**
The stock solution were prepared: a mixture of acetone and/or dimethylsulfoxide and the wetting agent/emulsifier Wettol, which is based on ethoxylated alkylphenoles, in a relation (volume) solvent-emulsifier of 99 to 1 was added to 25 mg of the compound to give a total of 5 ml. Water was then added to total volume of 100 ml.

This stock solution was diluted with the described solvent-emulsifier-water mixture to the given concentration.

### G1. Preventative fungicidal control of Botrytis cinerea on leaves of green pepper (Botrci P1)

Young seedlings of green pepper were grown in pots to the 4 to 5 leaf stage. These plants were sprayed to run-off with an aqueous suspension, containing the concentration of active ingredient or their mixture mentioned in the table below. The next day the plants were inoculated with an aqueous biomalt solution containing the spore suspension of *Botrytis cinerea.* Then the plants were immediately transferred to a humid chamber. After 5 days at 22 to 24□C and a relative humidity close to 100 % the extent of fungal attack on the leaves was visually assessed as % diseased leaf area. In this test, the plants which had been treated with 150 ppm of the active substance I-7, I-8, I-9, I-1, I-10, I-11, I-12, I-13, I-2, I-3, I-4, I-5 and I-14, respectively, showed an infection of 7 % or less whereas the untreated plants were 80% infected.

### G2. Preventative fungicidal control of early blight on tomatoes (Alternaria solani) (Alteso P1)

Young seedlings of tomato plants were grown in pots. These plants were sprayed to run-off with an aqueous suspension, containing the concentration of active ingredient or mixture mentioned in the table below. The next day, the treated plants were inoculated with an aqueous suspension of *Alternaria solani.* Then the trial plants were immediately transferred to a humid chamber. After 5 days at 18 to 20° C and a relative humidity close to 100 %, the extent of fungal attack on the leaves was visually assessed as % diseased leaf area. In this test, the plants which had been treated with 150 ppm of the active substance I-7, I-8, I-9, I-1, I-10, I-11, I-12, I-13, I-2, I-3, I-4, I-5, I-6 and I-14, respectively, showed an infection of 15 % or less whereas the untreated plants were 80% infected.

### G3. Curative control of soy bean rust on soy beans caused by Phakopsora pachyrhizi (Phakpa K2)

Leaves of pot-grown soy bean seedlings were inoculated with spores of *Phakopsora pachyrhizi.* To ensure the success of the artificial inoculation, the plants were transferred to a humid chamber with a relative humidity of about 95 % and 20 to 24□ C for 24 h. The next day the plants were cultivated for 1 days in a greenhouse chamber at 23-27°C and a relative humidity between 60 and 80 %. Then the plants were sprayed to run-off with an aqueous suspension, containing the concentration of active ingredient or their mixture as described below. The plants were allowed to air-dry. Then the trial plants were cultivated for 14 days in a greenhouse chamber at 23-27°C and a relative humidity between 60 and 80 %. The extent of fungal attack on the leaves was visually assessed as % diseased leaf area. In this test, the plants which had been treated with 150 ppm of the active substance I-7, I-8, I-9, I-1, I-10 and I-11, respectively, showed an infection of 3 % or less whereas the untreated plants were 100% infected.

### Microtest

The active compounds were formulated separately as a stock solution having a concentration of 10000 ppm in dimethyl sulfoxide.

### M1 Activity against the grey mold Botrytis cinerea in the microtiterplate test (Botrci)

The stock solutions were mixed according to the ratio, pipetted onto a micro titer plate (MTP) and diluted with water to the stated concentrations. A spore suspension of *Botrci cinerea* in an aqueous biomalt or yeast-bactopeptone-sodiumacetate solution was then added. The plates were placed in a water vapor-saturated chamber at a temperature of 18°C. Using an absorption photometer, the MTPs were measured at 405 nm 7 days after the inoculation. Compounds I-7, I-8, I-9, I-1, I-10, I-13 and I-14, respectively, showed a growth of 0 % at 31 ppm.

### M2 Activity against rice blast Pyricularia oryzae in the microtiterplate test (Pyrior)

The stock solutions were mixed according to the ratio, pipetted onto a micro titer plate (MTP) and diluted with water to the stated concentrations. A spore suspension of *Pyricularia oryzae* in an aqueous biomalt or yeast-bactopeptone-glycerine solution was then added. The plates were placed in a water vapor-saturated chamber at a temperature of 18°C. Using an absorption photometer, the MTPs were measured at 405 nm 7 days after the inoculation. Compounds I-7, I-8, I-9, I-1, I-10, I-13 and I-14, respectively, showed a growth of 2 % or less at 31 ppm.

### M3 Activity against leaf blotch on wheat caused by Septoria tritici (Septtr)

The stock solutions were mixed according to the ratio, pipetted onto a micro titer plate (MTP) and diluted with water to the stated concentrations. A spore suspension of *Septoria tritici* in an aqueous biomalt or yeast-bactopeptone-glycerine solution was then added. The plates were placed in a water vapor-saturated chamber at a temperature of 18°C. Using an absorption photometer, the MTPs were measured at 405 nm 7 days after the inoculation. Compounds I-7, I-8, I-9, I-1, I-10, I-13 and I-14, respectively, showed a growth of 4 % or less at 31 ppm.

The measured parameters were compared to the growth of the active compound-free control variant (100%) and the fungus-free and active compound-free blank value to determine the relative growth in % of the pathogens in the respective active compounds.

## Claims

1. Compounds of the formula I 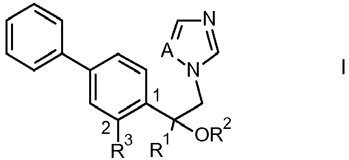 wherein
A is CH or N;
R¹ is C₁-C₆-alkyl, C₂-C₆-alkenyl, C₂-C₆-alkynyl or C₃-C₆-cycloalkyl;
wherein the aliphatic moieties of R¹ are not further substituted or carry one, two, three or up to the maximum possible number of identical or different groups R^{1a} which independently of one another are selected from:
R^{1a} halogen, OH, CN, C₁-C₄-alkoxy, C₃-C₆-cycloalkyl, C₃-C₆-halogencycloalkyl and C₁-C₄-halogenalkoxy;
wherein the cycloalkyl moieties of R¹ are not further substituted or carry one, two, three, four, five or up to the maximum number of identical or different groups R^{1b} which independently of one another are selected from:
R^{1b} halogen, OH, CN, C₁-C₄-alkyl, C₁-C₄-alkoxy, C₁-C₄-halogenalkyl, C₃-C₆-cycloalkyl, C₃-C₆-halogencycloalkyl and C₁-C₄-halogenalkoxy;
R² is hydrogen, C₁-C₄-alkyl, C₂-C₄-alkenyl or C₂-C₄-alkynyl;
wherein the aliphatic moieties of R² are not further substituted or carry one, two, three or up to the maximum possible number of identical or different groups R^{2a} which independently of one another are selected from:
R^{2a} halogen, OH, CN, C₁-C₄-alkoxy, C₃-C₆-cycloalkyl, C₃-C₆-halogencycloalkyl and C₁-C₄-halogenalkoxy;
R³ is selected from halogen, CN, C₁-C₄-alkyl, C₁-C₄-alkoxy, C₂-C₄-alkenyl, C₂-C₄-alkynyl, C₃-C₆-cycloalkyl and S(O)ₚ(C₁-C₄-alkyl), wherein each of R³ is unsubstituted or further substituted by one, two, three or four R^{3a}; wherein
R^{3a} is independently selected from halogen, CN, OH, C₁-C₄-alkyl, C₁-C₄-halogenalkyl, C₃-C₆-cycloalkyl, C₃-C₆-halogencycloalkyl, C₁-C₄-alkoxy and C₁-C₄-halogenalkoxy;
p is 0, 1 or 2;
and the N-oxides and the agriculturally acceptable salts thereof.

2. The compounds of claim 1, wherein A is N.

3. The compounds of claim 1 or 2, wherein R³ is F, Cl, Br or CN.

4. The compounds of claim 1 or 2, wherein R³ is C₁-C₄-alkyl or C₁-C₄-halogenalkyl.

5. The compounds of claim 1 or 2, wherein R³ is C₁-C₄-alkoxy or S(C₁-C₄-alkyl).

6. The compounds of any one of claims 1 to 5, wherein R¹ is C₁-C₆-alkyl, that is not further substituted or carries one, two, three, four or five identical or different groups R^{1a}. independently selected from F, OH, CN, C₃-C₆-cycloalkyl, C₃-C₆-halogencycloalkyl and C₁-C₂-halogenalkoxy.

7. The compounds of any one of claims 1 to 5, wherein R¹ is 1-(C₂-C₆)-alkenyl or 1-(C₂-C₆)-alkynyl, that are not further substituted or carry one, two, three, four or five identical or different groups R^{1a}.

8. The compounds of any one of claims 1 to 5, wherein R¹ is C₃-C₆-cycloalkyl, that is not further substituted or carries one, two, three, four or five identical or different groups R^{1b} independently selected from OH, C₁-C₄-alkyl, C₁-C₂-alkoxy, C₁-C₂-halogenalkyl, C₃-C₆-cycloalkyl, C₃-C₆-halogencycloalkyl and C₁-C₂-halogenalkoxy.

9. The compounds of claim 1, wherein R^{a} is F, OH, CN, C₃-C₆-cycloalkyl, C₃-C₆-halogencycloalkyl and C₁-C₂-halogenalkoxy and R^{b} is OH, C₁-C₄-alkyl, C₁-C₂-alkoxy, C₁-C₂-halogenalkyl, C₃-C₆-cycloalkyl, C₃-C₆-halogencycloalkyl and C₁-C₂-halogenalkoxy.

10. The compounds of any one of claims 1 to 9, wherein R² is hydrogen.

11. A composition, comprising one compound of formula I, as defined in any of the claims 1 to 10, an N-oxide or an agriculturally acceptable salt thereof.

12. The composition according to claim 11, comprising additionally a further active substance.

13. A use of a compound of the formula I, as defined in any of the claims 1 to 10, and/or of an agriculturally acceptable salt thereof or of the compositions, as defined in any of the claims 11 or 12, for combating phytopathogenic fungi.

14. A method for combating phytopathogenic fungi, comprising treating the fungi or the materials, plants, the soil or seeds to be protected against fungal attack with an effective amount of at least one compound of formula I, as defined in any of the claims 1 to 10 or with a composition, as defined in any of the claims 11 or 12.

15. Seed, coated with at least one compound of the formula I, as defined in any of the claims 1 to 10, and/or an agriculturally acceptable salt thereof or with a composition, as defined in any of the claims 11 or 12, in an amount of from 0.1 to 10 kg per 100 kg of seed.

## Patentansprüche

1. Verbindung der Formel I 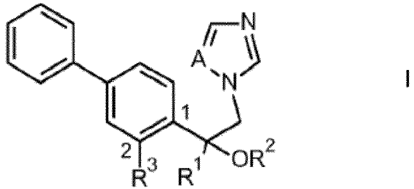 mit den folgenden Bedeutungen:
A bedeutet CH oder N;
R¹ bedeutet C₁-C₆-Alkyl, C₂-C₆-Alkenyl, C₂-C₆-Alkinyl oder C₃-C₆-Cycloalkyl; wobei die aliphatischen Reste von R¹ nicht weiter substituiert sind oder eine, zwei, drei oder bis zur maximal möglichen Anzahl identische oder unterschiedliche Gruppen R^{1a} tragen, die unabhängig voneinander aus den Folgenden ausgewählt sind:
R^{1a} Halogen, OH, CN, C₁-C₄-Alkoxy, C₃-C₆-Cyclo-alkyl, C₃-C₆-Halogencycloalkyl und C₁-C₄-Halogenalkoxy;
wobei die Cycloalkylreste von R¹ nicht weiter substituiert sind oder einen, zwei, drei, vier, fünf oder bis zur maximal möglichen Anzahl identische oder unterschiedliche Gruppen R^{1b} tragen, die unabhängig voneinander aus den Folgenden ausgewählt sind:
R^{1b} Halogen, OH, CN, C₁-C₄-Alkyl, C₁-C₄-Alkoxy, C₁-C₄-Halogenalkyl, C₃-C₆-Cycloalkyl, C₃-C₆-Halogencycloalkyl und C₁-C₄-Halogenalkoxy;
R² bedeutet Wasserstoff, C₁-C₄-Alkyl, C₂-C₄-Alkenyl oder C₂-C₄-Alkinyl; wobei die aliphatischen Reste von R² nicht weiter substituiert sind oder eine, zwei, drei oder bis zur maximal möglichen Anzahl identische oder unterschiedliche Gruppen R^{2a} tragen, die unabhängig voneinander aus den Folgenden ausgewählt sind:
R^{2a} Halogen, OH, CN, C₁-C₄-Alkoxy, C₃-C₆-Cycloalkyl, C₃-C₆-Halogencycloalkyl und C₁-C₄-Halogenalkoxy;
R³ ist aus Halogen, CN, C₁-C₄-Alkyl, C₁-C₄-Alkoxy, C₂-C₄-Alkenyl, C₂-C₄-Alkinyl, C₃-C₆-Cycloalkyl und S(O)ₚ(C₁-C₄-Alkyl) ausgewählt, wobei jedes R³ unsubstituiert oder weiter durch ein, zwei, drei oder vier R^{3a} substituiert ist; wobei
R^{3a} unabhängig aus Halogen, CN, OH, C₁-C₄-Alkyl, C₁-C₄-Halogenalkyl, C₃-C₆-Cycloalkyl, C₃-C₆-Halogencycloalkyl, C₁-C₄-Alkoxy und C₁-C₄-Halogenalkoxy ausgewählt ist;
p 0, 1 oder 2 bedeutet;
und N-Oxide und landwirtschaftlich annehmbare Salze davon.

2. Verbindungen nach Anspruch 1, wobei A N bedeutet.

3. Verbindungen nach Anspruch 1 oder 2, wobei R³ F, Cl, Br oder CN bedeutet.

4. Verbindungen nach Anspruch 1 oder 2, wobei R³ C₁-C₄-Alkyl oder C₁-C₄-Halogenalkyl bedeutet.

5. Verbindungen nach Anspruch 1 oder 2, wobei R³ C₁-C₄-Alkoxy oder S(C₁-C₄-Alkyl) bedeutet.

6. Verbindungen nach einem der Ansprüche 1 bis 5, wobei R¹ C₁-C₆-Alkyl, das nicht weiter substituiert ist oder eine, zwei, drei, vier oder fünf gleiche oder verschiedene Gruppen R^{1a}, die unabhängig aus F, OH, CN, C₃-C₆-Cycloalkyl, C₃-C₆-Halogencycloalkyl und C₁-C₂-Halogenalkoxy ausgewählt sind, trägt, bedeutet.

7. Verbindungen nach einem der Ansprüche 1 bis 5, wobei R¹ 1-(C₂-C₆)-Alkenyl oder 1-(C₂-C₆)-Alkinyl, die nicht weiter substituiert sind oder eine, zwei, drei, vier oder fünf gleiche oder verschiedene Gruppen R^{1a} tragen, bedeutet.

8. Verbindungen nach einem der Ansprüche 1 bis 5, wobei R¹ C₃-C₆-Cycloalkyl, das nicht weiter substituiert ist oder eine, zwei, drei, vier oder fünf gleiche oder verschiedene Gruppen R^{1b}, die unabhängig aus OH, C₁-C₄-Alkyl, C₁-C₂-Alkoxy, C₁-C₂-Halogenalkyl, C₃-C₆-Cycloalkyl, C₃-C₆-Halogencycloalkyl und C₁-C₂-Halogenalkoxy ausgewählt sind, trägt, bedeutet.

9. Verbindungen nach Anspruch 1, wobei R^{a} F, OH, CN, C₃-C₆-Cycloalkyl, C₃-C₆-Halogencycloalkyl und C₁-C₂-Halogenalkoxy bedeutet und R^{b} OH, C₁-C₄-Alkyl, C₁-C₂-Alkoxy, C₁-C₂-Halogenalkyl, C₃-C₆-Cycloalkyl, C₃-C₆-Halogencycloalkyl und C₁-C₂-Halogenalkoxy bedeutet.

10. Verbindungen nach einem der Ansprüche 1 bis 9, wobei R² Wasserstoff bedeutet.

11. Zusammensetzung umfassend eine Verbindung der Formel I wie in einem der Ansprüche 1 bis 10 definiert, ein N-Oxid oder ein landwirtschaftlich annehmbares Salz davon.

12. Zusammensetzung nach Anspruch 11, die zusätzlich einen weiteren Wirkstoff umfasst.

13. Verwendung einer Verbindung der Formel I wie in einem der Ansprüche 1 bis 10 definiert und/oder eines landwirtschaftlich annehmbaren Salzes davon oder der Zusammensetzungen wie in einem der Ansprüche 11 oder 12 definiert für die Bekämpfung von phytopathogenen Pilzen.

14. Verfahren zum Bekämpfen von phytopathogenen Pilzen, bei dem man die Pilze oder die Materialien, Pflanzen, den Boden oder die Samen, die gegen Pilzbefall geschützt werden sollen, mit einer wirksamen Menge an mindestens einer Verbindung der Formel I wie in einem der Ansprüche 1 bis 10 definiert oder mit einer Zusammensetzung wie in einem der Ansprüche 11 oder 12 definiert behandelt.

15. Saatgut, beschichtet mit mindestens einer Verbindung der Formel I wie in einem der Ansprüche 1 bis 10 definiert und/oder mit einem landwirtschaftlich annehmbaren Salz davon oder mit einer Zusammensetzung wie in einem der Ansprüche 11 oder 12 definiert, in einer Menge von 0,1 bis 10 kg pro 100 kg Saatgut.

## Revendications

1. Composés de formule I 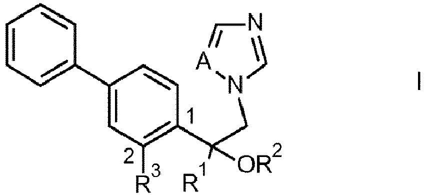 dans lesquels
A est CH ou N ;
R¹ est C₁-C₆-alkyle, C₂-C₆-alcényle, C₂-C₆-alcynyle ou C₃-C₆-cycloalkyle ;
où les motifs aliphatiques de R¹ ne sont pas substitués davantage ou portent un, deux, trois ou jusqu'au nombre maximum possible de groupements R^{1a} identiques ou différents qui sont choisis indépendamment les uns des autres parmi :
R^{1a} halogène, OH, CN, C₁-C₄-alcoxy, C₃-C₆-cycloalkyle, C₃-C₆-halogénocycloalkyle et C₁-C₄-halogénoalcoxy ;
où les motifs cycloalkyle de R¹ ne sont pas substitués davantage ou portent un, deux, trois, quatre, cinq ou jusqu'au nombre maximum de groupements R^{1b} identiques ou différents qui sont choisis indépendamment les uns des autres parmi :
R^{1b} halogène, OH, CN, C₁-C₄-alkyle, C₁-C₄-alcoxy, C₁-C₄-halogénoalkyle, C₃-C₆-cycloalkyle, C₃-C₆-halogénocycloalkyle et C₁-C₄-halogénoalcoxy ;
R² est hydrogène, C₁-C₄-alkyle, C₂-C₄-alcényle ou C₂-C₄-alcynyle ;
où les motifs aliphatiques de R² ne sont pas substitués davantage ou portent un, deux, trois ou jusqu'au nombre maximum possible de groupements R^{2a} identiques ou différents qui sont choisis indépendamment les uns des autres parmi :
R^{2a} halogène, OH, CN, C₁-C₄-alcoxy, C₃-C₆-cycloalkyle, C₃-C₆-halogénocycloalkyle et C₁-C₄-halogénoalcoxy ;
R³ est choisi parmi halogène, CN, C₁-C₄-alkyle, C₁-C₄-alcoxy, C₂-C₄-alcényle, C₂-C₄-alcynyle, C₃-C₆-cycloalkyle et S(O)ₚ(C₁-C₄-alkyl), où chacun parmi R³ est non substitué ou substitué en outre par un, deux, trois ou quatre R^{3a}; où
R^{3a} est choisi indépendamment parmi halogène, CN, OH, C₁-C₄-alkyle, C₁-C₄-halogénoalkyle, C₃-C₆-cycloalkyle, C₃-C₆-halogénocycloalkyle, C₁-C₄-alcoxy et C₁-C₄-halogénoalcoxy ;
p vaut 0, 1 ou 2 ;
ainsi que les N-oxydes et les sels acceptables sur le plan agricole de ceux-ci.

2. Composés selon la revendication 1, dans lesquels A est N.

3. Composés selon la revendication 1 ou 2, dans lesquels R³ est F, Cl, Br ou CN.

4. Composés selon la revendication 1 ou 2, dans lesquels R³ est C₁-C₄-alkyle ou C₁-C₄-halogénoalkyle.

5. Composés selon la revendication 1 ou 2, dans lesquels R³ est C₁-C₄-alcoxy ou S(C₁-C₄-alkyl).

6. Composés selon l'une quelconque des revendications 1 à 5, dans lesquels R¹ est C₁-C₆-alkyle, qui n'est pas substitué davantage ou porte un, deux, trois, quatre ou cinq groupements R^{1a} identiques ou différents, choisis indépendamment parmi F, OH, CN, C₃-C₆-cycloalkyle, C₃-C₆-halogénocycloalkyle et C₁-C₂-halogénoalcoxy.

7. Composés selon l'une quelconque des revendications 1 à 5, dans lesquels R¹ est 1-(C₂-C₆) alcényle ou 1-(C₂-C₆)alcynyle, qui ne sont pas substitués davantage ou portent un, deux, trois, quatre ou cinq groupements R^{1a} identiques ou différents.

8. Composés selon l'une quelconque des revendications 1 à 5, dans lesquels R¹ est C₃-C₆-cycloalkyle, qui n'est pas substitué davantage ou porte un, deux, trois, quatre ou cinq groupements R^{1b} identiques ou différents, choisis indépendamment parmi OH, C₁-C₄-alkyle, C₁-C₂-alcoxy, C₁-C₂-halogénoalkyle, C₃-C₆-cycloalkyle, C₃-C₆-halogénocycloalkyle et C₁-C₂-halogénoalcoxy.

9. Composés selon la revendication 1, dans lesquels R^{a} est F, OH, CN, C₃-C₆-cycloalkyle, C₃-C₆-halogénocycloalkyle et C₁-C₂-halogénoalcoxy, et R^{b} est OH, C₁-C₄-alkyle, C₁-C₂-alcoxy, C₁-C₂-halogénoalkyle, C₃-C₆-cycloalkyle, C₃-C₆-halogénocycloalkyle et C₁-C₂-halogénoalcoxy.

10. Composés selon l'une quelconque des revendications 1 à 9, dans lesquels R² est hydrogène.

11. Composition comprenant un composé de formule I, tel que défini selon l'une quelconque des revendications 1 à 10, un N-oxyde ou un sel acceptable sur le plan agricole de celui-ci.

12. Composition selon la revendication 11, comprenant en outre une autre substance active.

13. Utilisation d'un composé de formule I, tel que défini selon l'une quelconque des revendications 1 à 10, et/ou d'un sel acceptable sur le plan agricole de celui-ci, ou des compositions, telles que définies selon l'une quelconque des revendications 11 ou 12, pour lutter contre des champignons phytopathogènes.

14. Méthode de lutte contre des champignons phytopathogènes, comprenant le traitement des champignons ou des matériaux, des plantes, du sol ou des semences devant être protégés contre une attaque fongique, par une quantité efficace d'au moins un composé de formule I, tel que défini selon l'une quelconque des revendications 1 à 10, ou d'une composition, telle que définie selon l'une quelconque des revendications 11 ou 12.

15. Semence enrobée par au moins un composé de formule I, tel que défini selon l'une quelconque des revendications 1 à 10, et/ou un sel acceptable sur le plan agricole de celui-ci, ou par une composition, telle que définie selon l'une quelconque des revendications 11 ou 12, selon une quantité allant de 0,1 à 10 kg pour 100 kg de semences.
